(19) Europäisches Patentamt
European Patent Office
Office européen des brevets

(11) Publication number: **0 212 404 A1**

(12) **EUROPEAN PATENT APPLICATION**

(21) Application number: **86110710.0**

(22) Date of filing: **02.08.86**

(51) Int. Cl.⁴: **C 07 D 499/00**, C 07 D 205/08, C 07 F 7/18, A 61 K 31/43

(30) Priority: **16.08.85 GB 8520631**

(43) Date of publication of application: **04.03.87 Bulletin 87/10**

(84) Designated Contracting States: **AT BE CH DE FR IT LI LU NL SE**

(71) Applicant: **HOECHST AKTIENGESELLSCHAFT, Postfach 80 03 20, D-6230 Frankfurt am Main 80 (DE)**

(72) Inventor: **Cooke, Michael David, 10 Westbury Lane, Newport Pagnell Bucks (GB)**
Inventor: **Connolly, Stephen, 19 Page Hill Avenue, Buckingham Bucks (GB)**

(54) **7-oxo-4-thia-1-azabicyclo [3.2.0] hept-2-ene derivatives.**

(57) A compound of formula I

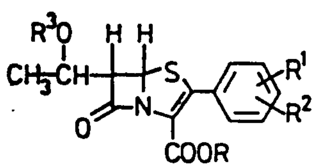

(I)

in which
R represents a hydrogen atom or a carboxylic acid esterifying group;
$R^1$ represents
(i) one of the following groups

-CN -CHO -CORa

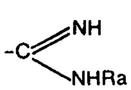 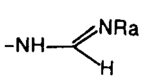 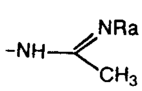 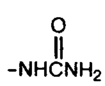

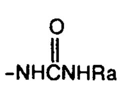 $-NO_2$ $-NHSO_2Ra$ 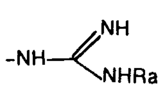

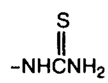 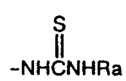 -NHCHO -NHCORa

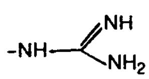 -CONHORa $-CONH_2$ -CONHRa

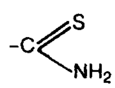 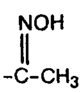 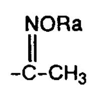 -SORa

$-SO_2Ra$ $-SO_2NH_2$ $-SO_2NHRa$ $-SO_2NRaRb$

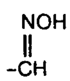 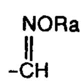

in which Ra and Rb, which may be the same or different, each represents an alkyl group having from 1 to 4 carbon atoms, or
(ii) a $-CONH(CH_2)_mQ$ group, in which
m represents an integer of from 1 to 3, and
Q represents one of the following groups

-SORc $-SO_2Rc$ -CN $-NH_2$ -NHCH=NH

EP 0 212 404 A1

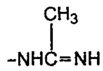 -NHRc 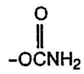 $-CONH_2$

$-CONHCH_3$ -CONHOH $-SO_2NH_2$ -SONHRc

in which Rc represents a methyl or ethyl group, or

(iii) a $-CO_2Rd$ group, in which Rd represents a methyl or ethyl group which is unsubstituted or is substituted by one or more substituents, which may be the same or different, selected from

(a) halogen atoms and vinyl groups,

(b) phenyl groups which are unsubstituted or are substituted by one or more groups selected from alkoxy groups having from 1 to 4 carbon atoms, nitro groups and halogen atoms,

(c) silyl groups SiReRfRg, the groups Re, Rf and Rg being the same or different, each representing a phenyl group or an alkyl group having from 1 to 4 carbon atoms, and

(d) groups Q as defined above; or

(iv) a $-CO_2SiReRfRg$ group, in which Re, Rf and Rg are defined as in (c) above, or a $-CO_2$-phenyl group, in which the phenyl moiety is unsubstituted or substituted as defined in (b) above;

$R^2$ represents (i) a hydrogen atom,

(ii) a group as defined above for $R^1$ ($R^1$ and $R^2$ being the same or different), or

(iii) a chlorine, bromine or iodine atom, an alkyl group having from 1 to 4 carbon atoms, an $-NH_2$, -NHRa or -NRaRb group, an -OH or -ORa group, or an $-OCOCH_3$ group, Ra and Rb being defined as above, and

$R^3$ represents a hydrogen atom or a hydroxy protecting group; and in which $R^1$ and $R^2$, independently of each other, may be present at any position on the phenyl ring; and salts thereof, and isomers thereof have antibacterial activity.

## 7-OXO-4-THIA-1-AZABICYCLO[3.2.0]HEPT-2-ENE DERIVATIVES

This invention relates to 7-oxo-4-thia-1-azabicyclo-[3,2,0]hept-2-ene derivatives, to a process for their preparation, to pharmaceutical preparations comprising them, and to intermediates for use in the preparation of substances having antibacterial activity and/or β-lactamase inhibitory and/or inactivating activity.

The penicillins as a class have in common the β-lactam structure A known semi-systematically as the "penam" nucleus. The introduction of a double bond between carbon atoms 2 and 3 in this structure gives rise to the "penem" nucleus B.

(A) 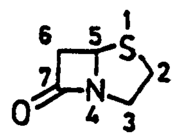

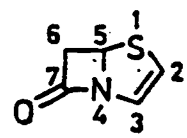 (B)

The penem nucleus B is given the systematic name "7-oxo-4-thia-1-azabicyclo[3,2,0]hept-2-ene", with the numbering as shown in formula C. A side chain at position 6 is numbered as shown in formula D.

(C) 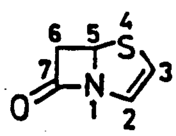

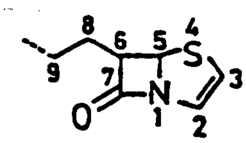 (D)

It is well known that penicillin antibiotics have been used extensively for many years to combat bacterial infections in humans and other animals. The first of the penicillin antibiotics to come into general therapeutic use was benzylpenicillin, and this compound still finds widespread use today.

There is, however, a continuing need for new antibiotics, not only to combat bacterial pathogens that do not respond satisfactorily to treatment with conventional penicillins, but also to combat those strains of bacteria which, although once susceptible to penicillin treatment, are now resistant to such therapy. Accordingly, in the search for new types of antibiotics, the discovery of compounds which possess a wide spectrum of activity together with an ability to combat infections caused by penicillin-resistant organisms is an important goal.

Various specifications disclose penem derivatives having a phenyl or substituted phenyl group at the 2-position. EP 0 002 210A describes penem derivatives having a wide selection of substitutents at the 2- and 6-positions, as does GB 2 042 515A. EP 0 002 210A includes in a Table of compounds those having a 1-hydroxyethyl group at position 6 and a phenyl, aminomethylphenyl, aminophenyl or trifluoromethylphenyl group at position 2. GB 2 042 515A similarly includes in a Table of compounds a 6-hydroxyethyl penem derivative having a phenyl group at position 2, that phenyl group being unsubstituted. EP 0 070 204A discloses penems with certain very specific substituted phenyl groups at the 2-position in combination with a hydrogen atom or an optionally protected hydroxyethyl group at position 6. Those Examples that relate to substituted phenyl goups at position 2 do so in combination with a hydrogen atom at position 6.

We have made and tested certain 6-hydroxyethyl-2-substituted phenyl derivatives disclosed generally in the prior art, and we have found that although they have activity in a representative antibacterial screen, there remains a need for compounds having higher activities.

The present invention provides a compound of formula I

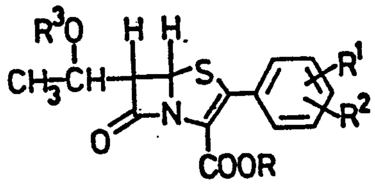

(I)

in which

R represents a hydrogen atom or a carboxylic acid esterifying group;

$R^1$ represents

(i) one of the following groups

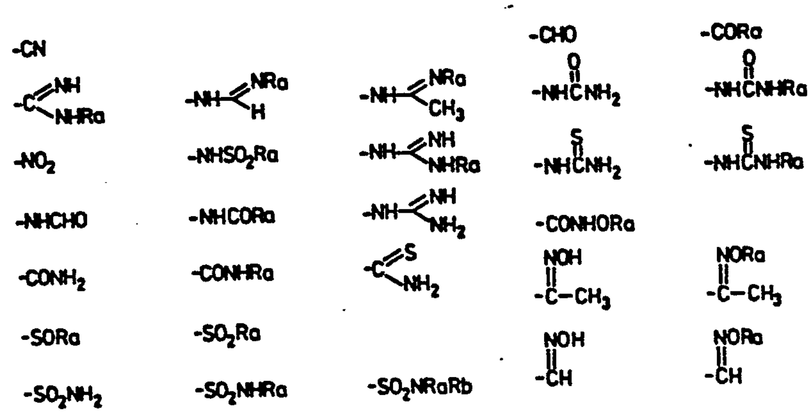

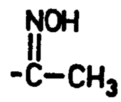 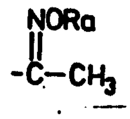 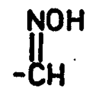 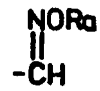

in which Ra and Rb, which may be the same or different, each represents an alkyl group having from 1 to 4 carbon atoms, or

(ii) a $-CONH(CH_2)_mQ$ or $-NHCO(CH_2)_mQ$ group, in which m represents an integer of from 1 to 3, and Q represents one of the following groups

| | | |
|---|---|---|
| $-CN$ | $-SO_2NH_2$ | $-SONHRc$ |
| $-SORc$ | $-SO_2Rc$ | |
| $-NH_2$ | $-NHCH=NH$ | 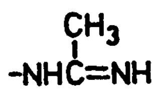 |
| $-CONH_2$ | $-CONHCH_3$ | $-CONHOH$ |
| $-NHRc$ | 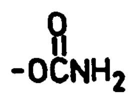 | |

in which Rc represents a methyl or ethyl group, or

(iii) a $-CO_2Rd$ group, in which Rd represents a methyl or ethyl group which is unsubstituted or is substituted by one or more substituents, which may be the same or different, selected from

(a) halogen atoms and vinyl groups,

(b) phenyl groups which are unsubstituted or are substituted by one or more groups selected from alkoxy groups having from 1 to 4 carbon atoms, nitro groups and halogen atoms,

(c) silyl groups SiReRfRg, the groups Re, Rf and Rg being the same or different, each representing a phenyl group or an alkyl group having from 1 to 4 carbon atoms, and

(d) groups Q as defined above; or

(iv) a $-CO_2SiReRfRg$ group, in which Re, Rf and Rg are

defined as in (c) above, or (v) a $-CO_2$-phenyl group, in which the phenyl moiety is unsubstituted or substituted as defined in (b) above;

$R^2$ represents (i) a hydrogen atom,

(ii) a group as defined above for $R^1$ ($R^1$ and $R^2$ being the same or different), or

(iii) a chlorine, bromine or iodine atom, an alkyl group having from 1 to 4 carbon atoms, an $-NH_2$, -NHRa or -NRaRb group, an -OH or -ORa group, or an $-OCOCH_3$ group, Ra and Rb being defined as above, and

$R^3$ represents a hydrogen atom or a hydroxy protecting group; and in which $R^1$ and $R^2$, independently of each other, may be in any position on the phenyl ring;

and salts thereof; and isomers thereof.

In a compound of formula I, a protected carboxy group -COOR is preferably an esterified carboxy group that can be converted by hydrolysis, by photolysis, by oxidation, by reduction or, especially, by esterase enzyme action, to give the free acid of formula I. Moreover, in a compound of formula I, a hydroxy protecting group $R^3$ is preferably a group that can be converted by hydrolysis, by photolysis, by reduction or, especially, by esterase enzyme action, to give a compound of formula I having a free 8-hydroxy group. Details of such carboxy and hydroxy protecting groups are given below.

Groups that can be removed by esterase action are groups that can be cleaved in vivo. Compounds having such groups are known as "prodrugs". Preferred compounds of formula I are those in which, independently, R represents a hydrogen atom, a physiologically tolerable salt-forming group, or a group that can be cleaved in vivo to give the free carboxy group or a carboxylate group, and $R^3$ represents a hydrogen atom or a group that can be cleaved in vivo to give the free hydroxy group.

The present invention provides salts of a compound of formula I, especially physiologically tolerable salts thereof. A salt may be formed at the 2-carboxylic acid group or at any acidic or basic centre present. Moreover,

when both an acidic centre and a basic centre are present, a compound of formula I may exist in a zwitterionic form. A carboxylic acid salt of a compound of formula I is preferable to the free acid if solubility is important.

In the definitions of $R^1$ and $R^2$, an alkyl group Ra or Rb is preferably a methyl or ethyl group, especially a methyl group. The symbol "m" preferably denotes the integer 1 or 2.

Of the groups $R^1$ given above, the following are preferred

| | | |
|---|---|---|
| $-CONH_2$ | $-CONHRa$ | $-NHCO(CH_2)_mQ$ |
| $-CONH(CH_2)_mQ$ | 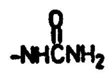 | |
| $-NHCHO$ | $-NHCORa$ | 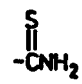 |
| $-SORa$ | $-SO_2NH_2$ | $-SO_2NHRa$ |

Ra, Q and m being as defined above, and especially having the preferred meanings given above. Particularly preferred as $R^1$ are $-CONH_2$, $-CONHCH_3$, $-NHCHO$ and $-NHCOCH_3$ groups.

A compound of formula I may exist in various isomeric forms, all of which are part of the present invention, for example, a substituent $R^1$ may be present at any position on the phenyl ring when $R^2$ represents a hydrogen atom. When $R^2$ represents other than a hydrogen atom, $R^1$ and $R^2$ may occupy any two positions on the ring, subject to known constraints regarding, for example, stereochemical considerations. Moreover, some substituents $R^1$ and $R^2$ may themselves exist in various tautomeric and/or geometric isomeric forms, for example, an oxime may be in <u>syn</u> or <u>anti</u> form. As mentioned above, all isomers are part of the present invention.

The stereochemistry at positions 5, 6 and 8 of a compound of formula I can be R or S, independently (R and S being as defined by the Cahn-Ingold-Prelog system of nomenclature). The preferred stereochemistry at position 5 is R, at position 6 is S, and at position 8 is R. 5R, 6S, 8R-stereochemistry is particularly preferred.

The present invention also provides a process for the production of a compound of formula I, which comprises (A) treating a compound of formula II

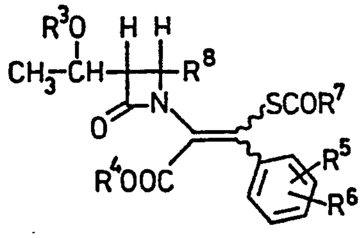

(II)

in which $R^3$ is as defined above,

$R^4$ represents a carboxy protecting group,

$R^5$ represents a group $R^1$ as defined above or a group that can be converted into a group $R^1$,

$R^6$ represents a group $R^2$ as defined above or a group that can be converted into a group $R^2$,

$R^7$ represents a phenyl group or an alkyl group having from 1 to 4 carbon atoms, and

$R^8$ represents a bromine or chlorine atom, especially a chlorine atom,

with a base; or

(B) effecting cyclisation of a compound of formula III

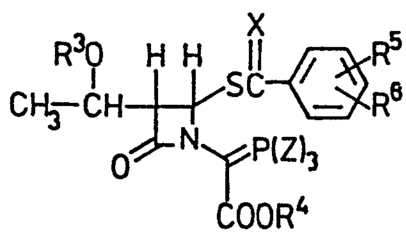

(III)

in which $R^3$, $R^4$, $R^5$ and $R^6$ are defined as above, X represents an oxygen or sulphur atom, and the group $P(Z)_3$ represents a group derived from a trivalent organophosphorus reagent, or

(C) treating a compound of formula IV or V

(IV) 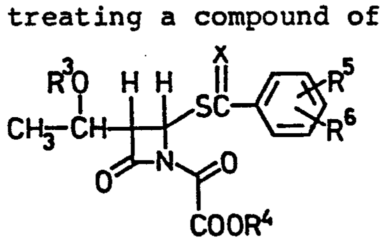

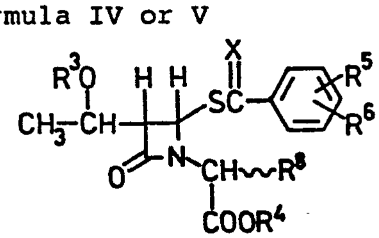 (V)

in which X, $R^3$, $R^4$, $R^5$, $R^6$ and $R^8$ are defined as above, with a trivalent organophosphorus compound and effecting cyclisation, or

(D) reacting a compound of formula VI

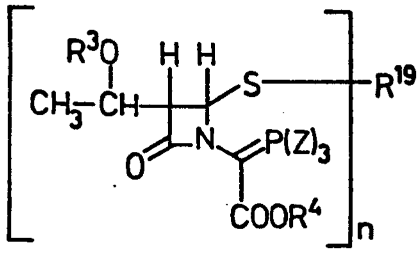

(VI)

in which $R^3$, $R^4$ and the group $P(Z)_3$ are defined as above, and $R^{19}$ represents Cu(II), Pb(II) or Hg(II), in which case n represents 2, or $R^{19}$ represents Ag(I), in which case n represents 1, with a compound of formula VII

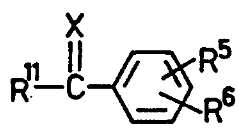

(VII)

in which $R^{11}$ represents an activating group, for example, an activating ester group or, preferably a halogen atom, and especially a chlorine atom, and X, $R^5$ and $R^6$ are defined as above, and effecting cyclisation, and

(E) in an appropriate compound in which $R^5$ and/or $R^6$ represents a group that can be converted into a group $R^1$ and/or $R^2$, respectively, converting such a group or groups $R^5$ and/or $R^6$ into such a group or groups $R^1$ and/or $R^2$ and,

(F) if desired or required, in an appropriate compound converting a group $R^1$ and/or a group $R^2$ into another group $R^1$ and/or $R^2$, respectively; and

(G) if desired or required, carrying out any one or more of the following steps in any desired order:

a) hydrolysing a 2-carboxylic ester group in an appropriate compound to give the corresponding free acid,

b) treating an appropriate free acid or a salt thereof with an agent capable of forming a 2-carboxylic acid ester, for example, with an alcohol, a phenol or a reactive derivative thereof, to give a 2-carboxylic acid ester thereof,

c) carrying out an acid- or base-catalysed ester interchange on an appropriate 2-carboxylic acid ester to give a different ester of that compound,

d) treating an appropriate free acid compound with a base

to give a salt at the carboxy group at position 2,
e) treating an appropriate free acid or 2-carboxylic acid ester having a basic group present with an acid to give an acid addition salt thereof,
f) treating a salt of an appropriate compound with an acid to give a free acid of that compound,
g) removing a hydroxy protecting group from an appropriate compound having a protected 8-hydroxy group to give the corresponding compound having a free 8-hydroxy group,
h) treating an appropriate compound having a free hydroxy group at the 8-position with an organic acid derivative to form an ester at the 8-position, and
i) treating an appropriate compound to effect a change in the stereochemical configuration.

In formula I, $R^3$ may represent a hydrogen atom or a protecting group. In certain of the processes involved in the production of a compound of formula I, it may be preferable to protect the 8-hydroxy group. Similarly, it may be preferable or essential to protect the 2-carboxy group during the formation of a compound of formula I. Hydroxy and carboxy protecting groups and methods for their introduction and removal are known (that is to say, in actual use in the art or described in the literature of the art) see, for example, McOmie, Protecting Groups in Organic Chemistry, Plenum Press, London, 1973 and T.W. Greene, Protective Groups in Organic Synthesis, J. Wiley & Sons Inc. 1981. Hydroxy and carboxy protecting groups preferably used in the present invention and methods for their introduction and removal are described in more detail below.

Hydroxy and carboxy protecting groups may be present independently of each other in a compound of formula I or in any intermediate involved in the formation thereof. Moreover, protecting groups may be introduced and removed at any appropriate point in a reaction sequence.

A compound of formula II is treated with a base to give a compound of formula I or a precursor thereof. The

carboxy group in the compound of formula I is protected, but the hydroxy group may be free or protected. The base may be inorganic or organic, for example, ammonia, or an alkali metal, especially a sodium or potassium, carbonate, bicarbonate, or hydroxide; a primary amine, for example, methylamine, ethylamine, aniline or benzylamine; an alkali metal alkoxide, for example, sodium methoxide; or a heterocyclic base, for example, having a $pK_a$ within the range of from 5 to 9, for example, imidazole, pyridine or a substituted pyridine, for example, an alkyl-, amino-, monoalkylamino- or dialkylamino-substituted pyridine, for example, 4-methylpyridine or 4-dimethylaminopyridine. Imidazole is particularly preferred.

The reaction is generally carried out in a solvent or diluent, the choice of which is wide, provided that it is inert under the reaction conditions. Examples of solvents and diluents are oxygenated hydrocarbons, for example, ethers, for example, having up to 4 carbon atoms, for example, diethyl ether, also tetrahydrofuran and dioxane; ketones, for example, having up to 4 carbon atoms, for example, acetone and methyl ethyl ketone; esters, for example, methyl acetate and ethyl acetate; and amides, for example, dimethylformamide and dimethylacetamide; also chlorinated hydrocarbons, for example, chloroform, methylene chloride and carbon tetrachloride; aromatic hydrocarbons, for example, benzene and toluene; and other solvents for example, acetonitrile and nitromethane. A mixture of two or more solvents may be used, and solvents are preferably used in admixture with water; preferably a water-miscible solvent is used in admixture with 5 to 20% (v/v) water.

The reaction is generally carried out at a temperature within the range of from 0 to 40°C, preferably from 0 to 20°C.

A compound of formula Ia, that is to say, a compound analogous to compound I but having groups $R^5$ and $R^6$, may be prepared as shown in the following Reaction Scheme I:

REACTION SCHEME I

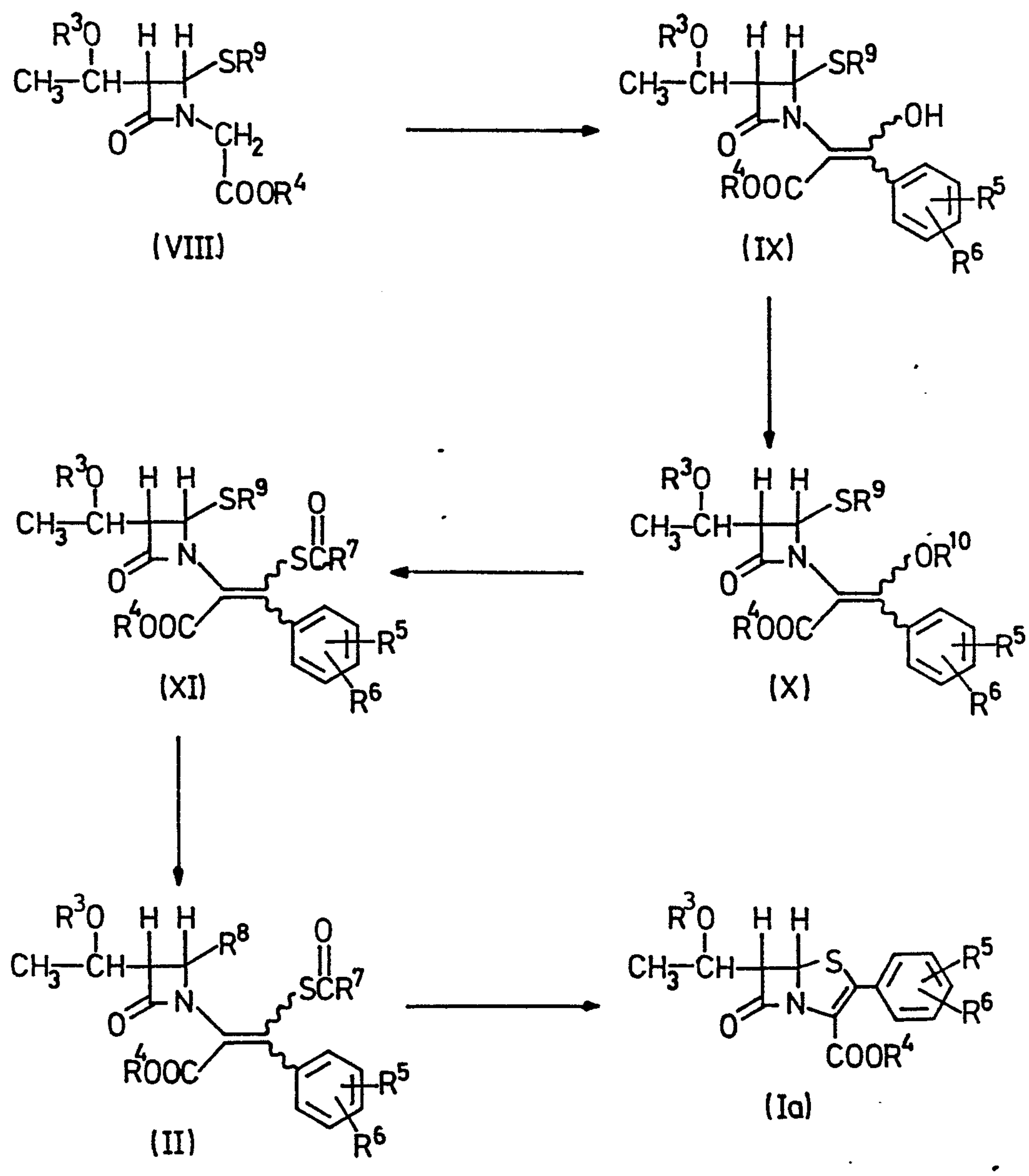

(VIII)

(IX)

(XI)

(X)

(II)

(Ia)

in which $R^3$, $R^4$, $R^5$, $R^6$ $R^7$ and $R^8$ are defined as above, $R^9$ represents an alkyl group having from 1 to 8, preferably from 1 to 4 carbon atoms, an alkenyl group having from 2 to 4 carbon atoms, or a phenyl group, and $R^{10}$ represents a group $-SO_2-Rh$ or -CORh in which Rh represents an alkyl group having from 1 to 4 carbon atoms, an optionally substituted phenyl group, or a polyfluoroalkyl group, especially a trifluoromethyl group.

Some compounds of formula VIII are known, see for example, GB 2 102 798A and Belgian Patent 887,886. Other compounds of formula VIII may be prepared analogously.

To obtain compound IX, compound VIII is reacted, in the presence of a base, with an activated carboxylic acid derivative of formula VIIa

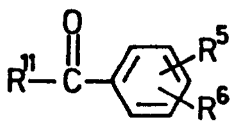

(VIIa)

in which $R^5$, $R^6$ and $R^{11}$ are as defined above.

Many compounds of formula VIIa are known, and the others may be prepared by methods analogous to those described for the preparation of the known compounds, see for example, Organic Syntheses, Collective Volume 4, page 715, Wiley, New York, 1963, or Collective Volume 2, page 528, 1943.

The reaction between compound VIII and compound VIIa is carried out in the presence of a base, preferably having a $pK_a \geq 20$, preferably a metallated amine, and examples of preferred bases are lithium diisopropylamide, lithium hexamethyldisilazide, lithium 2,2,6,6-tetramethylpiperidide, lithium cyclohexyl isopropylamide, and sodamide.

The reaction is generally carried out in an aprotic solvent, for example, an oxygenated hydrocarbon, preferably an ether, for example, diethyl ether, tetrahydrofuran, dioxane, glyme or diglyme. The reaction temperature is,

for example, from -120 to +30°C, preferably from -78 to -20°C.

The amount of base used is, for example, from 1 to 3 moles, calculated per mole of compound VIII, preferably from 1.5 to 2.5 moles of base. The compound of formula VIIa is preferably used in an amount of from 1 to 1.5 moles per mole of compound VIII, preferably from 1 to 1.1 moles of compound VIIa per mole of compound VIII.

The reaction may be carried out as follows: The base may be added to a stirred solution of compounds VIII and VIIa. Alternatively, to a stirred solution of compound VIII under an inert atmosphere is added the base and subsequently a solution of compound VIIa in the same or a different solvent.

Compound IX is generally obtained in the form of a mixture of isomers generally comprising the E, Z and oxo isomers. Such isomers may be separated, but this is not generally necessary. (The terms E and Z are as defined on page 142 of Allinger et al, "Organic Chemistry" 1971, Worth, New York.)

Compound IX is reacted, in the presence of a base, with a compound of formula XII

$$R^{10} - R^{11} \qquad (XII)$$

in which $R^{10}$ is as defined above, and represents, for example, a methylsulphonyl, phenylsulphonyl or poly-fluoroalkylsulphonyl group, especially a trifluoromethylsulphonyl group, or a trifluoroacetyl group and $R^{11}$ represents an activating group as defined above, to produce a compound of formula X.

The base used in the reaction between compounds IX and XII may be organic or inorganic and is, for example, a tertiary amine, for example, a trialkylamine, especially triethylamine or ethyldiisopropylamine, or a heterocyclic base, for example, pyridine or an alkyl-, dialkyl-, amino, monoalkylamino- or dialkylamino-substituted pyridine, for

example, 4-dimethylaminopyridine. The reaction is generally carried out in a solvent or diluent, for example, a chlorinated hydrocarbon, for example, dichloromethane, an ether, for example, diethyl ether or tetrahydrofuran, or an ester, for example, ethyl acetate. The temperature of the reaction is generally within the range of from -80 to + 20°C. Compound X is generally produced in the form of a mixture of isomers generally comprising the E- and Z-isomers. Such isomers may be separated, but this is not generally necessary.

Compound X is converted into compound XI by reaction with a compound of formula XIII

$$R^7COSH \qquad (XIII)$$

in which $R^7$ is as defined above and preferably represents a methyl or, especially, t-butyl group. Compound XIII is preferably in the form of a reactive derivative thereof, for example, as an alkali metal, alkaline earth metal or organic amine salt. Compound XIII may be converted into salt form in situ.

The reaction between compound X and compound XIII is generally carried out in a polar solvent, for example, acetonitrile, dimethylformamide or dimethyl sulphoxide. The reaction temperature is generally within the range of from 0 to 40°C, conveniently room temperature.

The $-SCOR^{10}$ group in the resulting compound of formula XI may be E or Z to the $-COOR^4$ group. The isomers may be separated for the subsequent reaction, but this is not generally necessary, and the isomeric mixture is generally used as both isomers give a compound of formula I.

The resulting compound XI is then halogenated to give a compound of formula II, using an agent capable of splitting a carbon-sulphur bond and of introducing a halogen atom. Such agents are well known in the art and include, for example, molecular chlorine, sulphuryl

chloride, t-butyl hypochlorite, cyanogen chloride and molecular bromine.

The reaction is generally carried out at a temperature within the range of from -60 to +20°C. The reaction is generally carried out in a solvent or diluent that is non-protic,and is inert under the reaction conditions, for example, an ether, a hydrocarbon or a halogenated hydrocarbon, for example, dioxane, benzene, chloroform or dichloromethane. A mixture of two or more solvents may be used. Examples of halogenating systems are: chlorine in chloroform, chlorine in benzene and t-butyl hypochlorite in benzene. In the latter two cases, the temperature is preferably from 5 to 20°C, and normally from 5 to 10°C. Generally, 1 to 2 moles of the halogenating agent are used per mole of compound VIIa, cf. S. Kukolja, J. Amer. Chem. Soc. (1971), 93, 6267 and P.C. Cherry, C.E. Newall and N.S. Watson, J.C.S. Chem. Comm. 1979 p. 663.

The resulting compound of formula II may be converted into a compound of formula I as described above.

In the Reaction Scheme I described above, it is preferable that the 8-hydroxy group in compound VIII is protected, to prevent it from reacting with the activated acid derivative of formula VIIa. The hydroxy group is also preferably protected during the reaction between compound IX and compound XII. A hydroxy protecting group may be retained in compounds XI and II, but it has been found that if a compound of formula XI having S-stereochemistry at position 3 and having a protected hydroxy group at position 8 is halogenated, the resulting compound of formula I has the less desirable 5S stereochemistry, and it is then necessary to change the sterochemistry at position 5, for example, by heating under reflux, if the 5R stereochemistry is wanted.

Accordingly, it is generally preferable to protect the 8-hydroxy group until compound X has been formed, and to remove the hydroxy protecting group before halogenating compound XI to give compound II. A hydroxy protecting

group may be removed from compound X before it is converted into compound XI, or it may be removed after formation of compound XI.

One type of preferred protected group $-OR^3$ is that from which the protecting group $R^3$ can be removed under acidic conditions. Such protected groups are well known in the art and are, for example, tetrahydropyranyloxy and tetrahydrofuranyloxy groups; acetal and ketal groups, for example, of formula

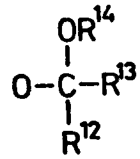

in which $R^{12}$ and $R^{13}$, which may be the same or different, each represents a hydrogen atom or a lower alkyl group, preferably a methyl group, or $R^{12}$ and $R^{13}$ together with the carbon atoms to which they are attached, represent a cycloalkyl ring having from 4 to 7 carbon atoms, and $R^{14}$ represents a lower alkyl group, preferably a methyl or ethyl group, or $R^{12}$ and $R^{14}$, together with the carbon atom and the oxygen atom to which they are attached, respectively, represent a tetrahydropyranyl ring; also silyl ethers, for example, having three substituents on the silicon atom, and preferably up to 24 carbon atoms in total, the three substituents being the same or different, and selected from alkyl, alkenyl and cycloalkyl groups, and phenyl and phenalkyl groups which may be unsubstituted or substituted as defined above, for example, -OSiReRfRg groups, in which Re, Rf and Rg are as defined above, that is to say, they may be the same or different, and each represents an alkyl group having from 1 to 4 carbon atoms or a phenyl group, for example, giving trimethylsilyloxy, triethylsilyloxy, diphenyl-t-butylsilyloxy, dimethyl-t-butylsilyloxy, and methyldiphenylsilyloxy groups.

Preferred hydroxy protecting groups $R^3$ are tetrahydropyranyl, 2-methoxyprop-2-yl, trimethylsilyl and, especially, triethylsilyl and t-butyldimethylsilyl groups.

Such groups may be removed by acid hydrolysis, for example, using 0.1 to 2M, preferably 0.5M hydrochloric acid, for example, using the appropriate amount of 6M HCl in, for example, tetrahydrofuran, cf. Belgian Patent Specification No. 881 012; n-$Bu_4NF$ in an acidic medium, for example, in acetic acid, cf. Belgian Patent Specification No. 882 764; or aqueous hydrogen fluoride, for example, in the presence of acetonitrile, cf. J. Chem. Soc. Perkin 1, 1981, 2055.

A compound of formula Ia may be produced from a compound of formula III, IV, V or VI, as shown in the following Reaction Scheme II:

A compound of formula III will cyclise to give a compound of formula I at room temperature if left for sufficient time, but generally heat is applied to accelerate the reaction. The cyclisation is preferably carried out in a solvent, for example, an ether, for example, diethyl ether, dioxane or tetrahydrofuran, an aromatic hydrocarbon, for example, benzene, toluene or xylene, a halogenated hydrocarbon, for example, chloroform or dichloromethane, or dimethylformamide or dimethyl sulphoxide. The temperature used may be from room temperature to the reflux temperature of the reaction mixture.

A compound of formula III may be produced from a compound of formula IV, V or VI, as shown in Reaction Scheme II, and may be isolated, if desired. Each of compounds IV, V and VI may, however, give a compound of formula Ia directly, also as shown in Reaction Scheme II. In general, if a compound of formula IV, V or VI is reacted at lower temperatures, a compound of formula III is formed, and may be isolated and then preferably heated as described above to give a compound of formula Ia. If higher reaction temperatures are used, a compound of formula Ia is the reaction product obtained. The latter reaction may proceed via a compound of formula III or via another intermediate. Further details of these procedures are given below.

REACTION SCHEME II

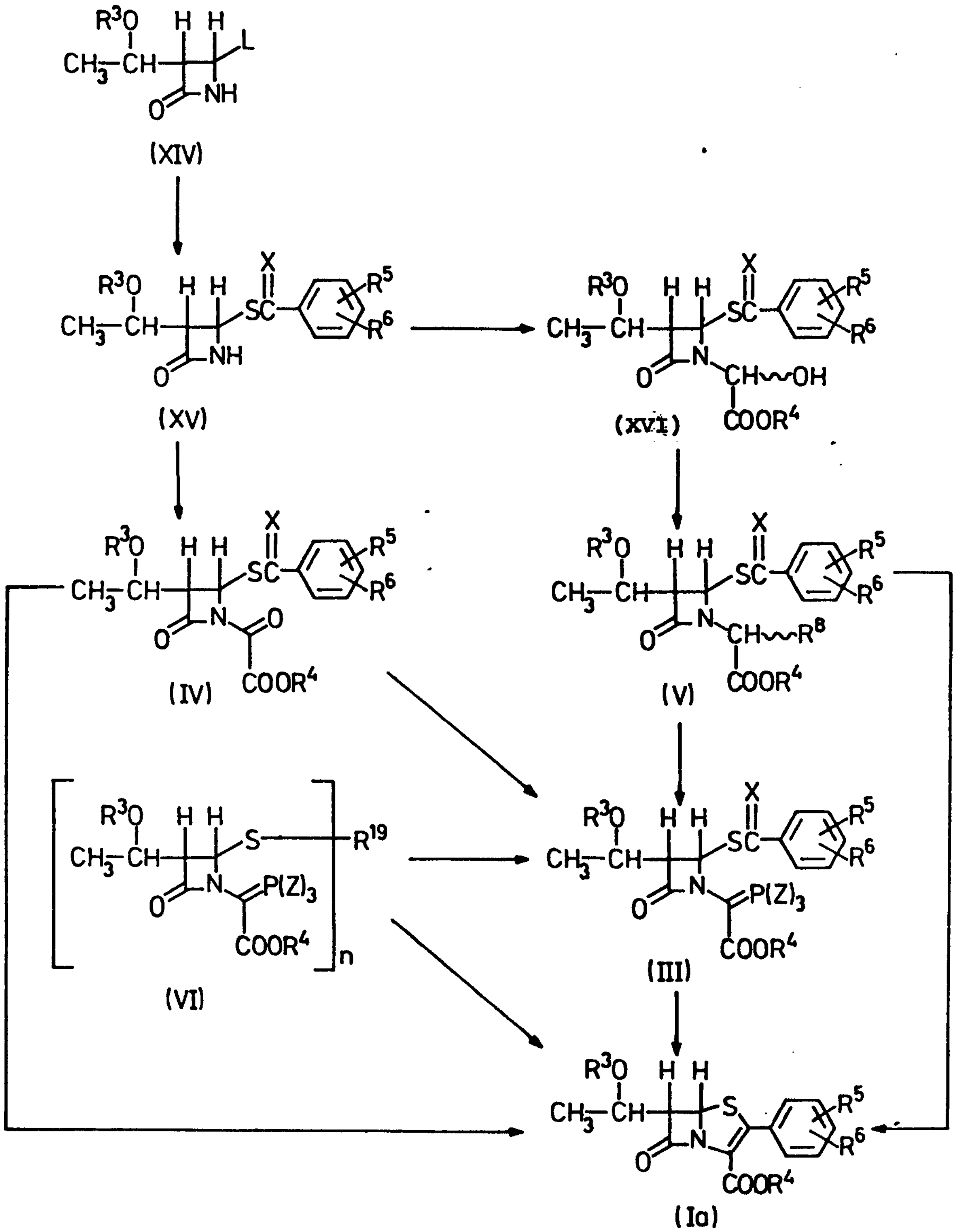

In Reaction Scheme II $R^3$, $R^4$, $R^5$, $R^6$, $R^8$, X and Z are defined as above, and L represents a leaving group, that is to say, a group that can be replaced in a nucleophilic displacement reaction, for example, a halogen atom, especially a chlorine atom; an alkylcarbonyloxy group in whcih the alkyl moiety has from 1 to 4 carbon atoms, has a straight or branched chain, and may be substituted by an electron-withdrawing group, for example, a halogen atom, especially a fluorine atom, for example, an acetoxy or trifluoroacetoxy group; a phenylcarbonyloxy group, for example a benzoyloxy group; or an $-SO_2Rj$ group in which Rj represents an alkyl group having from 1 to 4 carbon atoms or a phenyl group. L preferably represents an acetoxy group.

Certain compounds of formula XIV are known, for example, when $R^3$ represents a dimethyl-t-butylsilyloxy group (Belgian Patent Specification No. 882 764), and when $R^3$ represents a p-nitrobenzylcarbonyl group (EP 0 002 210A). Other compounds of formula XIV may be prepared analogously.

A compound of formula XV may be prepared by reacting a compound of formula XIV with a compound of formula XVII

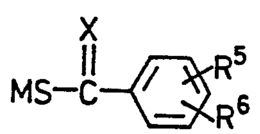

(XVII)

in which X, $R^5$ and $R^6$ are defined as above, and M represents a hydrogen atom or an alkali metal or alkaline earth metal atom, or an ammonium group that is unsubstituted or substituted by, for example, one to four groups selected from alkyl groups having from 1 to 4 carbon atoms. (When M represents hydrogen the reaction is carried out in the presence of a base, see below).

Compound XVII is generally prepared <u>in situ</u> and is not generally isolated before reaction with compound XIV.

A compound XVII may be prepared

(i) by reacting a compound of formula VII

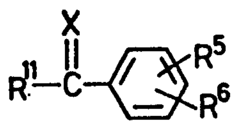

(VII)

in which X, $R^5$, $R^6$ and $R^{11}$ are defined as above, with a compound of formula MSH or $M_2S$, in which M is as defined above, (when M represents hydrogen the reaction is carried out in the presence of a base, for example, pyridine or a tri-alkylamine in which each alkyl moiety has from 1 to 4 carbon atoms); or

(ii) by reacting a compound of formula XVIII

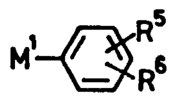

(XVIII)

in which $M^1$ represents an alkali metal or alkaline earth metal radical, together with a counter-ion, if required in the case where $M^1$ represents a divalent metal ion, with carbon disulphide or carbon oxysulphide, giving a compound of formula XVII in which M represents an alkali metal or alkaline earth metal atom.

In process (ii) above, the compound of formula XVIII is an organo-metallic derivative, for example, a phenyl lithium or phenyl magnesium halide derivative, and may be prepared, for example, by using the corresponding phenyl halide, especially the phenyl bromide or iodide, with the appropriate metal, for example, lithium or magnesium, in an inert solvent or diluent, for example, an ether, for example, diethyl ether, or an aromatic hydrocarbon, for example, toluene, or by using a metal-alkyl compound, for example, n-butyllithium or t-butyllithium, in an inert solvent, for example, an ether, for example, diethyl ether or tetrahydrofuran.

In each of the processes (i) and (ii) above, the reaction is generally carried out in a solvent that is inert under the reaction conditions, for example, in

an ether, for example, diethyl ether, dioxane or tetrahydrofuran, an aromatic hydrocarbon, for example, benzene, toluene or xylene, or a halogenated hydrocarbon, for example, chloroform, carbon tetrachloride or dichloromethane.

A compound of formula XV is then acylated using a compound of formula XIX

$$\begin{array}{l} COR^{15} \\ | \\ COOR^{4} \end{array} \qquad (XIX)$$

in which $R^4$ is as defined above and $R^{15}$ represents a group that can be displaced by the azetidinone nitrogen in the compound of formula XV to give a compound of formula IV.

A group $R^{15}$ is, for example, a halogen atom, an imidazolide group, or a mixed anhydride group, for example, a group -OCORk or -OC(O)ORk in which Rk represents a straight or branched chain alkyl group having from 1 to 4 carbon atoms. $R^{15}$ preferably represents a halogen atom, especially a chlorine atom.

The reaction between compound XV and the acylating agent of formula XIX is carried out in the presence of a base, for example, a tertiary amine, preferably a trialkylamine (each alkyl moiety having from 1 to 4 carbon atoms and having a straight or branched chain, the three alkyl moieties being the same or different), and especially triethylamine or ethyldiisopropylamine; or pyridine or a substituted pyridine, for example, 4-dimethylaminopyridine. Particularly preferred bases are triethylamine and ethyldiisopropylamine. A mixture of two or more bases may be used, and a base may be polymer-bound.

In addition to an organic base as described above, it is preferable to include an acid binding agent in the reaction mixture. Examples of acid binding agents are alkali metal and alkaline earth metal carbonates and bicarbonates, and organic epoxides, for example, propylene

oxide. Calcium carbonate is a commonly-used binding agent.

In general, for each mole of compound XV there is used from 1 to 2 moles of the acylating agent XIX, from 1 to 4 moles of an amine base (or a total of from 1 to 4 moles of a mixture of amine bases), and from 0 to 10 moles of the acid binding agent, if present.

The compound of formula XV and the acylating agent of formula XIX are generally reacted in a solvent or diluent that is inert to the reaction, for example, in a halogenated hydrocarbon, for example, dichloromethane or chloroform, in an oxygenated hydrocarbon,for example, an ether, for example, tetrahydrofuran, dioxane or diethyl ether, or in an aromatic hydrocarbon, for example, benzene or toluene. The reaction is generally carried out at a temperature within the range of from -20 to +60°C, preferably from 0 to 20°C.

Compound IV, the product of the acylation reaction, may be isolated if desired, but is generally reacted without further purification with a trivalent organophosphorus reagent to give a compound of formula III or to give a compound of formula Ia directly.

Compound IV may also be obtained by oxidation of a compound formula XXVII

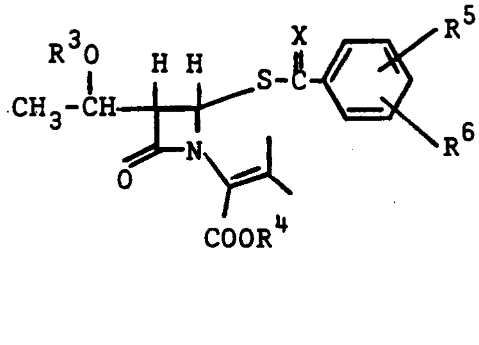

XXVII

in which $R^3$, $R^4$, $R^5$, $R^6$ and X are defined as above.

A suitable oxidising agent is ozone or a higher oxide of a metal for example a higher oxide of a metal selected from one of Groups Ia, Vb, VIb, VIIb or VIII of the periodic table of the elements (see the CRC Handbook of Chemistry and Physics, 52nd Ed., CRC Press, Cleveland, 1971), for example osmium tetroxide, ruthenium tetroxide, an alkali metal peroxide, for example potassium superoxide; a periodate, for example an alkali metal periodate, for example sodium periodate; or an alkali metal permanganate, for example potassium permanganate. A combination of two or more oxidising agents, for example selected from those particularly described above, may also be used. The reaction is generally carried out at a temperature within the range of from -80 to +50°C. An inert solvent or diluent may also be used, for example water; a halogenated hydrocarbon, for example dichloromethane; or acetonitrile. Particularly preferred conditions are ozone in dichloromethane at a temperature of -80 to 0°C, and potassium permanganate in water at a temperature within the range 0° to +40°C.

A compound of formula XXVII may be obtained by reacting a compound of formula XXVIII

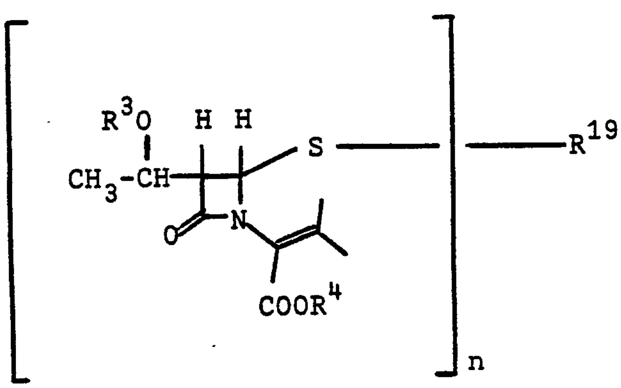

XXVIII

in which n, $R^3$, $R^4$ and $R^{19}$ are defined as above, with a compound of formula VII in an inert solvent or diluent, for example an ether, for example diethyl ether, tetrahydrofuran or dioxane; a halogenated hydrocarbon, for example chloroform or dichloromethane; acetonitrile; an ester, for example ethyl acetate; or an aromatic hydrocarbon, for example toluene. The reaction is generally carried out at a temperature within the range -80° to +60°C, preferably from -40 to 20°C. Optionally a base may also be used: examples are triethylamine, pyridine, and 4-dimethylaminopyridine.

Some compounds of formula XXVIII are known (M. Alpegiani et al, J. Amer. Chem. Soc., 107, 6398 (1985)), and others may be prepared analogously.

Examples of trivalent organophosphorus reagents which may be used to convert compound IV to compound III or compound Ia are phosphites and phosphoramides, for example, of the following formulae XX and XXI, and phosphines of formula XXII in combination with phosphites of formula XX

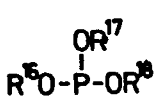
(XX)

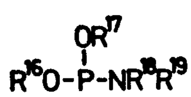
(XXI)

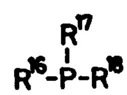
(XXII)

in which formulae $R^{16}$, $R^{17}$, $R^{18}$ and $R^{19}$, which may be the same or different, each represents a straight or branched chain alkyl group having from 1 to 6 carbon atoms or a phenyl group which may be unsubstituted or substituted, for example, by a methyl group, especially a para-methyl group; also cyclic trialkyl phosphites, each alkyl moiety having

followed by page 22

from 1 to 4 carbon atoms, for example, of formula XXIII

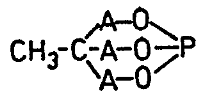

(XXIII)

in which each group A denotes an alkylene group having from 1 to 4 carbon atoms; and also catechol phosphites and catechol dimer phosphites, for example, of the following formulae XXIV and XXV, respectively:

(XXIV) 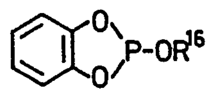 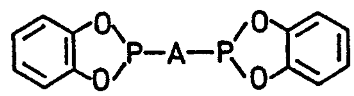 (XXV)

in which $R^{16}$ and A are defined as above. A trivalent organophosphorus compound may be bound to an inert polymer.

A preferred trivalent organophosphorus reagent is a phosphite or phosphoramide of formula XX or XXI respectively, or a combination of a phosphine of formula XXII with a phosphite of formula XX. Particularly preferred are the trialkyl phosphites, and especially trimethyl phosphite and triethyl phosphite.

When the organophosphorus reagent used is a phosphite or phosphoramide, generally from 2 to 3 moles thereof are used per mole of compound IV. When a phosphine is chosen, from 1 to 3 moles are generally used per mole of compound IV, in combination with a phosphite, generally 1 mole thereof. The phosphite is preferably added slowly to a mixture of the phosphine and the compound IV.

As decribed above, a resulting compound of formula III may be isolated and then cyclised to a compound of formula Ia, or a compound of formula IV may be reacted with the organophosphorus reagent and cyclised <u>in situ</u> to give a compound Ia. In the latter case, the reaction may proceed via compound III or via another intermediate.

The cyclisation reaction is generally carried out in a refluxing solvent. When X in compound IV or compound III represents a sulphur atom, preferred solvents are aromatic hydrocarbons, for example, benzene, toluene and xylene, and

halogenated hydrocarbons, for example, dichloromethane, chloroform and 1,2-dichloroethane. When X in compound III or IV represents an oxygen atom, preferred solvents are benzene, toluene and xylene.

In a compound of formula III, it can be seen that the group $-P(Z)_3$ is derived from the trivalent organophosphorus reagent or mixture of reagents used, which reagent or mixture of reagents is preferably selected from the reagents of formulae XX to XXVI described above. As mentioned above, the organophosphorus reagent may be polymer-bound, in which case a resulting compound of formula III will also be polymer-bound.

Compound Ia may be produced from compound XV by an alternative route via compounds XVI, V, and optionally III as shown in Reaction Scheme II.

By this route, a compound of formula XV is reacted with a glyoxylic ester of formula XXVI

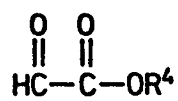

(XXVI)

in which $R^4$ is as defined above, or with a reactive derivative thereof, for example, a hydrate or hemiacetal, a hemiacetal preferably being formed with an alcohol having from 1 to 4 carbon atoms, for example, ethanol.

When a hydrate of a compound of formula XXVI is used, the water formed during the reaction is preferably removed, for example, azeotropically or by use of a dehydrating agent, for example, a molecular sieve.

The reaction is generally carried out in a solvent or diluent that is inert under the reaction conditions,for example, an ether, for example, tetrahydrofuran, dioxane or diethyl ether, an aprotic solvent, for example, dimethylacetamide or dimethylformamide, or an aromatic hydrocarbon, for example, benzene or toluene. A mixture of two or more solvents may be used. The reaction is generally carried out at a temperature within the range of from 0 to 100°C. Preferably from 1 to 2 moles of the glyoxylate derivative

are used per mole of compound XXII.

The resulting compound of formula XVI is obtained as a mixture of the R- and S-isomers at the >CH-OH group. These isomers may be separated, if desired, but the R,S-mixture is generally used for the next reaction.

In the next step, the alcohol of formula XVI is converted into a halide of formula V. The halogenation may be carried out in a conventional manner using an appropriate agent, for example, thionyl chloride or bromide, phosphorus oxychloride or oxybromide, or a phosphorus halide, for example, phosphorus pentachloride or pentabromide, or a mixture of two or more thereof.

The reaction is preferably carried out in the presence of a base, for example, a heterocyclic base, for example, pyridine, 4-dimethylaminomethylpyridine, or lutidine, or a trialkylamine, for example, triethylamine or diisopropylethylamine. The base may be polymer-bound.

The reaction is generally carried out in a solvent, for example, an ether, for example, diethyl ether or dioxane. The reaction temperature is generally within the range of from -40 to +40°C, preferably room temperature and, if desired, the reaction may be carried out under an inert atmosphere.

The resulting compound of formula V is obtained as a mixture of the R- and S-isomers at the >CH-Cl or >CH-Br group. As in the case of compound XVI, the isomeric mixture may be separated into the individual isomers, but generally the R,S-mixture is used.

A compound of formula V may be converted into a compound of formula Ia. This reaction may proceed via a compound of formula III or via another intermediate. If desired, a compound of formula V may be phosphorylated to give a compound of formula III, which may then be isolated and cyclised to give a compound of formula Ia.

A compound of formula V may be converted into a compound of formula Ia or of formula III by reaction at an appropriate temperature with a trivalent organophosphorus reagent, for example, a compound of formula XX, XXI, XXII,

XXIII, XXIV or XXV as described above. (In the present case, a phosphine of formula XX may be used alone.)

The reaction is preferably carried out in the presence of a base, for example, an organic amine, for example, a tertiary amine, for example, pyridine or a pyridine derivative, or a trialkylamine, for example, triethylamine or diisopropylethylamine. The reaction is generally carried out in a solvent that is inert under the reaction conditions, for example, an aromatic hydrocarbon, for example, benzene or toluene, or an ether, for example, diethyl ether, tetrahydrofuran or dioxane.

The reaction temperature is generally within the range of from -10 to 150°C. As mentioned above, the use of lower temperatures, for example, room temperature or below, generally enables a compound III to be isolated, whereas higher temperatures, for example, above room temperature, generally lead to compound Ia directly.

A compound of formula Ia may be produced by a third method, which is also shown in Reaction Scheme II. This method comprises reacting a heavy metal mercaptide of formula VI

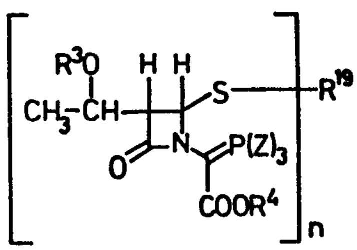

(VI)

in which $=P(Z)_3$, $R^3$ and $R^4$ are defined as above, and $R^{19}$ represents Cu(II), Pb(II) or Hg(II) in which case n represents the integer 2, or $R^{19}$ represents Ag(I), in which case n represents the integer 1, with a compound of formula VII

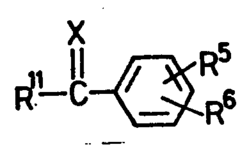

(VII)

in which $R^5$, $R^6$, $R^{11}$ and X are defined as above.

A compound of formula VI in which Z represents a

phenyl group is described in GB 2 042 515A, which also described a process for the production thereof. Other compounds of formula VI may be produced analogously, using the appropriate trivalent organophosphorus reagent.

Compounds VI and VII are generally reacted in a solvent, for example, an ether, for example, diethyl ether or dioxane, an aromatic hydrocarbon, for example, benzene, toluene or xylene, an ester, for example, ethyl acetate, a halogenated hydrocarbon, for example, dichloromethane or chloroform, or an aprotic solvent, for example, dimethylformamide or dimethylacetamide. The reaction temperature is generally within the range of from -40 to 100°C, preferably from 0 to 40°C.

Compound VI may be converted to compound Ia either via compound III or via another intermediate, or compound VI may be converted into compound III, which may then be isolated and converted to compound Ia. Lower temperatures, for example, from -40°C to room temperature, generally enable compound III to be isolated, whereas higher temperatures, for example, from room temperature to 150°C, generally lead to compound Ia.

In all the various intermediates involved in the production of a compound of formula Ia by the procedures described in Reaction Scheme II, the 8-hydroxy group and the 2-carboxy group, when present, are preferably protected, with removal of the protecting groups preferably being delayed until after compound Ia has been formed and, if appropriate, until any conversions of groups $R^5$ and/or $R^6$, and any interconversions of groups $R^1$ and/or $R^2$ have been carried out.

As mentioned above, a final product of formula I may have R- or S-stereochemistry at position 5, 5R-stereochemistry being preferred. The stereochemistry at the 6-position is preferably S.

In process (A), the stereochemistry at position 5 in compound I is predominantly inverse to that at position 4 in the precursor azetidinone compound II. In the preceding reaction steps shown in Reaction Scheme I, halogenation of

compound XI when $R^3$ represents a radical other than a hydrogen atom gives a compound of formula II with a mainly trans relationship between $R^8$ and and the protected hydroxyethyl group at the 8-position. The resulting penem compound of formula I then has a predominantly cis relationship between the ring sulphur atom and the protected hydroxyethyl group. Conversely, halogenation of a compound of formula XI in which $R^3$ represents hydrogen generally yields a compound of formula II with $R^8$ mainly cis to the free hydroxyethyl group, the resulting compound of formula I being predominantly trans.

In processes (B), (C) and (D), the stereochemistry of compound I at position 5 is predominantly determined by the stereochemistry of the attachment of the sulphur atom at position 4 of the azetidinone ring in the precursor compounds III, IV, V and VI. In these compounds, and in the precursor compounds shown in Reaction Scheme II, this sulphur atom is generally almost always trans to the optionally protected hydroxyethyl group at position 3, implying also a trans relationship at the hydrogen atoms at positions 3 and 4. Thus, in Reaction Scheme II, compound I with 6S-stereochemistry will naturally acquire 5R-stereochemistry, with the hydrogen atoms at positions 5 and 6 being trans.

In the formation of a compound of formula I, some degree of thermal equilibration at position 5 does occur, and this effect can be used in the production of compounds having a different relationship between the hydrogen atoms at positions 5 and 6. In particular, the equilibration process is facilitated, for example, by heating, for example, in a solvent or diluent at a temperature generally within the range of from 60 to 150°C, especially at the reflux temperature of the solvent system used.

As mentioned above, $R^5$ and/or $R^6$ may represent a group that can be converted into a group $R^1$ and/or $R^2$, respectively. Such a group $R^5$ and/or $R^6$ may be converted into the appropriate group $R^1$ and/or $R^2$ in any compound in which it appears, such a conversion being part of the

present invention.

Moreover, a group $R^1$ and/or a group $R^2$ may be converted into another group $R^1$ and/or $R^2$. As described above for $R^5$ and $R^6$, such a conversion may be carried out on any of the compounds containing such groups, and such a conversion is also part of the present invention.

The possibility of modifying substituents on the phenyl ring is particularly useful for the production of compounds of formula I having a substituent $R^1$ and/or $R^2$ that is potentially unstable in any of the reactions involved in the production of compound I, or incompatible with any of the reagents used. In such a case, a group $R^5$ and/or $R^6$, or another group $R^1$ and/or $R^2$, may function as a protected form of the desired group, or there may be used a different but stable or compatible group that can be converted into the desired group. The conversion step is, accordingly, carried out after the reaction or reactions in which the desired substituent is potentially unstable or incompatible.

Although a conversion may be carried out, if appropriate, on a precursor of a compound of formula I, it is generally preferable to retain convertible group(s) until after formation of a compound of formula I or formula Ia. (As shown above, a compound of formula Ia is a compound analogous to compound I but having groups $R^5$ and/or $R^6$ that can be converted into groups $R^1$ and/or $R^2$, respectively.)

In a compound of formula I or Ia, a conversion may be carried out before or after the removal of a protecting group from the 8-hydroxy group, and before or after the removal of a 2-carboxy protecting group, having regard to the potential reactivity of a free hydroxy group and of a free carboxy group in the reaction under consideration.

Examples of such conversions (including conversions of radicals forming a part of a larger group) are the following:

(i) -COORd, -COOSiReRfRg or -COO-phenyl to -COOH

(ii) -COOH to $-CONH_2$, -CONHRa or $-CONH(CH_2)_mQ$

(iii) -COOH to $-COOR^4$ or $-CO_2CH_2Q$ or $-CO_2CH_2CH_2Q$ or $-CO_2CH(CH_3)Q$

(iv) -COOH to -CORa

(v) -NHRm or -NRmRn in which Rm and Rn are protecting groups, to $-NH_2$

(vi) $-NH_2$ to

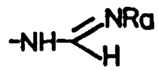 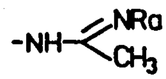 $-NHSO_2Ra$ 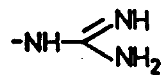

-NHCHO -NHCORa 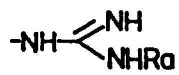

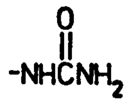 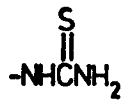 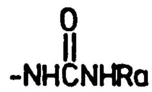 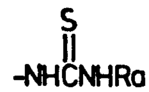

(vii) -CONRaRm, Rm being a protecting group, to -CONHRa

(viii) $-N_3$ to $-NH_2$, which is then optionally converted to a group $R^1$ as described in (v) above,

(ix) halogen to -CN or -COOH

(x) -SRa to -SORa or $-SO_2Ra$

(xi) -CN to $-CH_2NH_2$, which is then optionally converted to a group as defined in (v) above.

(xii) -SORa to $-SO_2Ra$

(xiii) $-NO_2$ to $-NH_2$, which may then converted further as described in (vi) above.

The conversions described above may be used in any appropriate combination, for example, conversion of a halogen atom to a nitrile group which may then be converted into an aminomethyl group, which may be reacted further. In some cases, the group that is converted may not be a group $R^1$ or $R^5$ itself, but may be a moiety forming part of such a group, for example, an $-NH_2$ group may be a group Q as defined above or the terminal moiety of a group Q.

Many of the methods for carrying out such reactions are known <u>per se</u> in the art, for example,

(i) a carboxy protecting group may be removed by conventional methods, for more details, see below;

(ii) a carboxy group may be amidated using an amine and a condensing agent, for example, a carbodiimide, or by reacting an amine with an activated carboxylic acid derivative, for example, an active ester or an anhydride

(symmetrical or asymmetrical), or an acid chloride; (iii) a carboxylic acid group may be esterified using an alcohol and an activated carboxylic acid derivative, for example, an active ester, acid anhydride or acid chloride; (iv) a carboxy group may be converted to a ketone by reaction with an alkyllithium compound, for example, RaLi; (v) and (vii) a protected amine group may be deprotected by conventional methods, for example, as described in McOmie, loc. cit. and in Greene, loc. cit.; (vi) substitution of the amine group by an acyl group or an alkyl or substituted alkyl group as defined in (vi) may be carried out conventionally, for example, an amino group may be acylated with, for example, an acid chloride or an acid anhydride, for example, acetyl chloride or acetic anhydride, or with the appropriate acid derivative; (viii) an azide group may be converted into an amino group by catalytic reduction; (ix) a halide, especially an iodide, may be treated with an organometallic compound, for example, an organolithium compound, especially *t*-butyllithium, the resulting complex being treated with cyanogen to give the -CN group; (x) an alkylthio group may be oxidised, preferably with a carboxylic peracid, especially *m*-chloroperbenzoic acid, to give the corresponding alkylsulphinyl or alkylsulphonyl group; (xi) a cyano group may be converted to an amino group by reduction, for example, using a metal hydride, which amino group is then reacted further as described above (xii) an alkylsulphinyl group may be oxidised to an alkylsulphonyl group as described in (x) above; (xiii) a nitro group may be reduced to an amino group by noble metal catalysed hydrogenation, for example, using platinum or 10% palladium on carbon, c.f. M. Freifelder, Catalytic Hydrogenation in Organic Synthesis, Wiley Interscience, 1978, page 26, and P.N. Rylander, Catalytic Hydrogenation over Platinum Metals, Academic Press, 1967, Chapter 11, and the amino group is then reacted further as

described in (vi) above.

In process (A), and in Reaction Scheme I, it is not generally necessary to protect the 8-hydroxy group, and the special considerations regarding protection and deprotection of the 8-hydroxy group in the formation of a compound of formula II are discussed above. In processes (B), (C) and (D), and in Reaction Scheme II, however, it is generally preferable to protect the 8-hydroxy group, and to remove the protecting group as one of the last steps in the formation of compound I. Accordingly, in compounds of formulae III, IV, V, VI, XIV, XV and XVI, $R^3$ preferably represents a hydroxy protecting group.

As mentioned above, hydroxy protecting groups and methods for their introduction and removal are well known see, for example, McOmie loc cit and Greene loc cit.

Particularly useful hydroxy protecting groups $R^3$ and methods for their removal are described in detail above.

In a compound of formula I, the 8-hydroxy group, if esterified, is preferably esterified with a group that can be removed *in vivo* to give the free hydroxy group, that is to say, an ester group that can be removed under physiological conditions. Examples of suitable esterifying groups are carboxylic acid acyl groups of the formula $R^{20}CO-$ in which $R^{20}$ represents a hydrogen atom or a straight or branched chain alkyl group having from 1 to 6 carbon atoms, especially a methyl, ethyl or *t*-butyl group, or represents a phenyl group or a phenoxyalkyl group in which the alkyl moiety is straight-chained or branched and has from 1 to 4 carbon atoms, and is especially a methylene group.

A non-physiologically removable protecting group $R^3$ is generally removed from a resulting compound of formula I, and may be replaced by a physiologically removable group, if desired. In some cases, a carboxylic acid acyl group $R^3$ may fulfil a protective role during the synthesis of compound I. Such a dual function protective group may be removed, retained or replaced in formula I, as desired.

An ester group at the 8-position may be the only ester group present, or it may be present in addition to an ester group at the 2-carboxyl group. As mentioned above, a physiologically removable group may be present as a protecting group $R^3$ during the formation of a compound of formula I, or it may be introduced at the free 8-hydroxy group of a compound of formula I, after removal of a non-physiologically removable hydroxy protecting group, if present. An esterifying group may be introduced at the 8-hydroxy group by a reaction with an organic acid derivative in known manner. A particularly convenient method is to react a compound of formula I with an activated acid derivative, for example, an acid anhydride in the presence of an organic base, for example, 4-dimethylaminopyridine.

As indicated above, a compound of formula I may be in the form of an ester at the carboxy group at position 2. Such an ester is particularly one that can be converted into the free acid by hydrolysis, photolysis, reduction oxidation or esterase enzyme action. Examples of such esters are those formed with unsubstituted or substituted aliphatic alcohols or phenols having up to 20 carbon atoms in total. In an esterified carboxy group -COOR, the group R may be, for example, a straight or branched chain substituted or unsubstituted alkyl, alkenyl or alkynyl group having up to 18 carbon atoms, preferably up to 8 carbon atoms, and especially up to 6 carbon atoms, for example, a methyl, ethyl, n-propyl, iso-propyl, n-butyl, sec-butyl, iso-butyl, tert-butyl, n-pentyl, n-hexyl, allyl or vinyl group. An aliphatic group R, especially a methyl or ethyl group, may be substituted, for example, by an acyloxy group (further details of such groups are given below); by an aminoalkanoyloxy group; by an optionally substituted amino group; or, in the case of a methyl group, by one or more unsubstituted or substituted phenyl groups. A phenyl group, either as a phenol or as a substituent of a methyl group, may be substituted, for example, by one or

more substituents, selected especially from methoxy and nitro groups and halogen atoms. Examples of phenyl substituted-aliphatic groups, are benzyl, nitrobenzyl, methoxybenzyl, dimethoxybenzyl, benzhydryl and trityl groups.

As indicated above, an ester group is especially one that can be removed by hydrolysis, photolysis, oxidation, reduction or enzyme action, or two or more of these methods may be used, for example, reduction followed by hydrolysis. A group R that can be removed readily without substantial degradation of the rest of the molecule is particularly useful as a carboxy protecting group $R^4$. Examples of esters that are readily split by reduction are trichloroethyl esters, and phenyl-substituted-methyl esters, which may be unsubstituted or substituted, for example, benzyl p-nitrobenzyl, benzhydryl and trityl esters.

Reduction of an ester, for example, a phenyl substituted-methyl ester, for example, a p-nitrobenzyl ester, may be carried out using hydrogen and a metal catalyst, for example, a noble metal catalyst, for example, platinum, palladium or rhodium, which catalyst may be supported, for example, on charcoal or kieselguhr. Such reductions may be carried out in the presence of a salt forming agent, for example, sodium or potassium bicarbonate, if it is desired to form directly a salt at the 2-carboxylic acid group of formula I.

Alternatively, a p-nitrobenzyl ester may be converted into the corresponding free acid by a two-step method, with an initial reduction of the nitro group followed by hydrolysis. The nitro group may be reduced by noble metal catalysed hydrogenation, for example, using platinum, or palladium on carbon, or by a metal reducing agent, for example, zinc in acetic acid. Other metal reducing agents are, for example, aluminium amalgam, and iron and ammonium chloride, see for example, British Patent Specification No. 1,582,960. Reduction of the nitro group is followed by hydrolysis which may occur *in situ* during reduction of

the nitro group or which may be carried out subsequently by treatment with an acid or a base.

An o-nitrobenzyl ester may be converted into the corresponding free acid by photolysis.

Certain ester groups may be split off by base hydrolysis, for example, acetylmethyl, acetoxymethyl and phenoxyethyl esters.

Some ester groups may be cleaved by oxidative hydrolysis, for example, the dimethoxybenzyl group.

Other cleavable esters include, for example, silyl esters, for example, trialkylsilyl and trialkylsilyalkyl esters, in which alkyl moieties have, independently, from 1 to 4 carbon atoms, for example, trimethylsilyl and trimethylsilylethyl esters.

In the above process, removal of esterifying groups by oxidative and reductive processes may be achieved electrochemically.

There may be used an esterifying group that is removable under physiological conditions, that is to say, the esterifying group is split off in vivo to give the free acid or a carboxylate, for exmaple, an acyloxymethyl or acyloxyethyl ester having from 2 to 12 carbon atoms in the acyl moiety, for example, an acetoxymethyl, 1'-(acetoxy)ethyl or pivaloyloxymethyl ester, a 5-methyl-1,3-dioxalen-2-on-4-yl-methyl ester, an aminoalkanoyloxymethyl ester having from 2 to 12 carbon atoms in the alkanoyl moiety, for example, a glycyloxymethyl, L-valinyloxymethyl or L-leucyloxymethyl ester, or a phthalidyl ester, or a 1'-(alkoxycarbonyloxy)ethyl ester, for example, a 1'-(methoxycarbonyloxy)ethyl or 1'-(ethoxycarbonyloxy)ethyl ester, or an optionally substituted 2-aminoethyl ester, for example, a 2-diethylaminoethyl or 2-(1-morpholino)-ethyl ester.

Preferred esters are the p-nitrobenzyl, phthalidyl, pivaloyloxymethyl, ethoxycarbonyloxymethyl, 5-methyl-dioxalen-2-on-4-yl-methyl, acetylmethyl, acetoxymethyl, 1'-(acetoxy)ethyl, 1'(acetyl)ethyl and 1'(ethoxycarbonyloxy)ethyl esters.

An ester at any position in a compound of formula I or of any other free acid described herein, may be prepared

by reaction of the appropriate free acid or activated derivative thereof with an alcohol, a phenol or a reactive derivative thereof. The reaction is preferably carried out under mild conditions in order to prevent rupture of the ring system, for example, under neutral or mild acidic or basic conditions, and at temperatures within the range of from -70 to +35°C.

An ester derived from an alcohol may also be produced by reaction of a reactive derivative of the alcohol, for example, a halide, for example, a chloride, bromide or iodide, or hydrocarbonsulphonyl derivative, for example, a mesyl or tosyl ester, with a salt of an acid of formula I or of another free acid described herein, for example, an alkali or alkaline earth metal salt, for example, a lithium, sodium, potassium, calcium or barium salt, or an amine salt, for example, a triethylammonium salt. The reaction is preferably carried out in a substituted sulphoxide or amide solvent, for example, in dimethyl sulphoxide, dimethylformamide, or hexamethylphosphoramide or, alternatively, an ester may be prepared by reaction of an alcohol or phenol with the acid, for example, in an activated form, for example, in the presence of a condensing agent, for example, dicyclohexylcarbodiimide.

The present invention also provides salts of those compounds of formula I that have salt-forming groups, especially the salts of a free acid of formula I and acid addition salts of compounds of formula I having a basic group. The salts are especially physiologically tolerable salts, for example, alkali metal and alkaline earth metal salts, for example, sodium, potassium, lithium, calcium, and magnesium salts, ammonium salts, and salts with organic amines; also physiologically tolerable acid addition salts. These may be formed with a suitable inorganic or organic acid, for example, hydrochloric acid, sulphuric acid, or an organic carboxylic or organic sulphonic acid, for example, p-toluene-sulphonic acid.

A salt of a free acid of formula I may be produced by reacting the free acid with the appropriate base in a solvent, preferably under those conditions under which

the salt precipitates. A preferred base is potassium 2-ethylhexanoate.

A salt may be produced directly from an ester by splitting off the ester group under suitable reaction conditions, for example, catalytic reduction of an ester, for example, a p-nitrobenzyl ester, in an aqueous/organic solvent, for example, comprising water and ethyl acetate, dioxane or tetrahydrofuran, in the presence of a metal salt, especially a metal bicarbonate, for example, in an equivalent amount or in a slight excess, yields the salt directly.

When an acidic centre and a basic centre are both present in a compound of formula I, the compound may exist in zwitterionic form.

Protecting groups may be introduced or removed at any appropriate point in the reactions involved in the production of a compound of formula I.

At any stage in the production of a compound of formula I, a compound produced may be isolated from the reaction mixture in which it was prepared and, if desired, purified by the appropriate techniques used for the purification of organic compounds, for example, chromatography and crystallisation.

As indicated above, various intermediates may be produced in the form of mixtures of isomers of various kinds. Such mixtures may be separated or resolved at any stage, or an isomeric mixture may be used *per se* for subsequent reactions.

A compound of formula I may have the R or S stereochemistry independently at positions 5, 6 and 8. Any mixture of two or more isomeric forms may be resolved, if desired, or a compound of formula I can be used in the form of an isomeric mixture. The preferred stereochemistry at position 5 in compound I is generally R, corresponding to that in naturally occurring penicillins and cephalosporins, at position 6 is S and at position 8 is R.

Compounds of formula I possess excellent activity

against gram positive bacteria and gram negative bacteria. Moreover, the compounds of formula I and salts thereof show activity against these organisms in the presence of β-lactamase enzymes produced by both gram positive organisms, for example, Staphylococcus aureus and gram negative organisms, for example, Enterobacter cloacae, thus indicating resistance to these enzymes.

Compounds of formula I are also inhibitors of β -lactamase enzymes.

The compounds of formula I and physiologically tolerable salts thereof may be used in humans and other animals to treat, for example, bacterial infections caused by both gram positive and gram negative bacteria, for example, Staphylococcus aureus, Streptococcus pyogenes, Escherichia coli, Bacillus subtilis and Proteus morganii, some strains of which are resistant to conventional penicillin therapy.

It has been found that compounds of formula I (and esters thereof at the 2-carboxylic acid group) having an esterified hydroxy group at the 8-position also have antibacterial and/or β-lactamase inhibiting properties, in particular since the 8-ester group can be cleaved in vivo by esterases. In addition, esterification at the 8-hydroxy group can enhance the degree of absorption on oral administration.

Compounds of the present formula I have certain advantages with respect to antimicrobial activity compared with the corresponding unsubstituted phenyl and also the 4-chloro-, 4-fluoro-, 4-methoxy-, 4-methylthio- and 3-methylthiophenyl compounds for example, 5R,3-(3-aminocarbonylphenyl)-6S-(1R-hydroxyethyl)-7-oxo-4-thia-1-azabicyclo[3.2.0]hept-2-ene-2-carboxylate and the corresponding (4-aminocarbonylphenyl), (3-formylaminophenyl) and (4-formylaminophenyl) compounds have greater antibacterial activity against a variety of microorganisms, particularly β-lactamase producing organisms than do the above comparision compounds, as shown in the Table below:

..... followed by pages 38a-38d

The antimicrobial activity was determined by measuring the Minimum Inhibitory Concentration (MIC). The MIC of the compounds was determined by a standard test, the "agar dilution test" according to Lorian (Antibiotics in Laboratory Medicine, Williams and Wilkins, Baltimore/London 1980) as follows:

A two-fold decreasing concentration series of each compound was prepared in Petri dishes containing 15 ml of Mueller Hinton agar (Difco). One Petri dish containing only the Mueller Hinton agar served as control for bacterial growth. Each Petri dish was inoculated with a multi-point inoculator (Denley), which transferred 0.6µl of a 1:100 diluted 18 hours culture of the appropriate test bacterium. After 16 to 18 hours of incubation at 37°C the Petri dishes were examined for growth of bacteria. The lowest concentration of the compound which causes complete inhibition of growth is taken as the MIC except that the growth of a single colony or a haze is not taken as evidence of growth.

Table of antimicrobial activity (mg/l)

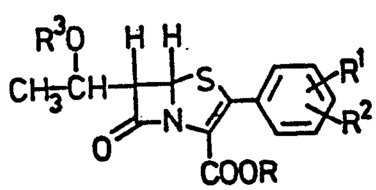

In all the compounds in the Table below, $R^3$ is hydrogen, $R^2$ is hydrogen, and the stereochemistry is 5R, 6S, 8R

| Organism | $R^1$ = | H | 4-F | 4-Cl | 4-NHCHO | 3-$CONH_2$ |
|---|---|---|---|---|---|---|
| | R = | H | K | K | K | Na |
| Str.pyogenes 77A | | <0.05 | <0.05 | <0.05 | <0.013 | 0.013 |
| Str.pyogenes 308A | | <0.05 | <0.05 | <0.05 | <0.013 | 0.006 |
| Str.faecium D | | 1.56 | 3.13 | 1.56 | 0.78 | 1.56 |
| Staph.aureus SG511 | | 0.1 | 0.19 | 0.05 | 0.05 | 0.05 |
| Staph.aureus 285 | | 0.19 | 0.19 | 0.1 | 0.1 | 0.1 |
| Staph.aureus 503 | | 0.1 | 0.19 | 0.1 | 0.1 | 0.1 |
| Bac.subt. ATCC6633 | | 0.1 | 0.1 | 0.05 | 0.025 | 0.05 |
| E.coli 055 | | 0.78 | 1.56 | 3.13 | 0.19 | 0.1 |
| E.coli DCO | | 3.12 | 25 | 25 | 0.78 | 0.39 |
| E.coli DC2 | | 0.39 | 1.56 | 0.39 | 0.19 | 0.1 |
| E.coli TEM | | 1.56 | 6.25 | 12.5 | 0.39 | 0.19 |
| E.coli 1507E | | 1.56 | 6.25 | 12.5 | 0.39 | 0.19 |
| E.coli KN126 | | 3.12 | 6.25 | 12.5 | 0.39 | 0.1 |
| Kl.aerog. 1082E | | 0.78 | 1.56 | 0.78 | 0.19 | 0.1 |
| Kl.aerog. 1522E | | 1.56 | 12.5 | 12.5 | 0.39 | 0.19 |
| Ent.cloacae P99 | | 12.5 | 25 | 50 | 6.25 | 1.56 |
| Ent.cloacae 1321E | | 1.56 | 3.13 | 6.25 | 0.39 | 0.1 |
| Salm.typh. MZII | | 0.78 | 3.13 | 6.25 | 0.39 | 0.19 |

| Organism | $R^1$= | 4-$SCH_3$ | 3-$SCH_3$ | 4-$OCH_3$ | 3-NHCHO | 4-$CONH_2$ |
|---|---|---|---|---|---|---|
| | R = | K | K | K | H | H |
| Str.pyogenes 77A | | <0.05 | <0.05 | <0.05 | <0.025 | 0.013 |
| Str.pyogenes 308A | | <0.05 | <0.05 | <0.05 | <0.025 | 0.013 |
| Str.faecium D | | 3.13 | 1.56 | 3.13 | 1.56 | 1.56 |
| Staph.aureus SG511 | | (ND) | 0.1 | 0.1 | 0.1 | 0.05 |
| Staph.aureus 285 | | 0.19 | 0.19 | 0.19 | 0.19 | 0.1 |
| Staph.aureus 503 | | 0.19 | 0.19 | 0.19 | 0.19 | 0.1 |
| Bac.subt. ATCC6633 | | 0.1 | 0.05 | 0.1 | 0.1 | 0.05 |
| E.coli 055 | | 6.25 | 3.13 | 3.13 | 0.39 | 0.19 |
| E.coli DCO | | 50 | 12.5 | 25 | 1.56 | 0.78 |
| E.coli DC2 | | 0.78 | 0.39 | 0.78 | 0.39 | 0.39 |
| E.coli TEM | | 25 | 6.25 | 12.5 | 0.78 | 0.39 |
| E.coli 1507E | | 25 | 6.25 | 12.5 | 0.78 | 0.39 |
| E.coli KN126 | | 12.5 | 6.25 | 12.5 | 0.39 | 0.39 |
| Kl.aerog 1082E | | 0.78 | 0.39 | 0.78 | 0.39 | 0.39 |
| Kl.aerog 1522E | | 25 | 6.25 | 12.5 | 0.39 | 0.39 |
| Ent.cloacae P99 | | 50 | 50 | 100 | 12.5 | 6.25 |
| Ent.cloacae 1321E | | 12.5 | 6.25 | 12.5 | 0.39 | 0.39 |
| Salm.typh. MZII | | 12.5 | 6.25 | 6.25 | 0.39 | 0.39 |

ND implies not determined

The present invention accordingly provides a pharmaceutical preparation which comprises a compound of formula I, or a physiologically tolerable salt thereof, or a mixture of two or more such substances as active ingredient, in admixture or conjunction with a pharmaceutically suitable carrier. The preparation may also comprise one or more other pharmaceutically active substances, for example another antibacterial substance, especially one having a β-lactam ring. The preparations may be in a form suitable for enteral or parenteral administration, for example, for oral, intravenous or intramuscular administration, for example, as tablets, capsules, syrups, or sterile injectable or infusion solutions. The preparations are advantageously in unit dosage form and preferably comprise from 10 to 2000 mg of the active ingredient per unit dose. The daily dosage of the active ingredient is generally from 20 to 8000 mg, in divided doses, generally up to 4 doses.

The invention also provides the use of a compound of formula I or a physiologically tolerable ester or salt thereof for the manufacture of a medicament for the treatment of bacterial infections.

The invention further provides a method of treating mammals, especially humans, to combat a bacterial infection, which comprises administering to the mammal a compound of formula I or a physiologically tolerable ester or salt thereof.

The invention further provides a pharmaceutical preparation which comprises an active ingredient as defined above, in unit dosage form.

The invention also provides a pharmaceutical preparation which comprises an active ingredient as defined above, or a physiologically tolerable salt thereof or a mixture of two or more such substances, and one or more further pharmaceutically active substances, in unit dosage form. Unit dosages are preferably as described above.

Compounds of formula I are also useful in the production of the antibacterial active compounds.

Particularly interesting compounds of formula I are

(i) 3-(3-aminocarbonylphenyl)-6-(1-hydroxyethyl)-7-oxo-4-thia-1-azabicyclo[3.2.0]hept-2-ene 2-carboxylic acid;

(ii) 3-(4-aminocarbonylphenyl)-6-(1-hydroxyethyl)-7-oxo-4-thia-1-azabicyclo[3.2.0]hept-2-ene 2-carboxylic acid;

(iii) 3-(3-formylaminophenyl)-6-(1-hydroxyethyl)-7-oxo-4-thia-1-azabicyclo[3.2.0]hept-2-ene 2-carboxylic acid;

(iv) 3-(4-formylaminophenyl)-6-(1-hydroxyethyl)-7-oxo-4-thia-1-azabicyclo[3.2.0]hept-2-ene 2 carboxylic acid.

Of these compounds, the 3-aminocarbonylphenyl compound (i) and the 4-formylaminophenyl compound (iv) are particularly preferred.

In each of compounds (i) to (iv) above, 5R,6S,8R-stereochemistry is preferred. The compounds may be in the form of esters or salts as described above.

The present invention also provides compounds of formulae II, III, IV, V, IX, X, XI, XV and XVI.

The following Examples illustrate the invention, but are not limiting.

In the Examples, temperatures are given in degrees Celcius, and ratios of solvents are calculated by volume.

...followed by page 39

Example 1

4-Nitrobenzyl 3-(4-cyanophenyl)-2-{3S-[1R-(dimethyl(2-methylprop-2-yl)silyloxy)ethyl]-4R-ethylthioazetidin-2-on-1-yl}-3-hydroxypropenoate

A mixture of 4-nitrobenzyl 2-{3S-[1R-(dimethyl(2-methylprop-2-yl)silyloxy)ethyl]-4R-ethylthioazetidin-2-on-1-yl}acetate (9.65g) and 4-cyanobenzoyl chloride (3.97g) in tetrahydrofuran (100ml) was cooled to $-40^{o}$ and treated with a solution of lithium hexamethyldisilazide [prepared from n-butyl lithium in tetrahydrofuran (1.6M, 28.10ml) and hexamethyldisilazane (10.46ml)]. The mixture was stirred for 1 hour then glacial acetic acid (4.80g) was added. Evaporation gave a residue which was partitioned between ethyl acetate and water. The organic layer was separated, washed with saturated aqueous sodium bicarbonate and brine and evaporated. Chromatography of the resulting oil with ethyl acetate-hexane mixtures over silica gel afforded the title compound as a brown gum (10.18g) whose $^{1}H$ n.m.r. spectrum showed it was a complex mixture of E and Z isomers and the corresponding keto-tautomer.

$\delta$ ($CDCl_3$) inter alia 3.02 (1H,m) 4.26 (1H,m), 5.62 (1H,d,J = 1.5Hz).

Example 2

4-Nitrobenzyl 3-(4-cyanophenyl)-2-{3S-[1R-(dimethyl(2-methylprop-2-yl)silyloxy)ethyl]-4R-ethylthioazetidin-2-on-1-yl}-3-(methylsulphonyloxy)propenoate

A solution of 4-nitrobenzyl 3-(4-cyanophenyl)-2-{3S-[1R-(dimethyl(2-methylprop-2-yl)silyloxy)ethyl]-4R-ethylthioazetidin-2-on-1-yl}3-hydroxypropenoate (4.04g) in dichloromethane (100ml) was cooled to $-60^{o}$, treated with triethylamine (1.00g) followed by methanesulphonyl chloride (1.13g) and stirred for 1

hour. The solution was washed successively with 0.5M aqueous hydrochloric acid, saturated aqueous sodium bicarbonate and brine, and evaporated. The resulting gum was chromatographed with ethyl acetate-hexane mixtures over silica gel to give the product as a pale yellow foam (2.29g) as a mixture of E and Z isomers.

$\delta$ ($CDCl_3$) 0.00-0.01 (6H,m); 0.96-1.06 (9H,2xs); 2.16-2.46 (6H,m); 2.50 and 2.77 (2H,2q,J = 8.4Hz); 3.08 (3H,2xs); 3.10 and 3.40 (1H,2xdd,J= 2.6 and 3.0Hz); 4.26 and 4.43 (1H,m); 5.07 and 5.40 (2H,2xAB,Jgem=12.9Hz); 7.23-7.27 (1H,m); 7.56-8.14 (5H,m); 8.21-8.27 (2H,2xd,J = 8.9Hz).

Example 3

4-Nitrobenzyl 3-(4-cyanophenyl)-2-[4R-ethylthio-3S-(1R-hydroxyethyl)azetidin-2-on-1-yl]-3-(methylsulphonyloxy)-propenoate.

A solution of 4-nitrobenzyl 3-(4-cyanophenyl)-2-{3S-[1R-(dimethyl(2-methylprop-2-yl)silyloxy)ethyl]-4R-ethylthioazetidin-2-on-1-yl}-3-(methylsulphonyloxy)-propenoate (4.36g) in tetrahydrofuran (60ml) was treated with water (2.0ml) and concentrated (35%) hydrochloric acid (1.94g) and stirred at room temperature for 22 hours. The tetrahydrofuran was removed *in vacuo* and the aqueous residue was neutralised with saturated aqueous sodium bicarbonate. After extraction with ethyl acetate, the organic extract was dried over anhydrous magnesium sulphate and evaporated, and the residue was chromatographed with ethyl acetate-hexane mixtures over silica gel to give the title compound (2.26g) as a mixture of E and Z isomers.

$\delta$ ($CDCl_3$) 0.96-1.30 (6H,m); 2.30 and 2.60 (2H,2xm); 2.10 (3H,s); 2.98 and 3.28 (1H,2xdd,J = 2.6 and 4.3 Hz); 4.15 and 4.30 (1H,2xm); 5.05 and 5.31 (2H,2xAB,J = 13.2Hz); 4.74 and 5.33 (1H,2xd,J = 2.6Hz); 8.22-8.80 (6H,m);

8.10-8.15 (2H,2xd,J = 8.7Hz).

Example 4

4-Nitrobenzyl 3-(4-cyanophenyl)-3-(2,2-dimethylpropanoylthio)-2-[4R-ethylthio-3S-(1R-hydroxyethyl)azetidin-2-on-1-yl]propenoate.

A stirred solution of 4-nitrobenzyl 3-(4-cyanophenyl)-2-[4R-ethylthio-3S-(1R-hyroxyethyl)azetidin-2-on-1-yl]-3-(methylsulphonyloxy)propenoate (1.32g) in acetonitrile (100ml) was treated with potassium 2,2-(dimethyl)thiopropanoate (0.54g). After 4.5 hours the solvent was removed *in vacuo* and the residue was partitioned between ethyl acetate and water. The organic layer was separated, washed with saturated aqueous sodium bicarbonate and brine, dried over anhydrous magnesium sulphate and evaporated. The resulting gum was chromatographed with ethyl acetate-hexane mixtures over silica gel to afford the title compound as a yellow gum (0.57g) which was a mixture of E and Z isomers.

$\delta$ ($CDCl_3$) 1.00-1.40 (15H,m); 2.45 and 2.68 (2H,2xm); 3.15 and 3.37 (1H,2xdd,J=2.9 and 4.3Hz); 4.23 and 4.31 (1H,2xm); 4.93,5.13 and 5.27,5.44 (2H,2xAB,Jgem = 13.0Hz); 4.43 and 5.45 (1H,2xd,J = 2.7Hz); 7.18-7.30 (1H,m); 7.40-7.68 (5H,m); 8.10-8.26 (2H,m).

Example 5

4-Nitrobenzyl 2-[4S-chloro-3S-(1R-hydroxyethyl)-azetidin-2-on-1-yl]-3-(4-cyanophenyl)-3-(2,2-dimethylpropanoylthio)propenoate.

A solution of 4-nitrobenzyl 3-(4-cyanophenyl)-3-(2,2-dimethylpropanoylthio)-2-[4R-ethylthio-3S-(1R-hydroxyethyl)azetidin-2-on-1-yl]propenoate (0.57g) in $CDCl_3$ (15ml) was cooled to $-60^{o}$ treated with a

solution of chlorine in carbon tetrachloride (30mg/ml, 2.5ml) and stirred for 1 hour. The mixture was evaporated and the resulting gum was chromatographed with ethyl acetate-hexane mixtures over silica gel to afford the title compound as a yellow foam (0.34g) as a mixture of E and Z isomers.

δ ($CDCl_3$) 1.10-1.50 (12H,m); 3.47 and 3.58 (1H,2xdd,J = 4.0Hz and 9.5Hz); 4.25 and 4.49 (1H,2xm); 5.04 and 5.44 (2H,2xAB,Jgem=12.7Hz); 5.28 and 6.31 (1H,2xd,J= 4.0Hz); 7.18-7.80 (6H,m); 8.16-8.27 (2H,m).

<u>Example 6</u>

<u>4-Nitrobenzyl 5R,3-(4-cyanophenyl)-6S-(1R-hydroxyethyl)-7-oxo-4-thia-1-azabicyclo[3.2.0]hept-2-ene-carboxylate.</u>

To a solution of 4-nitrobenzyl 2-[4S-chloro-3S-(1R-hydroxyethyl)azetidin-2-on-1-yl]-3-(4-cyanophenyl)-3-(2,2-dimethylpropanoylthio)propenoate (0.04g) in dioxane (5ml) was added a solution of imidazole (7 mg) in water (0.5ml) and the mixture was stirred at room temperature for 3 hours. The reaction mixture was chromatographed directly with ethyl acetate-hexane mixtures over silica gel to afford the title compound as a yellow gum (0.03g).

$\nu_{max}$ ($CDCl_3$) 3400 br, 1790 and 1720 $cm^{-1}$

δ ($CDCl_3$) 1.41 (3H,d,J = 6.3Hz); 3.89 (1H,dd,J = 1.6 and 6.3Hz); 4.32 (1H,m); 5.12,5.33 (2H,AB,Jgem = 13.6Hz); 5.80 (1H,d,J = 1.6Hz); 7.55,7.66 (4H,AA'BB',J = 8.5Hz); 7.50,8.20 (4H,AA'BB', J = 8.5Hz).

<u>Example 7</u>

<u>Potassium 5R,3-(4-cyanophenyl)-6S-(1R-hydroxyethyl)-7-oxo-4-thia-1-azabicyclo[3.2.0]hept-2-ene-2-carboxylate</u>

4-Nitrobenzyl 5R-3-(4-cyanophenyl)-6S-(1R-hydroxyethyl)-7-oxo-4-thia-1-azabicyclo[3.2.0]hept-2-ene-2-carboxylate (0.11g) was dissolved in dioxane (10ml) and mixed with a solution of potassium hydrogen carbonate (24mg) in water (10ml). The mixture was hydrogenated at 375kPa (4 atm ) over 10% palladium-on-charcoal (0.11g) for 1 hour then filtered through Hyflo*. The filtrate was freeze-dried. The residue was dissolved in water, washed with ethyl acetate and freeze-dried to give the title compound as a pale yellow powder (0.08g).

$\delta$ ($D_2O$) 1.31 (3H,d, J = 6.5Hz); 4.02 (1H,m); 4.25 (1H,m); 5.82 (1H,d,J = 1.4Hz); 7.55,7.75 (4H,AA'BB',J = 8.1Hz).

(* Hyflo is a Trade Mark.)

Example 8

4-Nitrobenzyl 3-(3-cyanophenyl)-2-{3S-[1R-(dimethyl(2-methylprop-2-yl)silyloxy)ethyl]-4R-ethylthioazetidin-2-on-1-yl}-3-hydroxypropenoate.

By a method analogous to that described in Example 1, and using 4-nitrobenzyl 2-{3S-[1R-(dimethyl(2-methyl-prop-2-yl)silyloxy)ethyl]-4R-ethylthioazetidin-2-on-1-yl}acetate (14.48g), 3-cyanobenzoyl chloride (5.96g), n-butyl lithium in tetrahydrofuran (1.6M, 42.2ml), hexamethyldisilazane (15.70ml), glacial acetic acid (7.20g) and tetrahydrofuran (200ml), the title compound was obtained as a brown foam (19.47g) whose $^1H$ n.m.r. spectrum showed it was a complex mixture of E and Z isomers and the corresponding keto-tautomer.

$\nu_{max}$ ($CDCl_3$) 1800, 1750 and 1700 $cm^{-1}$

$\delta$ ($CDCl_3$) inter alia 3.04 (1H,m); 4.25(1H,m); 5.60 (1H,d,J = 5.6Hz).

Example 9

4-Nitrobenzyl 3-(3-cyanophenyl)-2-{3S-[1R-(dimethyl(2-methylprop-2-yl)silyloxy)ethyl]-4R-ethylthioazetidin--2-on-1-yl}-3-(methylsulphonyloxy)propenoate.

According to the method of Example 2 and using 4-nitrobenzyl 3-(3-cyanophenyl)-2-{3S-[1R-(dimethyl-(2-[methylprop-2-yl)silyloxy)ethyl]-4R-ethylthioazetidin-2-on-1-yl}-3-hydroxypropenoate (19.47g), triethylamine (4.85g), methanesulphonyl chloride (5.50g) and dichloromethane (150ml) the title compound was obtained as an orange gum (20.47g) comprising a mixture of E and Z isomers.

δ ($CDCl_3$) 0.00-0.10 (6H,m); 0.81-0.91 (9H,2xs); 1.09-1.31 (6H,m); 2.41 and 2.66 (2H,2xq,J = 7.5Hz); 3.06 and 3.08 (3H,2xs); 2.94 and 3.28 (1H,2xdd,J = 2.7 and 4.3Hz); 4.13 and 4.31 (1H,2xm); 5.08 and 5.41 (2H,2xs); 5.01 and 5.43 (1H,2xd,J = 2.7Hz); 7.24-7.28 (1H,m); 7.43-7.74 (4H,m); 7.94-8.28 (3H,m).

Example 10

4-Nitrobenzyl 3-(3-cyanophenyl)-2-[4R-ethylthio-3S-(1R-hydroxyethyl)azetidin-2-on-1-yl]-3-(methylsulphonyloxy)-propenoate.

According to the method of Example 3 and using 4-nitrobenzyl 3-(3-cyanophenyl)-2-{3S-[1R-(dimethyl(2-methylprop-2-yl)silyloxy)ethyl]-4R-ethylthioazetidin-2-on-1-yl}-3-(methylsulphonyloxy)propenoate (20.47g), water (18.2ml), concentrated (35%) hydrochloric acid (18.15g) and tetrahydrofuran (174ml) the title compound was obtained as a mixture of E and Z isomers in the form of an orange foam.

δ ($CDCl_3$) 1.03-1.38 (6H,m); 2.40 and 2.70 (2H,2xm); 3.19 (3H,s); 3.07 and 3.33 (2H,2xdd, J = 2.7 and 4.3Hz); 4.24 and 4.38 (2H,2xm); 5.13 and 5.39 (2H,2xs); 4.75 and 5.40 (1H,2xd,J = 2.7Hz);

7.56-8.25 (8H,m).

Example 11

4-Nitrobenzyl 3-(3-cyanophenyl)-3-(2,2-dimethylpropanoylthio)-2-[4R-ethylthio-3S-(1R-hydroxyethyl)-azetidin-2-on-1-yl]propenoate.

According to the method of Example 4 and using 4-nitrobenzyl 3-(3-cyanophenyl)-2-[4R-ethylthio-3S-(1R-hydroxyethyl)azetidin-2-on-1-yl]-3-(methylsulphonyloxy)-propenoate (5.76g), potassium 2,2-(dimethyl)thiopropanoate (2.34g) and acetonitrile (250ml) the title compound was obtained as an orange gum (2.65g) comprising a mixture of E and Z isomers.

δ ($CDCl_3$) 1.40 (15H,m); 2.50 and 2.75 (2H,2xq,J= 7.5Hz); 3.13 and 3.42 (1H,2xdd, J = 2.7 and 4.3Hz); 4.25 and 4.35 (1H,2xm); 5.04,5.16 and 5.27,5.40 (2H,2xAB,Jgem = 13.0Hz); 5.43 and 5.44 (1H,2xd,J = 2.7Hz); 7.26-7.40 (3H,m); 7.45-7.84 (3H,m); 8.14-8.20 (2H,2xd, J = 8.7Hz).

Example 12

4-Nitrobenzyl 2-[4S-chloro-3S-(1R-hydroxyethyl)-azetidin-2-on-1-yl]3-(3-cyanophenyl)-3-(2,2-dimethylpropanoylthio)propenoate.

According to the method of Example 5 and using 4-nitrobenzyl 3-(3-cyanophenyl)-3-(2,2-dimethylpropanoylthio)-2-[4R-ethylthio-3S-(1R-hydroxyethyl)azetidin-2-on-1-yl]propenoate (1.39g), chlorine in carbon tetrachloride (33mg/ml, 5.76ml) and $CDCl_3$ (30ml), the title compound was obtained as a yellow gum (0.53g) comprising a mixture of E and Z isomers.

$\nu_{max}$ ($CDCl_3$) 1780 and 1700 $cm^{-1}$

δ ($CDCl_3$) 1.10-1.50 (12H,m); 3.36 and 3.58 (1H,2xdd,J = 4.3 and 9.3Hz); 4.20 and 4.50 (1H,2xm); 5.07 and 5.37 (2H,2xAB,Jgem = 13.6Hz); 5.42 and 6.32 (1H,

2xd, J = 4.3Hz); 7.16-7.36 (2H,m); 7.47-7.79 (4H,m); 8.14-8.28 (2H,2xd,J = 8.7Hz).

Example 13

4-Nitrobenzyl 5R,3-(3-cyanophenyl)-6S-(1R-hydroxyethyl)-7-oxo-4-thia-1-azabicyclo[3.2.0]hept-2-ene-2-carboxylate.

According to the method of Example 6 and using 4-nitrobenzyl 2-[4S-chloro-3S-(1R-hydroxyethyl)azetidin-2-on-1-yl]-3-(4-cyanophenyl)-3-(2,2-dimethylpropanoylthio)-propenoate (0.53g), dioxane (10ml), imidazole (95mg) and water (1ml) the title compound was obtained as a yellow gum (0.16g).

$\nu_{max}$ (Nujol mull)* 3440, 2220, 1770, and 1700 $cm^{-1}$

$\delta$ ($CDCl_3$) 1.40 (3H,d,J = 6.3Hz); 3.90 (1H,dd,J= 1.6 and 6.3Hz); 4.31 (1H,m); 5.13,5.32 (2H,AB,Jgem=13.6Hz); 5.80 (1H,d, J = 1.6Hz); 7.43-7.52 (3H,m); 7.66-7.74 (3H,m); 8.18 (2H,d,J = 8.8Hz).

(* Nujol is a Trade Mark).

Example 14

Potassium 5R,3-(3-cyanophenyl)-6S-(1R-hydroxyethyl)-7-oxo-4-thia-1-azabicyclo[3.2.0]hept-2-ene-2-carboxylate.

According to the method of Example 7 and using 4-nitrobenzyl 5R,3-(3-cyanophenyl)-6S-(1R-hydroxyethyl)-7-oxo-4-thia-1-azabicyclo[3.2.0]hept-2-ene-2-carboxylate (0.16g), dioxane (10ml), potassium bicarbonate (35mg) water (10ml) and palladium-on-charcoal (0.16g) the title compound was obtained as a pale yellow powder (0.09g).

$\delta$ ($D_2O$) 1.27 (3H,d, J = 5.8Hz); 3.97 (1H,dd,J = 1.4 and 4.2Hz); 4.26 (1H,m); 5.76 (1H,d, J = 1.4Hz); 7.43-7.97 (4H,m).

Example 15

4-Methylthio-thiobenzoic acid.

To a stirred solution of 4-methylthiobenzoyl chloride (9.82g) in dichloromethane (150ml) at $0^{o}$ was added dropwise pyridine (8.5ml); then after the mixture had been stirred for a further 10 minutes hydrogen sulphide (excess) was bubbled through. After a further 60 minutes stirring the mixture was extracted with saturated aqueous sodium bicarbonate. The aqueous extract was washed with dichloromethane, then acidified to pH2 with dilute hydrochloric acid, and extracted with ethyl acetate. This organic extract was washed with water, and brine, dried over anhydrous sodium sulphate and evaporated *in vacuo* to afford the title compound as a yellow solid (5.72g).

$\delta$ ($CDCl_3$) 2.53 (3H,s); 5.40 (1H,bs); 7.28,7.83 (4H,AA'BB',J = 8.0Hz).

Example 16

3S-{1R-[Dimethyl-(2-methylprop-2-yl)silyloxy]ethyl}-4R-(4-methylthiobenzoylthio)azetidin-2-one.

To a stirred solution of 4-methylthio-thiobenzoic acid (5.7g) in 100ml acetone was added 1M sodium hydroxide until the pH was 8.5 followed by a solution of 4-acetoxy-3R-{1R-[dimethyl-(2-methylprop-2-yl)-silyloxy]ethyl}azetidin-2-one (7.41g) in acetone (100ml). After having been stirred for a further 1 hour, the mixture was partitioned between ethyl acetate and water; the organic phase was washed with water, with saturated aqueous sodium bicarbonate, with water, and with brine, was dried over anhydrous magnesium sulphate, and evaporated *in vacuo* to afford a yellowish solid (11g) chromatography over silica gel, and elution with ethyl acetate-hexane mixtures afforded the title compound (9.9g).

$\nu_{max}$ ($CDCl_3$) 1770 $cm^{-1}$

$\delta$ ($CDCl_3$) 1.23 (3H,d,J = 6.4Hz); 2.52 (3H,s); 3.27 (1H,dd, J = 2.5 and 3.6Hz); 4.30 (1H,m); 5.46 (1H,d, J = 2.5Hz); 6.50 (1H,bs); 7.25 (2H,d,J = 8.7Hz); 7.81 (2H,d,J = 8.7Hz).

Example 17

4-Nitrobenzyl {3S-[1R-(dimethyl(2-methylprop-2-yl)-silyloxy)ethyl]-4R-(4-methylthiobenzoylthio)azetidin-2-on-1-yl}oxoacetate.

To a stirred mixture of 3S-{1R-[dimethyl(2-methylprop-2-yl)silyloxy]ethyl}-4R-(4-methylthiobenzoylthio)azetidin-2-one (9.89g), calcium carbonate (10g) and diisopropylethylamine (6.27ml) at $0^o$ was added a solution of 4-nitrobenzyl chlorooxoacetate (6.99g) in dichloromethane (150ml). After the mixture had been stirred for a further 30 minutes, it was partitioned between water and dichloromethane; the organic layer was washed successively with dilute hydrochloric acid, with water, and with brine, and was dried over anhydrous sodium sulphate. Evaporation of the solvent afforded the title compound (15.6g) as an orange oil.

$\delta$ ($CDCl_3$) 0.09 (6H,s); 0.82 (9H,s); 1.26 (3H,d,J=6.3Hz); 2.53 (3H,s); 3.63 (1H,t, J = 3.0Hz); 4.40 (1H,m); 5.39 (2H,AB, Jgem = 13.0Hz); 6.20 (1H,d,J = 3.0Hz); 7.25 and 7.63 (4H,AA'BB', J = 8.6Hz); 7.56 and 8.21 (4H,AA'BB', J = 8.8Hz).

Example 18

4-Nitrobenzyl 5R,6S-{1R-[dimethyl(2-methylprop-2-yl)-silyloxy]ethyl}-3-(4-methylthiophenyl)-7-oxo-4-thia-1-azabicyclo[3.2.0]hept-2-ene-2-carboxylate.

A mixture of 4-nitrobenzyl {3S-[1R-(dimethyl(2-methylprop-2-yl)silyloxy)ethyl]-4R-(4-methylthiobenzoylthio)-azetidinyl-2-on-1-yl}oxoacetate (15.6g),triethyl phosphite (8.23ml) and *o* -xylene (100ml) were heated under reflux (bath temperature 130°) for 12 hours, and then evaporated *in vacuo* . Chromatography of the residue over silica gel, and elution with diethyl ether-hexane mixtures afforded the title compound (6.87g).

$\nu_{max}$ ($CDCl_3$) 1783 $cm^{-1}$

$\delta$ ($CDCl_3$) 0.06 (3H,s); 0.08 (3H,s); 0.85 (9H,s) 1.27 (3H,d, J = 6.3Hz); 2.48 (3H,s); 3.80 (1H,dd, J = 1.6 and 4.2Hz); 4.30 (1H,m); 5.21 (2H,AB,Jgem = 13.8Hz); 5.68 (1H,d,J= 1.6Hz); 7.18 (2H,d, J = 8.5Hz); 7.41 (4H,m); 8.16 (2H,d,J = 8.8Hz).

Example 19

4-Nitrobenzyl 5R,6S-(1R-hydroxyethyl)-3-(4-methylthiophenyl)-7-oxo-4-thia-1-azabicyclo[3.2.0]hept-2-ene-2-carboxylate.

To a stirred solution of 4-nitrobenzyl 5R,6S-{1R-[dimethyl(2-methylprop-2-yl)silyloxy]ethyl}-3-(4-methylthiophenyl)-7-oxo-4-thia-1-azabicyclo[3.2.0]-hept-2-ene-2-carboxylate (6.87g) in dry tetrahydrofuran (100ml) was added glacial acetic acid (9.37ml) and a 1M solution of tetra-n-butylammonium fluoride in tetrahydrofuran (35ml). After the mixture had been stirred for 16 hours it was partitioned between ethyl acetate and water; the organic layer was successively washed with saturated aqueous sodium bicarbonate, with water, with brine, was dried over anhydrous magnesium sulphate and evaporated *in vacuo* . Chromatography of the crude residue over silica gel, and elution with ethyl acetate-hexane mixtures afforded the title compound as a yellow solid (3.3g).

$\delta$ ($CDCl_3$) 1.19 (3H,d,J = 6.3Hz); 2.38 (3H,s); 3.78 (1H,dd,J =1.5 and 6.3Hz); 4.08 (1H,m); 5.14 (2H,AB,Jgem = 14.0Hz); 5.71 (1H,d,J = 1.5Hz);

7.11,7.31 (4H,AA'BB', J = 8.5Hz); 7.39,8.06 (2H,AA'BB',J = 9.0Hz).

Example 20

4-Nitrobenzyl 5R,6S-(1R-hydroxyethyl)-3-(4-methylsulphinylphenyl)-7-oxo-4-thia-1-azabicyclo[3.2.0]hept-2-ene-2-carboxylate.

To a mixture of 4-nitrobenzyl 5R,6S-(1R-[hydroxyethyl)-3-(4-methylthiophenyl)-7-oxo-4-thia-1-azabicyclo[3.2.0]hept-2-ene-2-carboxylate (306mg), ethyl acetate (10ml) and acetone (10ml) at $-40^o$ was added a solution of 80% 3-chloroperbenzoic acid (154mg) in ethyl acetate (10ml). After the mixture had been warmed over two hours to $0^o$, and this stirred for a further one hour at room temperature, it was partitioned between ethyl acetate and 5% aqueous potassium metabisulphite. The organic layer was washed with saturated aqueous sodium bicarbonate, with water and with brine, and then evaporated to dryness. Chromatography over silica gel, and elution with ethyl acetate-hexane mixtures afforded the title compound (173mg).

$\nu_{max}$ ($CDCl_3$) 1780 $cm^{-1}$

$\delta$ (acetone-$d_6$) 1.19 (3H,d,J = 6.3Hz); 2.59 (3H,s); 3.84 (1H,dd,J = 1.5 and 6.1Hz); 4.09 (1H,m); 5.07,5.22 (2H,AB,Jgem = 13.8Hz); 5.79 (1H,d, J = 1.5Hz); 7.41,8.06 (4H,AA'BB',J = 8.4Hz); 7.56 (4H,s).

Example 21

Potassium 5R,6S-(1R-hydroxyethyl)-3-(4-methylsulphinylphenyl)-7-oxo-4-thia-1-azabicyclo[3.2.0]hept-2-ene-2-carboxylate .

The title compound (140mg) was obtained by a procedure analogous to that described in Example 7 and

using 4-nitrobenzyl 5R,6S-(1R-hydroxyethyl)-3-(4-methylsulphinylphenyl)-7-oxo-4-thia-1-azabicyclo-[3.2.0]hept-2-ene-2-carboxylate (170mg), potassium bicarbonate (35.3mg), 10% palladium on charcoal (170mg), dioxane (5ml) and water (5ml).

δ ($D_2O$) 1.26 (3H,d,J = 6.3Hz); 2.83 (3H,s); 3.96 (1H,dd,J = 1.5 and 5.8Hz); 4.22 (1H,m); 5.76 (1H,d,J = 1.5Hz); 7.56,7.64 (4H,AA'BB', J = 8.2Hz)

Example 22

4-Nitrobenzyl 5R,6S-(1R-hydroxyethyl)-3-(4-methylsulphonylphenyl)-7-oxo-4-thia-1-azabicyclo[3.2.0]hept-2-ene-2-carboxylate.

The title compound (148mg) was obtained by a procedure analogous to that described in Example 20 by using 4-nitrobenzyl 5R,6S-(1R-hydroxyethyl)-3-(4-methylthiophenyl)-7-oxo-4-thia-1-azabicyclo[3.2.0]hept-2-ene-2-carboxylate (1.00g), 80% 3-chloroperbenzoic acid (1.00g), ethyl acetate (35ml) and acetone (15ml).

$\nu_{max}$ ($CDCl_3$) 1780 $cm^{-1}$

δ (acetone-$d_6$) 1.31 (3H,d, J = 6.3Hz); 3.13 (3H,s); 3.98 (1H,dd,J = 1.6 and 6.0Hz); 4.22 (1H,m); 5.26 (2H,AB, Jgem = 13.8Hz); 5.94 (1H,d,J = 1.6Hz); 7.52,7.93 (4H,AA'BB',J = 8.4Hz); 7.73,8.18 (4H,AA'BB',J = 8.7Hz).

Example 23

Potassium 5R,6S-(1R-hydroxyethyl)-3-(4-methylsulphonylphenyl)-7-oxo-4-thia-1-azabicyclo[3.2.0]hept-2-ene-2-carboxylate.

The title compound (91mg) was obtained by a procedure analogous to that described in Example 7 by using 4-nitrobenzyl 5R,6S-(1R-hydroxyethyl)-3-(4-methylsulphonylphenyl)-7-oxo-4-thia-1-azabicyclo[3.2.0]-hept-2-ene-2-carboxylate (148mg), potassium bicarbonate (29.7mg) 10% palladium on charcoal (70mg), water (5ml) and dioxane (5ml).

$\delta$ ($D_2O$) 1.29 (3H,d,J = 6.3Hz); 3.25 (3H,s); 4.02 (1H,dd,J = 1.5 and 5.9Hz); 4.25 (1H,m); 5.82 (1H,d, J = 1.5Hz); 7.64,7.91 (4H,AA'BB',J = 8.5Hz).

Example 24

4-Cyanothiobenzoic acid .

A solution of 4-cyanobenzoyl chloride (1.66g) in dichloromethane (50ml) was treated with pyridine (0.81ml) and saturated with hydrogen sulphide for 30 minutes . The mixture was flushed with nitrogen then evaporated and the residue was partitioned between ethyl acetate and 2M aqueous hydrochloric acid. The organic layer was separated and extracted with saturated aqueous sodium bicarbonate. The basic aqueous extract was acidified with 2M aqueous hydrochloric acid and extracted with ethyl acetate. After drying over anhydrous magnesium sulphate the organic extract was evaporated to give 4-cyanothiobenzoic acid as a yellow solid (1.25g).

$\nu_{max}$ (Nujol mull) 2500, 2230 and 1700 $cm^{-1}$

$\delta$ (acetone-$d_6$) 7.90,8.20 (4H,AA'BB',J = 8.0Hz)

Example 25

4R-(4-Cyanobenzoylthio)-3S-{1R-(dimethyl(2-methylprop-2-yl)silyoxy]ethyl}-azetidin-2-one.

A solution of 4-cyanothiobenzoic acid (0.34g) in acetone (5ml) was treated with 1M aqueous sodium hydroxide (2.1ml) and the mixture was added dropwise to a cooled (0°) solution of 4R-acetoxy-3R-{1R-[dimethyl (2-methylprop-2-yl)silyloxy]ethyl}-azetidin-2-one (0.58g) in acetone-water (70:30) (10ml). The mixture was stirred

for 45 minutes then partitioned between ethyl acetate and brine. The organic phase was separated, dried over anhydrous magnesium sulphate and evaporated. Chromatography of the resulting oil with ethyl acetate-hexane mixtures over silica gel afforded the title compound as a colourless oil (0.56g).

δ ($CDCl_3$) 0.07 (6H,s); 0.89 (9H,s); 1.25 (3H,d,J = 6.2Hz); 3.32 (1H,m); 4.32 (1H,m); 5.50 (1H,d,J = 2.3Hz); 6.46 (1H,bs); 7.80,8.00 (4H,AA'BB',J = 8.5Hz).

Example 26

4-Nitrobenzyl 4R-(4-cyanobenzoylthio)-3S-{1R-[dimethyl(2-methylprop-2-yl)silyloxy]ethyl}-azetidin-2-on-1-yl-oxoacetate.

A solution of 4R-(4-cyanobenzoylthio)-3S-[1R-dimethyl(2-methylprop-2-yl)silyloxyethyl]-azetidin-2-one (0.76g) in dichloromethane (15ml) was cooled ($0^o$) and stirred. Solid calcium carbonate (0.49g) was added followed by diisopropylethylamine (0.51ml). A solution of 4-nitrobenzyl chlorooxoacetate (0.57g) in dichloromethane (6ml) was introduced dropwise and stirring was continued for 30 minutes. The mixture was filtered and the filtrate was washed with 0.1M aqueous hydrochloric acid, dried over anhydrous magnesium sulphate and evaporated to afford the title compound as a yellow foam (1.16g).

δ ($CDCl_3$) 0.03 (3H,s); 0.09 (3H,s); 0.87 (9H,s); 1.26 (3H,d,J = 6.4Hz); 3.63 (1H,m); 4.41 (1H,m); 5.43 (2H,s); 6.17 (1H,d,J = 3.5Hz); 7.57,8.04 (4H,AA'BB',J = 8.8Hz); 7.80,8.23 (4H,AA'BB',J = 8.8Hz).

Example 27

4-Nitrobenzyl 5R,3-(4-cyanophenyl)-6S-{1R-[dimethyl(2-methylprop-2-yl)silyloxy]ethyl}-7-oxo-4-thia-1-azabicyclo[3.2.0]hept-2-ene-2-carboxylate.

A solution of 4-nitrobenzyl 4R-(4-cyanobenzoylthio) 3S-{1R-[dimethyl(2-methylprop-2-yl)silyloxy]ethyl}-azetidin-2-on-1-yl-oxoacetate (1.16g) in xylene (25ml) was warmed to 70° and treated with triethyl phosphite (0.67ml). The mixture was heated to reflux for 1 hour then evaporated and the residue was chromatographed in ether-hexane over silica to afford the product as a yellow oil (0.61g).

$\delta$ ($CDCl_3$) 0.05 (3H,s); 0.09 (3H,s); 0.89 (9H,s); 1.27 (3H,d,J = 6.2Hz); 3.86 (1H,dd,J = 1.6 and 3.8Hz); 4.33 (1H,m); 5.12,5.31 (2H,AB,Jgem = 13.6Hz); 5,79 (1H,d,J = 1.6Hz); 7.50,7.66 (4H,AA'BB',J = 8.5Hz); 7.56,8.18 (4H,AA'BB',J = 8.5Hz).

Example 28

4-Nitrobenzyl 5R,3-(4-cyanophenyl)-6S-(1R-hydroxyethyl)-7-oxo-4-thia-1-azabicyclo[3.2.0]hept-2-ene-2-carboxylate

To a stirred solution of 4-nitrobenzyl 5R,3-(4-cyanophenyl)-6S-{1R-[dimethyl(2-methylprop-2-yl)-silyloxy]-ethyl}-7-oxo-4-thia-1-azabicyclo[3.2.0]hept-2-ene-2-carboxylate (0.29g) in tetrahydrofuran (10ml), cooled to 0°, was added glacial acetic acid (0.30g). A solution of tetra-n-butylammonium fluoride in tetrahydrofuran (1M, 1.47ml) was introduced dropwise and the stirring was continued for 65 hours. Evaporation gave a residue which was dissolved in ethyl acetate, washed with saturated aqueous sodium bicarbonate, water and brine. The organic extract was dried over anhydrous magnesium sulphate, evaporated and

the resulting gum was chromatographed with ethyl acetate-hexane mixtures over silica gel to afford the title compound as a yellow gum (0.11g). This compound had IR and $^1H$ n.m.r. spectra identical to that of the compound prepared in Example 6.

Example 29

4-(2,4-Dimethoxybenzyloxycarbonyl)thiobenzoic acid

A solution of terephthaloyl chloride (20.30g) in dioxane (250ml) was treated dropwise with a solution of 2,4-dimethoxybenzyl alcohol (20.18g) in dioxane (40ml). Triethylamine (16.70ml) was added and the mixture was stirred for 1 hour before being filtered. The filtrate was evaporated and the resulting gum was dissolved in dichloromethane (200ml), treated with pyridine (9.71ml) and saturated with hydrogen sulphide for 40 minutes. The mixture was flushed with nitrogen then evaporated and the residue was partitioned between ethyl acetate and 2M aqueous hydrochloric acid. The organic layer was separated and extracted with saturated aqueous sodium bicarbonate. The basic aqueous extract was acidified with 2M aqueous hydrochloric acid and extracted with ethyl acetate. After drying over anhydrous magnesium sulphate the organic extract was evaporated to afford the title compound as a yellow solid (18.00g).

$\nu_{max}$ ($CDCl_3$) 2580, 1720, and 1700 $cm^{-1}$

$\delta$ ($CDCl_3$) 3.85 (6H,s); 5.39 (2H,s); 6.53 (3H,m); 7.40 (1H,d,J = 9.0Hz); 8.10 (4H,m).

Example 30

4R-[4-(2,4-Dimethoxybenzyloxycarbonyl)benzoylthio]-3S-{1R-[dimethyl(2-methylprop-2-yl)silyloxy]ethyl}-azetidin-2-one.

A solution of 4-(2,4-dimethyloxybenzyloxycarbonyl)-thiobenzoic acid (16.2g) in acetone (150ml) was treated with 1M aqueous sodium hydroxide (62ml) and the mixture was added dropwise to a cooled ($0^o$) solution of 4R-acetoxy-3R-{1R-[dimethyl(2-methylprop-2-yl)silyloxy] ethyl}-azetidin-2-one (11.5g) in acetone water (70:30) (100ml). The mixture was stirred for 45 minutes then partitioned between ethyl acetate and brine. The organic phase was separated, dried over anhydrous magnesium sulphate and evaporated. Chromatography of the resulting oil with ethyl acetate-hexane mixtures over silica gel gave the title compound as a colourless foam (17.40g).

$\nu_{max}(CDCl_3)$ 3420, 1775 and 1720 $cm^{-1}$

$\delta$ $(CDCl_3)$ 0.07 (6H,s); 0.87 (9H,s); 1.24 (3H,d,J = 6.3Hz); 3.82 (6H,s); 4.30 (1H,m); 5.34 (2H,s); 5.49 (1H,d,J = 2.4Hz); 6.49 (2H,m); 6.61 (1H,bs); 7.32 (1H,d,J = 8.9Hz); 7.93,8.13 (4H,AA'BB',J = 8.5Hz).

Example 31

4-Nitrobenzyl 4R-[4-(2,4-dimethoxybenzyloxycarbonyl)-benzoylthio]-3S-{1R-[dimethyl(2-methylprop-2-yl)-silyloxy]ethyl-azetidin-2-on-1yl-oxoacetate.

To a cooled ($0^0$), stirred solution of 4R-[4-(2,4-dimethoxybenzyloxycarbonyl)benzoylthio]-3S-{1R-[dimethyl(2-methylprop-2-yl)silyloxy]ethyl}-azetidin-2-one (5.60g) in dichloromethane (50ml) was added calcium carbonate (2.50g) followed by diisopropylethylamine (2.61ml). A solution of 4-nitrobenzyl chloro-oxoacetate (2.92g) in dichloromethane (10ml) was added dropwise and after 30 minutes the mixture was filtered, washed with 0.1M aqueous hydrochloric acid, dried over anhydrous magnesium sulphate and evaporated to afford the title compound as a pale yellow foam

(7.67g).

δ ($CDCl_3$) 0.01 (3H,s); 0.08 (3H,s); 0.82 (9H,s); 1.27 (3H,d,J= 6.3Hz); 3.64 (1H,dd,J = 2.7 and 3.5Hz); 3.82 (6H,s); 4.39 (1H,dq,J = 2.7 and 6.3Hz); 5.35 (2H,s); 5.40 (2H,s); 6.18 (1H,d,J = 3.5Hz); 6.49 (2H,m); 7.32 (1H,d,J = 8.9Hz); 7.96,8.12 (4H,AA'BB', J = 8.6Hz); 7.57,8.22 (4H,AA'BB',J = 8.7Hz).

<u>Example 32</u>

<u>4-Nitrobenzyl 5R,3-[4-(2,4-dimethyloxybenzyloxycarbonyl)-phenyl]-6S-{1R-[dimethyl(2-methylprop-2-yl)silyloxy]-ethyl}-7-oxo-4-thia-1-azabicyclo[3.2.0]hept-2-ene-2-carboxylate.</u>

A solution of 4-nitrobenzyl 4R-[4-(2,4-dimethyoxy-benzyloxycarbonyl)benzoylthio]-3S-{1R-[dimethyl(2-methylprop-2-yl)silyloxy]ethyl}-azetidin-2-on-1-yl-oxoacetate (7.67g) in xylene (100ml) was warmed to 70° and treated with triethyl phosphite (3.43ml). The mixture was heated to reflux for 4 hours then evaporated and the residue was chromatographed with ether-hexane mixtures over silica gel to afford the product as a yellow foam (4.38g).

δ ($CDCl_3$) 0.07 (3H,s); 0.08 (3H,s); 0.85 (9H,s); 1.27 (3H,d,J = 6.2Hz); 3.82 (7H,m); 4.30 (1H,m); 5.09,5.27 (2H,AB, Jgem = 13.6Hz); 5.34 (2H,s); 5.75 (1H,d,J = 1.6Hz); 6.48 (2H,m); 7.32 (1H,d,J=8.4Hz); 7.40,8.03 (4H,AA'BB',J = 8.6Hz); 7.48,8.13 (4H,AA'BB', J = 8.7Hz).

<u>Example 33</u>

<u>4-Nitrobenzyl 5R,3-(4-carboxyphenyl)-6S-{1R-[dimethyl-(2-methylprop-2-yl)silyl]oxy}ethyl)-7-oxo-4-thia-1-azabicyclo[3.2.0]hept-2-ene-2-carboxylate.</u>

A solution of 4-nitrobenzyl 5R,3-[4-(2,4-dimethoxybenzyloxycarbonyl)phenyl]-6S-{1R-[dimethyl(2-methylprop-2-yl)silyloxy]ethy}-7-oxo-4-thia-1-azabicyclo[3.2.0]hept-2-ene-2-carboxylate (2.02g) in dichloromethane (100ml) containing water (1ml) was treated with 2,3-dichloro-5,6-dicyano-1,4-benzoquinone (DDQ) (1.63g). The mixture was stirred at room temperature for 30 hours then evaporated and the residue was chromatographed with ethyl acetate-hexane mixtures over silica gel to afford the title compound as a brown foam (1.11g).

$\nu_{max}$($CDCl_3$) 3660br, 1790, 1720 and 1700 $cm^{-1}$

$\delta$ (acetone-$d_6$) 0.07 (3H,s); 0.10 (3H,s); 0.86 (9H,s); 1.29 (3H,d,J = 6.3Hz); 4.04 (1H,dd,J = 1.6 and 3.5Hz); 4.36 (1H,dq, J = 3.5 and 6.3Hz); 5.15,5.31 (2H,AB, Jgem = 13.7Hz); 5.91 (1H,d,J=1.6Hz); 7.59,7.98 (4H,AA'BB', J = 8.6Hz); 7.52,8.14 (4H,AA'BB', J = 8.9Hz).

Example 34

4-Nitrobenzyl 5R,3-(4-aminocarbonylphenyl)-6S-{1R-[dimethyl(2-methylprop-2-yl)silyloxy]ethyl}-7-oxo-4-thia-1-azabicyclo[3.2.0]hept-2-ene-2-carboxylate.

To a stirred solution of 4-nitrobenzyl 5R,3-(4-carboxyphenyl)-6S-{1R-[dimethyl(2-methylprop-2-yl)silyloxy]ethyl}-7-oxo-4-thia-1-azabicyclo[3.2.0]hept-2-ene-2-carboxylate (0.41g) in acetonitrile (10ml) was added a solution of 1-hydroxybenzotriazole hydrate (HOBT) (0.19g) in tetrahydrofuran (2ml) followed by 1-(3-dimethylaminopropyl)-3-ethylcarbodiimide hydrochloride (0.20g). After 20 minutes a solution of ammonia in ethanol (39mg $ml^{-1}$, 1ml) was introduced and the mixture was stirred for a further 10 minutes. Evaporation and chromatography of the resulting residue over silica gel and elution with ethyl acetate-hexane mixtures gave the product as a yellow oil

(0.25g).

δ ($CDCl_3$) 0.04 (3H,s); 0.07 (3H,s); 0.85 (9H,s); 1.26 (3H,d,J = 6.3Hz); 3.83 (1H,dd,J = 1.6 and 4.0Hz); 4.29 (1H,dq, J = 4.0 and 6.3Hz); 5.08,5.26 (2H,AB, Jgem = 13.7Hz); 5.75 (1H,d,J = 1.6Hz); 6.32 (2H,bs); 7.49,7.78 (4H,AA'BB',J = 8.3Hz); 7.42,8.13 (4H,AA'BB',J = 8.7Hz).

Example 35

4-Nitrobenzyl 5R,3-(4-aminocarbonylphenyl)-6S-(1R-hydroxyethyl)-7-oxo-4-thia-1-azabicyclo[3.2.0]hept-2-ene-2-carboxylate.

A solution of 4-nitrobenzyl 5R,3-(4-aminocarbonylphenyl)-6S-{1R-[dimethyl(2-methylprop-2-yl)silyloxy]-ethyl}-7-oxo-4-thia-1-azabicyclo[3.2.0]hept-2-ene-2-carboxylate (0.41g) in tetrahydrofuran (20ml) cooled ($0^o$) and treated with glacial acetic acid (0.42g) followed by a solution of tetra-n-butylammonium fluoride in tetrahydrofuran (1M,2.1ml). The mixture was stirred for 45 hours then evaporated and the resulting gum was chromatographed with ethyl acetate over silica gel to afford the title compound as a pale yellow powder (0.13g).

$\nu_{max}$ (Nujol mull) 3460-3260br, 1775, 1705 and 1650 $cm^{-1}$

δ (acetone-$d_6$) 1.31 (3H,d,J = 6.3Hz); 3.94 (1H,dd,J = 1.6 and 6.2Hz); 4.21 (1H,m); 5.18,5.31 (2H,AB,Jgem=13.9Hz); 5.89 (1H,d,J = 1.6Hz); 6.73 (1H,bs); 7.54,7.90 (4H,AA'BB',J = 8.4Hz); 7.46,8.14 (4H,AA'BB',J = 8.9Hz).

Example 36

Potassium 5R,3-(4-aminocarbonylphenyl)-6S-(1R-hydroxyethyl)-7-oxo-4-thia-1-azabicyclo[3.2.0]hept-2-ene-2-carboxylate.

4-Nitrobenzyl 5R,3-(4-aminocarbonylphenyl)-6S-(1R-hydroxyethyl)-7-oxo-4-thia-1-azabicyclo[3.2.0]hept-2-ene-2-carboxylate (0.12g) was dissolved in dioxane (10ml), potassium hydrogen carbonate (25mg) was dissolved in water (10ml) and the two solutions were mixed and hydrogenated at 375KPa (4 atm ) over 10% palladium-on-charcoal (0.12g) for 1 hour. The mixture was filtered through Hyflo and the filtrate was freeze-dried. The residue was dissolved in water, washed with ethyl acetate and freeze-dried to give the title compound as a pale yellow powder (0.09g).

$\delta$ ($D_2O$) 1.30 (3H,d,J = 6.3Hz); 3.99 (1H,dd,J = 1.1 and 5.9Hz); 4.26 (1H,m); 5.79 (1H,d,J = 1.1Hz); 7.50,7.80 (4H,AA'BB',J = 8.3Hz).

## Example 37

4-Nitrobenzyl 5R,3-(4-carboxyphenyl)-6S-(1R-hydroxyethyl)-7-oxo-4-thia-1-azabicyclo[3.2.0]hept-2-ene-2-carboxylate

A solution of 4-nitrobenzyl 5R,3-(4-carboxyphenyl)-6S-{1R-[dimethyl(2-methylprop-2-yl)silyloxy]ethyl}-7-oxo-4-thia-1-azabicyclo[3.2.0]hept-2-ene-2-carboxylate (0.20g) in tetrahydrofuran (10ml) was cooled ($0^o$) and treated with glacial acetic acid (0.20g) followed by a solution of tetra-n-butylammonium fluoride in tetrahydrofuran (1M, 1.02ml). The mixture was stirred for 24 hours then evaporated and the residue was partitioned between ethyl acetate and saturated aqueous sodium bicarbonate. The basic aqueous layer was separated, carefully acidified to pH6 with 2M aqueous hydrochloric acid and extracted with ethyl acetate. Evaporation of the extract afforded the title compound as a brown oil (0.08g).

$\nu_{max}$ (Nujol mull) 1770 and 1700 $cm^{-1}$

$\delta$ (acetone-$d_6$) 1.31 (3H,d,J = 6.3Hz);

3.95 (1H,dd,J = 1.6 and 6.4Hz); 4.21 (1H,m); 5.16,5.30 (2H,AB,Jgem = 13.8Hz); 5.94 (1H,d,J = 1.6Hz); 7.55,7.98 (4H,AA'BB',J = 8.4Hz); 7.47,8.13 (4H,AA'BB', J = 8.8Hz).

## Example 38

**4-Nitrobenzyl 5R,3-(4-aminocarbonylphenyl)-6S-(1R-hydroxyethyl)-7-oxo-4-thia-1-azabicyclo[3.2.0]hept-2-ene-2-carboxylate.**

A solution of 4-nitrobenzyl 5R,3-(4-carboxyphenyl)-6S-(1R-hydroxyethyl)-7-oxo-4-thia-1-azabicyclo[3.2.0]hept -2-ene-2-carboxylate (0.08g) in acetonitrile (10ml) was stirred and treated with a solution of 1-hydroxybenzotriazole (HOBT) (0.04g) in tetrahydrofuran (2ml) followed by 1-(3-dimethylaminopropyl)-3-ethyl-carbodiimide hydrochloride (0.05g). After 1 hour a solution of ammonia in ethanol (50mgml$^{-1}$, 0.5ml) was added and stirring was continued for a further 10 minutes. The mixture was evaporated and the residue dissolved in dichloromethane , washed with 1M aqueous citric acid, saturated aqueous sodium bicarbonate and brine. Evaporation of the organic extract afforded the title compound (0.07g) with properties identical to those of the compound prepared in Example 35.

## Example 39

**4-Nitrobenzyl 5R,3-[4-(2,4-dimethoxybenzyloxycarbonyl)-phenyl]-6S-(1R-hydroxyethyl)-7-oxo-4-thia-1-azabicyclo-[3.2.0]hept-2-ene-2-carboxylate.**

A solution of 4-nitrobenzyl 5R,3-[4-(2,4-dimethoxybenzyloxycarbonyl)phenyl]-6S-{1R-[dimethyl(2-methylprop-2-yl)silyloxy]ethyl}-7-oxo-4-thia-1-azabicyclo[3.2.0] hept-2-ene-2-carboxylate (1.00g) in tetrahydrofuran

(40ml) was cooled ($0^o$) and treated with glacial acetic acid (0.82g) followed by a solution of tetra-n-butylammonium fluoride in tetrahydrofuran (1M, 4.1ml). The mixture was stirred for 24 hours then evaporated and the residue was chromatographed over silica gel and eluted with ethyl acetate-hexane mixtures to give the title compound as a yellow oil (0.27g).

$\delta$ ($CDCl_3$) 1.38 (3H,d, J = 6.3Hz); 3.82 (6H,s); 3.87 (1H,dd,J = 1.6 and 6.3Hz); 4.30 (1H,m); 5.08,5.25 (2H,AB,Jgem = 13.6Hz); 5.33 (2H,s); 5.77 (1H,d,J = 1.6Hz); 6.48 (2H,m); 7.31 (3H,m); 7.45,7.99 (4H,AA'BB',J = 8.5Hz); 8.11 (2H,d,J=8.7Hz).

## Example 40

**4-Nitrobenzyl 5R,3-(4-carboxyphenyl)-6S-(1R-hydroxyethyl)-7-oxo-4-thia-1-azabicyclo[3.2.0]hept-2-ene-2-carboxylate.**

A solution of 4-nitrobenzyl 5R,3-[4-(2,4-dimethoxybenzyloxycarbonyl)phenyl]-6S-(1R-hydroxyethyl)-7-oxo-4-thia-1-azabicyclo[3.2.0]hept-2-ene-2-carboxylate (0.27g) in dichloromethane (20ml) containing water (1ml) was stirred and treated with 2,3-dichloro-5,6-dicyano-1,4-benzoquinone (DDQ) (0.20g). After 24 hours the mixture was evaporated and the residue partitioned between ethyl acetate and water. The organic layer was separated, dried over anhydrous magnesium sulphate, evaporated and the resulting gum was chromatographed over silica gel with ethyl acetate-hexane mixtures to afford the title compound (0.13g) with properties identicial to the compound prepared in Example 37.

## Example 41

**4-Nitrobenzyl 5R,3-{4-[N-(cyanomethyl)aminocarbonyl]-**

phenyl}-6S-{1R-[dimethyl(2-methylprop-2-yl)silyloxy]-ethyl}-7-oxo-4-thia-1-azabicyclo[3.2.0]hept-2-ene-2-carboxylate

To a stirred solution of 4-nitrobenzyl 5R,3-(4-carboxyphenyl)-6S-{1R-[dimethyl(2-methylprop-2-yl)silyloxy]ethyl}-7-oxo-4-thia-1-azabicyclo[3.2.0]hept-2-ene-2-carboxylate (0.36g) in acetonitrile (10ml) was added a solution of 1-hydroxybenzotriazole (HOBT) (0.17g) in tetrahydrofuran (2ml) followed by 1-(3-dimethylamino-propyl)-3-ethylcarbodiimide hydrochloride (0.18g). After 30 minutes aminoacetonitrile hydrochloride (0.17g) was added closely followed by triethylamine (0.26ml), and stirring was continued for a further 30 minutes. The mixture was evaporated, the residue was dissolved in ethyl acetate and washed with 2M aqueous hydrochloric acid, saturated aqueous sodium bicarbonate and brine, and evaporation of the organic phase gave a brown gum which was chromatographed with ethyl acetate-hexane mixtures over silica gel to afford the product as a yellow foam (0.24g).

$\nu_{max}$ (Nujol mull) 3400-3300br, 1790, 1720 and 1665cm $^{-1}$

$\delta$ ($CDCl_3$) 0.04 (3H,s,); 0.06 (3H,s); 0.84 (9H,s); 1.25 (3H,d,J = 6.3Hz); 3.82 (1H,dd,J = 1.6 and 3.9Hz); 4.28 (1H,dq,J = 3.9 and 6.3Hz); 4.35 (2H,d,J = 5.9Hz); 5.08,5.26 (2H,AB,Jgem = 13.6Hz); 5.75 (1H,d,J = 1.6Hz); 6.67 (1H,bt,J = 5.9Hz); 7.51,7.72 (4H,AA'BB',J = 8.5Hz); 7.43,8.12 (4H,AA'BB',J = 8.7Hz).

Example 42

4-Nitrobenzyl 5R,3-{4-[N-(cyanomethyl)aminocarbonyl]-phenyl}-6S-(1R-hydoxyethyl)-7-oxo-4-thia-1-azabicyclo-[3.2.0]hept-2-ene-2-carboxylate.

To a cooled (0$^{o}$) solution of 4-nitrobenzyl 5R,3-{4-[N-(cyanomethyl)aminocarbonyl]phenyl}-6S-{1R-[dimethyl(2-methylprop-2-yl)silyloxy]ethyl}-7-oxo-4-thia-1-azabicyclo[3.2.0]hept-2-ene-2-carboxylate (0.23g) in tetrahydrofuran (50ml) was added glacial acetic acid (0.22g) and a solution of tetra-n-butylammonium fluoride in tetrahydrofuran (1M,1.1ml). The mixture was stirred for 40 hours then evaporated and the residue was chromatographed with ethanol-ethyl acetate mixtures over silica gel to afford the title compound as a pale yellow solid (0.10g).

(acetone-$d_6$) 1.31 (3H,d,J = 6.3Hz); 3.95 (1H,dd,J = 1.7 and 6.2Hz); 4.21 (1H,m); 4.41 (2H,d,J = 3.3Hz); 5.17,5.31 (2H,AB,Jgem = 13.8Hz); 5.90 (1H,d,J = 1.7Hz); 7.57,7.68 (4H,AA'BB',J = 8.5Hz); 7.49,8.15 (4H,AA'BB',J = 8.8Hz); 8.51 (1H,bt,J = 3.3Hz).

## Example 43

**Potassium 5R,3-{4-[N-(cyanomethyl)aminocarbonyl]phenyl}-6S-(1R-hydroxyethyl)-7-oxo-4-thia-1-azabicyclo[3.2.0]-hept-2-ene-2-carboxylate.**

4-Nitrobenzyl 5R,3-{4-[N-(cyanomethyl)-aminocarbonyl]phenyl-6S-(1R-hydroxyethyl)-7-oxo-4-thia-1-azabicyclo-[3.2.0]hept-2-ene-2-carboxylate (0.10g) was dissolved in dioxane (10ml), potassium hydrogen carbonate (19mg) was dissolved in water (10ml) and the two solutions were mixed and hydrogenated at about 375 pKa (4 atmospheres) over 10% palladium-on-charcoal (0.10g) for 1 hour. The mixture was filtered through Hyflo (Trade Mark) and the filtrate was freeze-dried. The residue was dissolved in water, washed with ethyl acetate and freeze-dried to afford the title compound as a pale yellow powder (0.07g).

($D_2O$) 1.30 (3H,d,J = 6.4Hz); 4.00 (1H,dd,J=1.4 and 5.9Hz); 4.25 (1H,m); 4.36 (2H,s,); 5.79 (1H,d,J = 1.4Hz); 7.51 and 7.75 (4H,AA'BB',J = 8.5Hz).

Example 44

4-(Methoxyaminocarbonyl)thiobenzoic acid

To a solution of terephthalyl chloride (7.0g) in dichloromethane (250ml) was added a 25% aqueous solution of methoxyamine hydrochloride (15ml) followed by a solution of triethylamine (12.5ml) in dichloromethane (250ml). After the mixture had been sitrred for 1 hour, pyridine (4.2ml) was added, and then gaseous hydrogen sulphide (excess) was bubbled through over 30 minutes. After it had been stirred for a further 30 minutes the mixture was flushed with nitrogen, and partitioned between ethyl acetate and aqueous citric acid. The organic layer was washed with water and then extracted with saturated aqueous sodium bicarbonate. This aqueous extract was washed with ethyl acetate, and then reacidified to pH2 with 11M hydrochloric acid. Extraction with ethyl acetate, gave giving an organic extract which was then washed with water and with brine, and evaporated *in vacuo*, to afford a residue, which was chromatographed over silica gel. Elution with ethyl acetate-hexane mixtures afforded the title compound (2.0g).

$\delta$ (acetone-$d_6$) 3.80 (3H,s); 7.79-8.30 (4H,m).

Example 45

3S-{1R-[Dimethyl(2-methylprop-2-yl)silyloxy]ethyl}-4R-(4-methoxyaminocarbonylbenzoylthio)-azetidin-2-one

The title compound (1.85g) was prepared by a procedure analogous to that described in Example 16 by using 4-acetoxy-3R-{1R-[dimethyl(2-methylprop-2-yl)-silyloxy]ethyl}azetidinone (21.9g), 4-(methoxyaminocarbonyl)thiobenzoic acid (1.9g), 1M sodium hydroxide (8.7ml) and acetone (10ml).

$\nu_{max}$ (film) 1758, 1665 $cm^{-1}$

δ ($CDCl_3$) 0.10 (6H,s); 0.90 (9H,s); 1.25 (3H,d,J = 6.3Hz); 3.30 (1H,dd,J = 2.4 and 3.7Hz); 3.93 (3H,s); 4.30-4.35 (1H,m); 5.49 (1H,d,J = 2.4Hz); 6.52 (1H,bs); 7.85,7.98 (4H,AA'BB',J = 8.4Hz); 9.13 (1H,bs).

Example 46

4-Nitrobenzyl {3S-[1R-(dimethyl(2-methylprop-2-yl)-silyloxy)ethyl]-4R-[4-(methoxy-(4-nitrobenzyloxycarbonyl-carbonyl)-aminocarbonyl)benzoylthio]-azetidin-2-on-1-yl}oxoacetate.

The title compound (4.1mmol) was prepared by a procedure analogous to that described in Example 17 by using 3S-{1R-[dimethyl(2-methylprop-2-yl)silyloxy]-ethyl}-4R-(4-methoxyaminocarbonylbenzoylthio)azetidin-2-one (1.8g), 4-nitrobenzyl chlorooxoacetate (2.5g), calcium carbonate (1.8g), diisopropylethylamine (2.2ml) and dichloromethane (20ml).

$\nu_{max}$ ($CDCl_3$) 1815 $cm^{-1}$

δ ($CDCl_3$) 0.10 (6H,s); 0.84 (9H,s); 1.27 (3H,d,J = 6.3Hz); 3.64-3.68 (1H,m); 3.90 (3H,s); 4.40-4.43 (1H,m); 5.39-5.44 (4H,m); 6.19 (1H,d,J=3.4Hz); 7.55-8.26 (12H,m).

Example 47

4-Nitrobenzyl 5R,6S-{1R-[dimethyl(2-prop-2-yl)silyloxy]-ethyl}-3-{4-[methoxy-(4-nitrobenzyloxycarbonylcarbonyl)-aminocarbonyl]phenyl}-7-oxo-4-thia-1-azabicyclo[3.2.0]hept-2-ene-2-carboxylate.

The title compound (113mg) was obtained by a procedure analogous to that described in Example 18 by using 4-nitrobenzyl {3S-[1R-(dimethyl(2-methylprop-2-yl)-silyloxy)ethyl]-4R-[4-(methoxy-(4-nitrobenzyloxycarbonyl-carbonyl)-aminocarbonyl)benzoylthio]-azetidin-2-on-1-yl}-oxoacetate (1.25mmol), triethyl phosphite (428μl) and

xylene (5ml).

ν max (film) 1775, 1748 and 1725 $cm^{-1}$

δ ($CDCl_3$) 0.07 (6H,s); 0.83 (9H,s); 1.25 (3H,d,J=6.3Hz); 3.80-3.83 (1H,m); 3.89 (3H,s); 4.30-4.38 (1H,m); 5.07,5.20 (2H,AB,J = 13.6Hz); 5.37 (2H,s); 5.75 (1H,d,J = 1.5Hz); 7.39,7.98 (4H,AA'BB',J = 8.6Hz); 7.45,8.09 (4H,AA' BB',J = 8.3Hz); 7.50,8.22 (4H,AA'BB',J = 8.7Hz).

Example 48

4-Nitrobenzyl 5R,6S-(1R-hydroxyethyl)-3-(4-methoxyamino-carbonylphenyl)-7-oxo-4-thia-1-azabicyclo[3.2.0]hept-2-ene-2-carboxylate.

The title compound (250mg) was obtained by a procedure analagous to that described in Example 19, using 4-nitrobenzyl 5R,6S-{1R-[dimethyl(2-prop-2-yl)-silyloxy]ethyl}-3-{4-[methoxy-(4-nitrobenzyloxycarbonyl-carbonyl)-aminocarbonyl]phenyl}-7-oxo-4-thia-1-azabicyclo[3.2.0]hept-2-ene-2-carboxylate (670mg), acetic acid (570μl), 1M tetra-n-butylammonium fluoride in tetrahydrofuran (2.88ml), and tetrahydrofuran (10ml).

ν max (film) 1788, 1722 $cm^{-1}$

δ ($CDCl_3$) 1.41 (3H,d, J = 6.3Hz); 3.88 (1H,dd, J = 1.6 and 6.4Hz); 3.94 (3H,s); 4.28-4.35 (1H,m); 5.10,5.26 (2H,AB,Jgem = 13.5Hz); 5.79 (1H,d,J=1.6Hz); 7.32,7.98 (4H,AA'BB',J = 8.5Hz); 7.47,8.13 (4H,AA'BB'J = 8.7Hz).

Example 49

Potassium 5R,6S-(1R-hydroxyethyl)-3-(4-methoxyamino-carbonylphenyl)-7-oxo-4-thia-1-azabicyclo[3.2.0]hept-2-ene-2-carboxylate.

The title compound (28mg) was obtained by a procedure analogous to that described in Example 7 by using 4-nitrobenzyl 5R,6S-(1R-hydroxyethyl)-3-(4-methoxyaminocarbonylphenyl)-7-oxo-4-thia-1-azabicyclo [3.2.0]hept-2-ene-2-carboxylate (60mg), 10% palladium-on-charcoal (60mg), potassium bicarbonate (12mg), dioxane (5ml) and water (5ml).

δ ($D_2O$) 1.29 (3H,d, J = 6.4Hz); 3.90 (3H,s); 4.00 (1H,dd,J = 1.5 and 5.9Hz); 4.21-4.28 (1H,m); 5.79 (1H,d, J = 1.5Hz); 7.49,7.97 (4H,AA'BB',J=8.5Hz).

Example 50

4-(Methylaminocarbonyl)thiobenzoic acid.

The title compound (3.1g) was obtained by a procedure analogous to that described in Example 44 by using terephthaloyl chloride (10.0g), dichloromethane (300ml), methylamine hydrochloride (4.2g), triethylamine (17.4ml), pyridine (7.76ml), and excess hydrogen sulphide.

δ (acetone-$d_6$) 2.90 (3H,s); 7.96,8.10 (4H,AA'BB', J = 8.4Hz).

Example 51

3S-{1R-[Dimethyl(2-methylprop-2-yl)silyloxy]ethyl}-4R-(4-methylaminocarbonylbenzoylthio)-azetidin-2-one.

The title compound (1.7g) was obtained by a procedure analogous to that described in Example 16, by using 4-acetoxy-3R-{1R-[dimethyl(2-methylprop-2-yl)-silyloxy]ethyl}-azetidin-2-one (3.0g), 4-(methylaminocarbonyl)thiobenzoic acid (3.0g), 1M sodium hydroxide (15.6ml), and acetone (50ml).

$\nu_{max}$ (film) 1765, 1660 $cm^{-1}$

δ ($CDCl_3$) 0.08 (6H,s); 0.88 (9H,s); 1.23 (3H,d,J = 6.3Hz); 3.03 (3H,d,J = 4.8Hz);

3.28 (1H,dd,J = 2.4Hz and 3.8Hz); 4.28-4.32 (1H,m); 5.47 (1H,d,J = 2.4Hz); 6.37 (1H,bd,J = 4.8Hz); 6.54 (1H,bs); 7.83,7.94 (4H,AA'BB',J = 8.5Hz).

Example 52

4-Nitrobenzyl {3S-[1R-(dimethyl(2-methylprop-2-yl)-silyloxy)ethyl]-4R-[4-(methylaminocarbonyl)-benzoylthio]-azetidin-2-on-1-yl}oxoacetate .

The title compound (2.5g) was obtained by a procedure analogous to that used in Example 17 by using 4-nitrobenzyl chlorooxoacetate (1.17g), 3S-(1R-{dimethyl-(2-methylprop-2-yl)silyloxy}ethyl})-4R-(4-methylaminocarbonylbenzoylthio)-azetidin-2-one (1.7g), calcium carbonate (1.7g), diisopropylethylamine (1.05ml), and dichloromethane (15ml).

δ ($CDCl_3$) 0.09 (6H,s); 0.86 (9H,s); 1.27 (3H,d,J= 6.3Hz); 3.04 (3H,d,J = 4.9Hz); 3.63 (1H,dd,J = 3.4 and 2.7Hz); 4.39-4.43 (1H,m); 5.35,5.43 (2H,AB,Jgem = 13.7Hz); 6.19 (1H,d,J = 3.4Hz); 7.57,7.97 (4H,AA'BB',J = 8.1Hz); 7.86,8.22 (4H,AA'BB',J = 8.7Hz).

Example 53

4-Nitrobenzyl 5R,6S-{1R-[dimethyl(2-methylprop-2-yl)-silyloxy]ethyl}-3-(4-methylaminocarbonylphenyl)-7-oxo-4-thia-1-azabicyclo[3.2.0]hept-2-ene-2-carboxylate.

The title compound (0.66g) was obtained by a procedure analogous to that used in Example 18 by using 4-nitrobenzyl {3S-[1R-(dimethyl(2-methylprop-2-yl)-silyloxy)ethyl]-4R-[4-(methylaminocarbonyl)benzoylthio]-azetidin-2-on-2-yl}oxoacetate (2.5g), triethyl phosphite (1.37ml), hydroquinone (5mg), o-xylene (15ml).

$\nu_{max}$ (film) 1778, 1715 $cm^{-1}$
$\delta$ ($CDCl_3$) 0.082 (6H,s); 0.90 (9H,s); 1.22 (3H,d,J = 6.3Hz); 3.01 (3H,d,J = 4.6Hz); 3.83 (1H,dd, J = 1.5 and 4.2Hz); 4.12-4.19 (1H,m); 5.10,5.27 (2H,AB,Jgem = 13.6Hz); 5.76 (1H,d,J = 1.5Hz); 6.15 (1H,bd,J = 4.6Hz); 7.43,7.71 (4H,AA'BB',J=8.4Hz); 7.46,8.14 (4H,AA'BB',J = 8.7Hz).

Example 54

4-Nitrobenzyl 5R,6S-(1R-hydroxyethyl)-3-(4-methylaminocarbonylphenyl)-7-oxo-4-thia-1-azabicyclo[3.2.0]hept-2-ene-2-carboxylate.

The title compound (175mg) was obtained by a procedure analogous to that used in Example 19 by using 4-nitrobenzyl 5R,6S-{1R-[dimethyl(2-methylprop-2-yl)-silyloxy]ethyl}-3-(4-methylaminocarbonylphenyl)-7-oxo-4-thia-1-azabicyclo[3.2.0]hept-2-ene-2-carboxylate (660mg), glacial acetic acid (660µl), 1M tetra-n-butylammonium fluoride in tetrahydrofuran (3.3ml), and tetrahydrofuran (5ml).

$\nu_{max}$ (KBr) 1770 $cm^{-1}$
$\delta$ (DMSO-$d_6$) 1.17 (3H,d,J = 6.1Hz); 2.78 (3H,d,J = 4.5Hz); 3.96-4.02 (2H,m); 5.20(2H,m); 5.82 (1H,d, J = 1.5Hz); 7.32,7.76 (4H,AA'BB',J=7.2Hz); 7.46,8.10 (4H,AA'BB',J = 8.6Hz); 8.49 (1H,bd,J= 4.0Hz).

Example 55

Potassium 5R,6S-(1R-hydroxyethyl)-3-(4-methylaminocarbonylphenyl)-7-oxo-4-thia-1-azabicyclo[3.2.0]hept-2-ene-2-carboxylate.

The title compound (117mg) was obtained by a procedure analogous to that used in Example 7 by using 4-nitrobenzyl 5R,6S-(1R-hydroxyethyl)-3-(4-methylamino-

carbonylphenyl)-7-oxo-4-thia-1-azabicyclo[3.2.0]hept-2-ene-2-carboxylate (175mg), 10% palladium on charcoal (175mg), potassium bicarbonate (36.2mg), dioxane (5ml) and water (5ml).

$\delta$ ($D_2O$) 1.26 (3H,d, J = 6.4Hz); 2.86 (3H,s); 3.96 (1H,dd, J = 1.3 and 6.0Hz); 4.19-4.26 (1H,m); 5.75 (1H,d, J = 1.3Hz); 7.44,7.64 (4H,AA'BB',J = 8.3Hz).

Example 56

4-(Propylaminocarbonyl)-thiobenzoic acid

The title compound (3.4g) was obtained by a procedure analogous to that used in Example 44 by using terephthaloyl chloride (10.0g), propylamine (4.84ml), triethylamine (10.2ml), pyridine (8.36ml), excess hydrogen sulphide and dichloromethane (300ml).

$\delta$ (acetone-$d_6$) 0.93 (3H,t, J = 7.4Hz); 1.60 (2H,m); 3.30-3.40 (2H,m); 7.96-8.13 (4H,m).

Example 57

3S-{1R-[Dimethyl(2-methylprop-2-yl)silyloxy]ethyl}-4R-(4-propylaminocarbonylbenzoylthio)azetidin-2-one.

The title compound (250mg) was obtained by a procedure analogous to that used in Example 16 by using 4-(propylaminocarbonyl)-thiobenzoic acid (330mg), 4-acetoxy-3R-{1R-[dimethyl(2-methylprop-2-yl)silyloxy]-ethyl}azetidin-2-one (280mg) 1M sodium hydroxide solution (1.46ml), water (1.5ml), and acetone (7ml).

$\nu_{max}$ (film) 1758, 1660 $cm^{-1}$

$\delta$ ($CDCl_3$) 0.10 (6H,s); 0.90 (9H,s); 0.93 (3H,t, J = 7.3Hz); 1.19 (3H,d, J = 6.2Hz); 1.43-1.63 (2H,m); 3.23 (1H,dd, J = 2.4 and 3.8Hz); 3.34 (2H,m); 4.23-4.27 (1H,m); 5.42 (1H,d,J = 2.4Hz); 6.18 (1H,bs); 6.41 (1H,bs); 7.78,7.91 (4H,AA'BB',

J = 8.6Hz).

Example 58

4-Nitrobenzyl {3S-[1R-(dimethyl(2-methylprop-2-yl)-silyloxy)ethyl]-4R-[4-(propylaminocarbonyl)benzoylthio)-azetidin-2-on-1-yl}oxoacetate.

The title compound (361mg) was obtained by a procedure analogous to that used in Example 17 by using 3S-{1R-[dimethyl-(2-methylprop-2-yl)-silyloxy]ethyl}-4R-(4-propylaminocarbonylbenzoylthio)azetidin-2-one (250 mg), 4-nitrobenzyl chlorooxoacetate (162mg), diisopropylethylamine (0.145ml), calcium carbonate (250mg), and dichloromethane (5ml).

$\delta$ ($CDCl_3$) 0.11 (6H,s); 0.92 (9H,s); 1.01 (3H,t, J = 7.3Hz); 1.27 (3H,d,J = 6.3Hz); 1.53-1.65 (2H,m); 3.46-3.51 (2H,m); 3.66 (1H,dd,J = 2.8 and 3.5Hz); 4.32-4.39 (1H,m); 5.43 (2H,s); 6.22 (1H,d, J = 3.5Hz); 7.57-8.30 (8H,m).

Example 59

4-Nitrobenzyl 5R,6S-{1R-[dimethyl(2-methylprop-2-yl)-silyloxy]ethyl}-7-oxo-3-[4-(propylaminocarbonyl)phenyl]-4-thia-1-azabicyclo[3.2.0]hept-2-ene-2-carboxylate.

The title compound (45mg) was obtained by a procedure analogous to that used in Example 18 by using 4-nitrobenzyl {3S-[1R-(dimethyl-(2-methylprop-2-yl)-silyloxy)ethyl]-4R-(4-propylaminocarbonylbenzoylthio)-azetidin-2-on-1-yl}oxoacetate (361mg), triethyl phosphite (0.185ml), and o-xylene (5ml).

$\nu_{max}$ (film) 1789 $cm^{-1}$

$\delta$ ($CDCl_3$) 0.90 (6H,s); 0.94 (9H,s); 1.04 (3H,t, J = 7.4Hz); 1.23 (3H,d, J = 6.3Hz); 1.27-1.34 (2H,m); 3.44-3.52 (2H,m); 3.89 (1H,dd, J = 1.6 and 4.2Hz); 4.19-4.30 (1H,m); 5.15 and 5.34 (2H,AB, Jgem = 13.6Hz); 5.81 (1H,d, J = 1.6Hz); 6.10 (1H,bs);

7.40,7.80 (4H,AA'BB', J = 9Hz);
7.56,8.22 (4H,AA'BB', J = 8.7Hz).

Example 60

4-Nitrobenzyl 5R,6S-(1R-hydroxyethyl)-7-oxo-3-[4-(propylaminocarbonyl)phenyl]-4-thia-1-azabicyclo[3.2.0]-hept-2-ene-2-carboxylate.

The title compound (50mg) was obtained by a procedure analogous to that used in Example 19 by using 4-nitrobenzyl 5R,6S-{1R-[dimethyl(2-methylprop-2-yl)-silyloxy]ethyl}-7-oxo-3-[4-(propylaminocarbonyl)phenyl]-4-thia-1-azabicyclo[3.2.0]hept-2-ene-2-carboxylate (670mg), glacial acetic acid (0.64ml), tetrahydrofuran (5ml), and 1M tetra-n-butylammonium fluoride in tetrahydrofuran (3.2ml).

$\nu_{max}$ ($CDCl_3$) 1788, 1728 $cm^{-1}$

$\delta$ ($CDCl_3$) 0.99 (3H,t, J = 7.4Hz); 1.29 (2H,m); 1.39 (3H,d, J = 6.3Hz); 3.38-3.47 (2H,m); 3.87 (1H,dd, J = 1.6 and 6.6Hz); 4.2-4.3 (1H,m); 5.11, 5.30 (2H,AB,Jgem = 13.7Hz); 5.70 (1H,d,J=1.6Hz); 6.10 (1H,bt); 7.41,7.72 (4H,AA'BB', J = 8.5Hz); 7.50,8.15 (4H,AA'BB', J = 8.9Hz).

Example 61

Potassium 5R,6S-(1R-hydroxyethyl)-7-oxo-3-[4-(propylaminocarbonyl)phenyl]-4-thia-1-azabicyclo[3.2.0]hept-2-ene-2-carboxylate.

The title compound (30mg) was obtained by a procedure analogous to that used in Example 7 by using 4-nitrobenzyl 5R,6S-(1R-hydroxyethyl)-7-oxo-3-[4-(propylaminocarbonyl)phenyl]-4-thia-1-azabicyclo[3.2.0]-hept-2-ene-2-carboxylate (50mg), 10% palladium on charcoal (50mg), potassium bicarbonate (9.8mg), dioxane (5ml), and water (5ml).

$\delta$ ($D_2O$) 0.91 (3H,t, J = 7.3Hz); 1.28 (3H,d,J= 6.4Hz);

1.50-1.59 (2H,m); 3.27-3.37 (2H,m); 3.96 (1H,dd,J=1.5 and 5.9Hz); 4.20-4.26 (1H,m); 5.76 (1H,d, J = 1.5Hz); 7.46 and 7.65 (4H,AA'BB', J = 8.3Hz).

Example 62

4-(Acetylamino)-thiobenzoic acid.

To a stirred suspension of 4-acetylaminobenzoic acid (10.0g) in dry dichloromethane (50ml) at -15° was added triethylamine (6ml) followed by ethyl chloroformate (6ml). After 3 hours hydrogen sulphide (excess) was bubbled through; the mixture was warmed to room temperature, treated with 2M hydrochloric acid and filtered. The filtrate was partitioned and the aqueous layer extracted with ethyl acetate. The combined organic layers were washed with water, with brine, were dried and were evaporated *in vacuo*. Chromatography of the residue over silica gel and elution with ethyl acetate-hexane mixtures afforded the title compound (2.2g).

$\delta$ (acetone-$d_6$) 2.17 (3H,s); 3.42 (1H,bs); 7.90,8.01 (4H,AA'BB',J = 8.8Hz).

Example 63

4R-(4-Acetylaminobenzoylthio)-3S-{1R-[dimethyl(2-methylprop-2-yl)silyloxy]ethyl}-azetidin-2-one.

The title compound (0.85g) was obtained by a procedure analogous to that used in Example 16 by using 4-(acetylamino)-thiobenzoic acid (2.0g), 4-acetoxy-3R-{1R-[dimethyl(2-methylprop-2-yl)silyloxy]ethyl}-azetidin-2-one (2.2g) sodium hydroxide (0.44g), water (2ml), dichloromethane (5ml) and tetrahydrofuran (2ml).

$\nu_{max}$ ($CDCl_3$) 1769, 1700, 1655 $cm^{-1}$

$\delta$ ($CDCl_3$) 0.09 (6H,s); 0.89 (9H,s); 1.24 (3H,d, J = 6.3Hz); 2.23 (3H,s); 3.28 (1H,dd, J = 2.4 and 4.0Hz); 4.24-4.36 (1H,m);

5.47 (1H,d, J = 2.4Hz); 6.41 (1H,bs); 7.41 (1H,bs); 7.62,7.88 (4H,AA'BB',J = 8.8Hz).

Example 64

4-Nitrobenzyl {4R-[4-(acetylamino)benzoylthio]-3S-[1R-(dimethyl(2-methylprop-2-yl)silyloxy)ethyl]-azetidin-2-on-1-yl}oxoacetate.

The title compound (151mg) was obtained by a procedure analogous to that used in Example 17 by using 4R-(4-acetylaminobenzoylthio)-3S-(1R-{dimethyl-(2-methylprop-2-yl)-silyloxy}ethyl)-azetidin-2-one (100mg), 4-nitrobenzyl choro-oxoacetate (69mg), calcium carbonate (100mg), diisopropylethylamine (49µl), and dichloromethane (4ml).

$\nu_{max}$ ($CDCl_3$) 1813, 1760 $cm^{-1}$

$\delta$ ($CDCl_3$) 0.09 (6H,s); 0.83 (9H,s); 1.26 (3H,d,J = 6.3Hz); 2.22 (3H,s); 3.60 (1H,dd, J = 3.0 and 3.4Hz); 4.35 (1H,m); 5.41 (2H,s); 6.15 (1H,d, J = 3.4Hz); 7.4-8.3 (8H,m).

Example 65

4-Nitrobenzyl 5R,3-(4-acetylaminophenyl)-6S-{1R-[dimethyl-(2-methylprop-2-yl)silyloxy]ethyl}-7-oxo-4-thia-1-aza-bicyclo[3.2.0]hept-2-ene-2-carboxylate.

The title compound (430mg) was obtained by a procedure analogous to that used in Example 18 by using 4-nitrobenzyl {4R-[4-(acetylamino)benzoylthio]-3S-[1R-(dimethyl(2-methylprop-2-yl)silyloxy)ethyl]-azetidin-2-on-1-yl}oxoacetate (1.0g), triethyl phosphite (0.566ml), and o-xylene (8ml).

$\nu_{max}$ ($CDCl_3$) 1785, 1705 $cm^{-1}$

$\delta$ ($CDCl_3$) 0.08 (6H,s); 0.85 (9H,s); 1.27 (3H,d, J = 6.3Hz); 2.19 (3H,s); 3.78 (1H,dd,J = 1.6 and 4.2Hz); 4.30 (1H,m); 5.10,5.30 (2H,AB, Jgem = 13.7Hz); 5.65 (1H,d, J = 1.6Hz); 7.29-8.19 (8H,m).

Example 66

4-Nitrobenzyl 5R,3-(4-acetylaminophenyl)-6S-(1R-hydroxyethyl)-7-oxo-4-thia-1-azabicyclo[3.2.0]hept-2-ene-2-carboxylate.

The title compound (131mg) was obtained by a procedure analogous to that used in Example 19 by using 4-nitrobenzyl 5R,3-(4-acetylaminophenyl)-6S-{1R-[dimethyl-(2-methylprop-2-yl)-silyloxy]ethyl}-7-oxo-4-thia-1-aza-bicyclo[3.2.0]hept-2-ene-2-carboxylate (430mg), glacial acetic acid (0.534ml), tetrahydrofuran (10ml), and 1m tetra-n-butylammonium fluoride in tetrahydrofuran (2.7ml).

$\nu_{max}$ ($CDCl_3$) 1786 $cm^{-1}$

$\delta$ ($CDCl_3$) 1.40 (3H,d, J = 6.3Hz); 1.63 (1H,bs); 2.20 (3H,s); 3.60 (1H,dd, J = 1.6 and 6.6Hz); 4.2 (1H,m); 5.10,5.29 (2H,AB, Jgem=13.7Hz); 5.70 (1H,d,J = 1.6Hz); 7.23-8.16 (8H,m).

Example 67

Potassium 5R,3-(4-acetylaminophenyl)-6S-(1R-hydroxyethyl)-7-oxo-4-thia-1-azabicyclo[3.2.0]hept-2-ene-2-carboxylate

The title compound (65mg) was obtained by a procedure analogous to that used in Example 7 by using 4-nitrobenzyl 5R,3-(4-acetylaminophenyl)-6S-(1R-hydroxyethyl)-7-oxo-4-thia-1-azabicyclo[3.2.0]hept-2-ene-2-carboxylate (123mg), 10% palladium on charcoal (123mg), potassium bicarbonate (33.3mg), dioxane (5ml) and water (5ml).

$\delta$ ($D_2O$) 1.34 (3H,d,J = 6.4Hz); 2.19 (3H,s); 4.00 (1H,dd, J = 1.6 and 6.1Hz); 4.17-4.28 (1H,m); 5.79 (1H,d, J = 1.6Hz); 7.46 (4H,s).

Example 68

3-(Methylaminocarbonyl)-thiobenzoic acid

The title compound (4.5g) was obtained by a procedure analogous to that used in Example 44 by using isophthaloyl chloride (10.0g), triethylamine (17.8ml), methylamine hydrochloride (5g), dichloromethane (200ml), pyridine (7.5ml), and hydrogen sulphide (excess).

$\delta$ (acetone-$d_6$) 2.91 (3H,d, J = 1.2Hz); 7.5-8.3 (4H,m).

Example 69

3S-{1R-[Dimethyl(2-methylprop-2-yl)silyloxy]ethyl}-4R-[3-(methylaminocarbonyl)benzoylthio]azetidin-2-one.

The title compound (1.0g) was obtained by a procedure analogous to that used in Example 16 by using 4-acetoxy-3R-{1R-[dimethyl(2-methylprop-2-yl)silyloxy]-ethyl}azetidin-2-one (2.04g) 3-(methylaminocarbonyl)-thiobenzoic acid (4.5g), 1M sodium hydroxide (21ml), and acetone (50ml).

$\nu_{max}$ (film) 1768, 1655 $cm^{-1}$

$\delta$ ($CDCl_3$) 0.10 (6H,s); 0.89 (9H,s); 1.24 (3H,d, J = 6.3Hz); 3.03 (3H,d, J = 4.8Hz); 3.29 (1H,dd, J = 2.4 and 3.8Hz); 4.30-4.36 (1H,m); 5.49 (1H,d, J = 2.4Hz); 6.96 (1H,bs,J = 4.8Hz); 7.14 (1H,bs); 7.53-8.30 (4H,m).

Example 70

4-Nitrobenzyl {3S-[1R-(dimethyl(2-methylprop-2-yl)-silyloxy)ethyl]-4R-[3-(methylaminocarbonyl)benzoylthio]-azetidin-2-on-1-yl}oxoacetate.

The title compound (1.43g) was obtained by a procedure analogous to that used in Example 17 by using 3S-{1R-[dimethyl-(2-methylprop-2-yl)-silyloxy]ethyl}-4R-(3-methylaminocarbonylbenzoylthio)-azetidin-2-one (1.0g), 4-nitrobenzyl chloro-oxoacetate (720mg),

dichloromethane (10ml), calcium carbonate (1.0g), and diisopropylethylamine (0.60ml).

δ ($CDCl_3$) 0.09 (6H,s); 0.83 (9H,s); 1.25 (3H,d, J = 6.3Hz); 3.04 (3H,d, J = 4.9Hz); 3.60-3.66 (1H,m); 4.30-4.40 (1H,m); 5.38,5.42 (2H,AB, J = 13.0Hz); 6.19 (1H,d,J=3.4Hz); 7.53-8.26 (8H,m).

Example 71

4-Nitrobenzyl 5R,6S-{1R-[dimethyl(2-methylprop-2-yl)-silyloxy]ethyl}-3-[3-(methylaminocarbonyl)phenyl]-7-oxo-4-thia-1-azabicyclo[3.2.0]hept-2-ene-2-carboxylate.

The title compound (0.48g) was obtained by a procedure analogous to that used in Example 18 by using 4-nitrobenzyl {3S-[1R-(dimethyl(2-methylprop-2-yl)-silyloxy)ethyl]-4R-[3-(methylaminocarbonyl)benzoylthio]-azetidin-2-on-1-yl}oxoacetate (1.43g), triethyl phosphite (0.78ml), hydroquinone (5mg), and o-xylene (10ml).

$\nu_{max}$ (film) 1779 $cm^{-1}$

δ ($CDCl_3$) 0.08 (6H,s); 0.86 (9H,s); 1.25 (3H,d, J = 6.4Hz); 3.01 (3H,d, J = 4.9Hz); 3.81 (1H,dd,J = 1.6 and 4.2Hz); 4.10-4.25 (1H,m); 5.20,5.28 (2H,AB, J = 13.6Hz); 5.74 (1H,d,J = 1.6Hz); 6.11 (1H,bd, J = 4.9Hz); 7.39-8.29 (8H,m).

Example 72

4-Nitrobenzyl 5R,6S-(1R-hydroxyethyl)-3-[3-(methylaminocarbonyl)phenyl]-7-oxo-4-thia-1-azabicyclo[3.2.0]-hept-2-ene-2-carboxylate.

The title compound (160mg) was obtained by a procedure analogous to that used in Example 19 by using 4-nitrobenzyl 5R,6S-{1R-[dimethyl(2-methylprop-2-yl)-silyloxy]ethyl}-3[3-(methylaminocarbonyl)phenyl]-7-oxo-4-thia-1-azabicyclo[3.2.0]hept-2-ene-2-carboxylate

(480mg), glacial acetic acid (0.48ml), tetrahydrofuran (10ml) and 1M tetra-n-butyalmmonium fluoride in tetrahydrofuran (2.4ml).

$\nu_{max}$ (KBr) 1785 $cm^{-1}$

$\delta$ ($CDCl_3$) 1.41 (3H,d, J = 6.3Hz); 1.68 (1H,bs); 3.01 (3H,d, J = 4.9Hz); 3.87 (1H,dd,J = 1.6 and 6.5Hz); 4.28-4.35 (1H,m); 5.13, 5.30 (2H,AB,J = 13.6Hz); 5.77 (1H,d,J = 1.6Hz); 6.08 (1H,bs); 7.37-8.17 (8H,m).

Example 73

Potassium 5R,6S-(1R-hydroxyethyl)-3-[3-(methylaminocarbonyl)phenyl]-7-oxo-4-thia-1-azabicyclo[3.2.0]hept-2-ene-2-carboxylate.

The title compound (80mg) was obtained by a procedure analogous to that used in Example 7 by using 4-nitrobenzyl 5R,6S-(1R-hydroxyethyl)-3-[3-(methylaminocarbonyl)phenyl]-7-oxo-4-thia-1-azabicyclo[3.2.0]hept-2-ene-2-carboxylate (160mg), 10% palladium on charcoal (160mg), potassium bicarbonate (33.1mg), water (5ml) and dioxane (5ml).

$\delta$ ($D_2O$) 1.30 (3H,d,J = 6.4Hz); 2.89 (3H,s); 3.99 (1H,dd, J = 1.2 and 5.8Hz); 4.21-4.29 (1H,m); 5.78 (1H,d, J = 1.2Hz); 7.44-7.79 (4H,m).

Example 74

4-(Nitro)-thiobenzoic acid

The title compound (9.8g) was obtained by a procedure analogous to that used in Example 24 by using 4-nitrobenzoyl chloride (10.0g), dichloromethane (200ml), pyridine (8.6ml); and hydrogen sulphide (excess).

$\delta$ (acetone-$d_6$) 4.30 (1H,bs); 8.30,8.37 (4H,AA'BB',J = 9.9Hz).

Example 75

3S-{1R-[Dimethyl(2-methylprop-2-yl)silyloxy]ethyl}-4R-(4-nitrobenzoylthio)azetidin-2-one.

The title compound (2.6g) was obtained by a procedure analogous to that used in Example 16 by using 4-acetoxy-3R-{1R-[dimethyl(2-methylprop-2-yl)silyloxy]ethyl}azetidin-2-one (2.0g), 4-(nitro)-thiobenzoic acid (1.7g), dichloromethane (50ml), water (50ml), and sodium hydroxide (0.36g).

$\nu_{max}$ ($CDCl_3$) 1775 $cm^{-1}$

$\delta$ ($CDCl_3$) 0.10 (6H,s); 0.89 (9H,s); 1.27 (3H,d, J = 6.3Hz); 3.32 (1H,dd, J = 2.3 and 3.8Hz); 4.30 (1H,m); 5.51 (1H,d, J = 2.3Hz); 6.50 (1H,bs); 8.07,8.32 (4H,AA'BB', J = 9.0Hz).

Example 76

4-Nitrobenzyl {3S-[1R-(dimethyl(2-methylprop-2-yl)-silyloxy)ethyl]-4R-(4-nitrobenzoylthio)azetidin-2-on-1-yl}oxoacetate.

The title compound (754mg) was obtained by a procedure analogous to that used in Example 17 by using 3S-{1R-[dimethyl(2-methylprop-2-yl)silyloxy]ethyl}-4R-(4-nitrobenzoylthio)azetidin-2-one (0.5g), calcium carbonate (0.5g), 4-nitrobenzyl chlorooxoacetate (0.36g), dichloromethane (10ml) and diisopropylethylamine (0.255ml).

$\nu_{max}$ (film) 1817, 1712 $cm^{-1}$

$\delta$ ($CDCl_3$) 0.10 (6H,s), 0.84 (9H,s); 1.28 (3H,d, J = 6.3Hz); 3.64 (1H,dd, J = 3.0 and 3.4Hz); 4.38-4.47 (1H,m); 5.41 (2H,s); 6.18 (1H,d,J = 3.4Hz); 7.57,8.22 (4H,AA'BB', J = 8.8Hz); 8.08,8.33 (4H,AA'BB', J = 8.9Hz).

Example 77

4-Nitrobenzyl 5R,6S-{1R-[dimethyl(2-methylprop-2-yl)-silyloxy]ethyl}-3-(4-nitrophenyl)-7-oxo-4-thia-1-azabicyclo[3.2.0]hept-2-ene-2-carboxylate.

The title compound (215mg) was obtained by a procedure analogous to that used in Example 18 by using 4-nitrobenzyl{3S-[1R-(dimethyl(2-methylprop-2-yl)silyloxy)ethyl]-4R-(4-nitrobenzoylthio)azetidin-2-on-1-yl}-oxoacetate (754mg), triethyl phosphite (0.418 ml), and o-xylene (5ml).

$\nu_{max}$ ($CDCl_3$) 1778 $cm^{-1}$

$\delta$ ($CDCl_3$) 0.09 (6H,s); 0.86 (9H,s); 1.27 (3H,d, J = 6.3Hz); 3.87 (1H,dd, J = 1.5 and 4.0Hz); 4.29 (1H,m); 5.12, 5.30 (2H,AB, Jgem = 13.6Hz); 5.81 (1H,d,J = 1.5Hz); 7.51,8.16 (4H,AA'BB',J=8.6Hz); 7.62,8.20 (4H,AA'BB',J = 8.8Hz).

Example 78

4-Nitrobenzyl 5R,6S-(1R-hydroxyethyl)-3-(4-nitrophenyl)-7-oxo-4-thia-1-azabicyclo[3.2.0]hept-2-ene-2-carboxylate

The title compound (106mg) was obtained by a procedure analogous to that used in Example 19 by using 4-nitrobenzyl 5R,6S-{1R-[dimethyl(2-methylprop-2-yl)silyloxy]ethyl}-3-(4-nitrophenyl)-7-oxo-4-thia-1-azabicyclo-[3.2.0]hept-2-ene-2-carboxylate (200mg), glacial acetic acid (0.204ml), tetrahydrofuran (5ml), and 1M tetra-n-butylammonium fluoride in tetrahydrofuran (1.02ml).

$\nu_{max}$ (film) 1790, 1712 $cm^{-1}$

$\delta$ ($CDCl_3$) 1.41 (3H,d, J = 6.3Hz); 1.61 (1H,bs); 3.91 (1H,dd, J = 1.6, 6.4Hz); 4.20-4.39 (1H,m); 5.12, 5.31 (2H,AB, Jgem = 13.5Hz); 5.82 (1H,d, J = 1.6Hz); 7.47,8.17 (4H,AA'BB',J=8.7Hz); 7.58,8.21 (4H,AA'BB', J = 8.9Hz).

Example 79

Potassium 5R,3-(4-aminophenyl)-6S-(1R-hydroxyethy)-7-oxo-4-thia-1-azabicyclo[3.2.0]hept-2-ene-2-carboxylate

The title compound (44mg) was obtained by a procedure analogous to that used in Example 7 by using 4-nitrobenzyl 5R,6S-(1R-hydroxyethyl)-3-(4-nitrophenyl)-7-oxo-4-thia-1-azabicyclo[3.2.0]hept-2-ene-2-carboxylate (100mg), 10% palladium on charcoal (100mg), potassium bicarbonate (21.2 mg), water (5ml), and dioxane (5ml). $\delta$ ($D_2O$) 1.33 (3H,d,J = 6.3Hz); 3.93 (1H,dd, J = 1.4 and 5.3Hz); 4.23 (1H,m); 5.70 (1H,d, J = 1.4Hz); 6.80,7.30 (4H,AA'BB', J = 8.4Hz).

Example 80

Potassium 5R-3-(4-acetylaminophenyl)-6S-(1R-hydroxyethyl)-7-oxo-4-thia-1-azabicyclo[3.2.0]hept-2-ene-2-carboxylate.

A solution of 4-nitrobenzyl 5R,3-(4-aminophenyl)-6S-(1R-hydroxyethyl)-7-oxo-4-thia-1-azabicyclo[3.2.0]hept-2-ene-2-carboxylate (33mg) in water (0.5ml) and dioxane (0.5ml) was treated at 0$^o$ with acetic anhydride (0.05ml). The mixture was warmed over 30 minutes to room temperature and stirred a further hour. Lyophilisation followed by partition of the solid between ethyl acetate and water, and lyophilisation of the aqueous layer afforded the title compound (29mg) with properties identical to those given in Example 67.

Example 81

2-(Trimethylsilyl)ethyl chlorooxoxacetate.

To a stirred solution of oxalyl chloride (12.7g) in dry diethyl ether (25ml) at 0$^o$C was added dropwise 2-(trimethylsilyl)ethanol (11.8g). The mixture was stirred for a further 16 hours, and then distilled to afford the title compound (19.9g) bp 80$^o$/8mm.

δ ($CDCl_3$) 0.83 (9H,s); 1.16 (2H,m); 4.46 (2H,m).

Example 82

2-(Trimethylsilyl)ethyl {3S-[1R-(dimethyl(2-methylprop-2-yl)silyloxy)ethyl]-4R-(4-nitrobenzoylthio)azetidin-2-on-1-yl}oxoacetate.

The title compound (8.0g) was obtained by a procedure analogous to that used in Example 17 by using 4-acetoxy-3S-{1R-[dimethyl(2-methylprop-2-yl)silyloxy]-ethyl}-azetidin-2-one (5.0g), calcium carbonate (5.0g), 2-(trimethylsilyl)ethyl chlorooxoacetate (3.35ml), dichloromethane (100ml) and diisopropylethylamine (4.2ml).

δ ($CDCl_3$) 0.07 (9H,s); 0.12 (6H,s); 0.88 (9H,s); 1.11 (2H,t,J = 8.4Hz); 1.29 (3H,d,J = 6.3Hz); 3.62 (1H,t,J = 3.3Hz); 4.40 (3H,m); 6.18 (1H,d,J = 3.3Hz); 8.11, 8.34 (4H,AA'BB',J = 8.8Hz).

Example 83

2-(Trimethylsilyl)ethyl 5R,6S-{1R-[dimethyl(2-methyl-prop-2-yl)silyloxy]ethyl}-3-(4-nitrophenyl)-7-oxo-4-thia-1-azabicyclo[3.2.0]hept-2-ene-2-carboxylate.

The title compound (2.86g) was obtained by a procedure analogous to that used in Example 18 by using 2-(trimethylsilyl)ethyl {3S-[1R-(dimethyl(2-methylprop-2-yl)silyloxy)ethyl]-4R-(4-nitrobenzoylthio)azetidin-2-on-1-yl}oxoacetate (8.0g), triethyl phosphite (4.2ml), o-xylene (100ml), and hydroquinone (10mg).

$\nu_{max}$ (film) 1796, 1711 $cm^{-1}$

δ ($CDCl_3$) 0.01 (9H,s); 0.11 (3H,s); 0.12 (3H,s); 0.91 (11H,m); 1.28 (3H,d,J = 6.3Hz); 3.82 (1H,dd,J = 1.6 and 4.6Hz); 4.18 (2H,m); 4.29 (1H,m); 5.76 (1H,d,J = 1.6Hz); 7.64, 8.23 (4H,AA'BB',J=8.9Hz).

Example 84

2-(Trimethylsilyl)ethyl 5R,6S-(1R-hydroxyethyl)-3-(4-nitrophenyl)-7-oxo-4-thia-1-azabicyclo[3.2.0]hept-2-ene-2-carboxylate.

The title compound (142mg) was obtained by a procedure analogous to that used in Example 19 by using 2-(trimethylsilyl)ethyl 5R,6S-{1R-[dimethyl(2-methylprop-2-yl)silyloxy]ethyl}-3-(4-nitrophenyl)-7-oxo-4-thia-1-azabicyclo[3.2.0]hept-2-ene-2-carboxylate (360mg), glacial acetic acid (0.4ml), tetrahydrofuran (10ml), and 1M tetra-n-butylammonium fluoride in tetrahydorfuran (2ml).

$\nu_{max}$ ($CDCl_3$) 1789, 1710 $cm^{-1}$

$\delta$ ($CDCl_3$) 0.01 (9H,s); 0.92 (2H,m); 1.40 (3H,d,J = 6.3Hz); 3.86 (1H,dd,J = 1.6 and 6.6Hz); 4.20 (2H,m); 4.30 (1H,m); 5.79 (1H,d,J = 1.6Hz). 7.63,8.24 (4H,AA'BB',J = 8.9Hz).

Example 85

2-(Trimethylsilyl)ethyl 5R,6S-(1R-hydroxyethyl)-3-(4-hydroxyaminophenyl)-7-oxo-4-thia-1-azabicyclo[3.2.0]-hept-2-ene-2-carboxylate.

A mixture of 2-(trimethylsilyl)ethyl 5R,6S-(1R-hydroxyethyl)-3-(4-nitrophenyl)-7-oxo-4-thia-1-azabicyclo[3.2.0]hept-2-ene-2-carboxylate (2.1g), 10% palladium on charcoal (2.1g) and dioxane (50ml) was hydrogenolysed at 375 kPa (4 atm) for 2 hours, was filtered through diatomaceous earth and evaporated *in vacuo*. Chromatography at the residue over silica gel and elution with ethyl acetate-hexane mixtures afforded the title compound (1.5g).

$\delta$ (acetone-$d_6$) 0.02 (9H,s); 0.95 (2H,m); 1.31 (3H,d,J = 6.3Hz); 3.76 (1H,dd,J = 1.5 and 7.0Hz); 4.17 (3H,m); 4.42 (1H,d,J = 4.9Hz); 5.69 (1H,d,J=1.5Hz) 6.95 and 7.43 (4H,AA'BB',J = 8.6Hz); 7.90 (1H,broad);

8.15 (1H, broad).

Example 86

2-(Trimethylsilyl)ethyl 5R,3-(4-aminophenyl)-6S-(1R-hydroxyethyl)-7-oxo-4-thia-1-azabicyclo[3.2.0]-hept-2-ene-2-carboxylate.

A mixture of 2-(trimethylsily)ethyl 5R,6S-(1R-hydroxyethyl)-3-(4-hydroxyaminophenyl)-7-oxo-4-thia-1-azabicyclo[3.2.0]hept-2-ene-2-carboxylate (1.4g), platinum dioxide (500mg), and dry ethyl acetate (30ml) was hydrogenolysed at 375 kPa (4 atm) at 20$^o$, and then filtered and evaporated *in vacuo* . Chromatography of the residue over silica gel and elution with ethyl acetate-hexane mixtures afforded the title compound (1.06g).

$\nu_{max}$ (KBr) 1780, 1706 $cm^{-1}$

$\delta$ (acetone-$d_6$) 0.01 (9H,s); 0.96 (2H,m); 1.29 (3H,d,J = 6.3Hz); 3.70 (1H,dd,J = 1.5 and 7.1Hz); 4.15 (3H,m); 5.20 (1H,broad); 5.60 (1H,d,J = 1.5Hz); 6.63 and 7.33 (4H,AA'BB',J = 8.8Hz).

Example 87

2-(Trimethylsilyl)ethyl 5R,3-(4-formylaminophenyl)-6S-(1R-hydroxyethyl)-7-oxo-4-thia-1-azabicyclo[3.2.0]-hept-2-ene-2-carboxylate.

To a stirred solution of 2-(trimethylsilyl)ethyl 5R,3-(4-aminophenyl)-6S-(1R-hydroxyethyl)-7-oxo-4-thia-1-azabicyclo[3.2.0]hept-2-ene-2-carboxylate (200mg) in deuterochloroform (5ml) at 0$^o$ was added formic acid (22.3μl), followed by 1-(3-dimethylamino-propyl)-3-ethylcarbodiimide hydrochloride (113mg). The mixture was stirred a further 15 minutes, and was then partitioned between water and chloroform. The organic layer was washed with 1M citric acid, with water, with saturated aqueous sodium bicarbonate, with brine, was

dried over anhydrous sodium sulphate and was evaporated *in vacuo* . Chromatography of the residue over silica gel and elution with ethyl acetate-hexane mixtures afforded the title compound (162mg).

$\nu_{max}$ (KBr) 1777, 1702 and 1623 $cm^{-1}$

$\delta$ (DMSO-$d_6$) 0.01 (9H,s); 0.84 (2H,m); 1.18 (3H,d,J = 6.3Hz); 3.85 (1H,dd,J = 1.5 and 6.3Hz); 4.00 (1H,m); 4.10 (2H,m); 5.23 (1H,d,J = 4.8Hz); 5.71 (1H,d,J = 1.5Hz); 7.42 and 7.61 (4H,AA'BB',J = 8.5Hz); 8.31 (1H,s); 10.31 (1H, broad).

Example 88

Potassium 5R,3-(4-formylaminophenyl)-6S-(1R-hydroxyethyl)-7-oxo-4-thia-1-azabicyclo[3.2.0]hept-2-ene-2-carboxylate.

To a stirred solution of 2-(trimethylsilyl)ethyl 5R,3-(4-formylaminophenyl)-6S-(1R-hydroxyethyl)-7-oxo-4-thia-1-azabicyclo[3.2.0]hept-2-ene-2-carboxylate (80mg) in tetrahydrofuran (3ml) at $0^{o}$ was added a solution of a 1M tetra-n-butylammonium fluoride in tetrahydrofuran (0.184ml) which had been dried over 4A molecular sieves. After 16 hours the mixture was treated with an aqueous solution of potassium bicarbonate (18.4mg). The aqueous solution was washed with ethyl acetate, and then lyophilised to afford a crude product (110mg). Purification either by chromatography over HP20A resin or by acidification/ neutralisation or preparative high pressure liquid reverse phase chromatography ($C_{18}$-silica gel) and elution with water-acetonitrile-formic acid mixtures afforded, after lyophilisation and reneutralisation with potassium bicarbonate the title compound (50mg).

$\delta$ ($D_2O$) 1.35 (3H,d,J = 6.3Hz); 3.95 (1H,dd, J = 1.5 and 5.5Hz); 4.25 (1H,m);

5.75 (1H,d,J = 1.5Hz); 7.43 and 7.49 (4H,AA'BB', J = 8.9Hz); 8.25 (1H, bs).

Example 89

2-(Trimethylsilyl)ethyl 5R,6S-(1R-hydroxyethyl)-3-{4-[(methylamino)carbonylamino]phenyl}-7-oxo-4-thia-1-azabicyclo[3.2.0]hept-2-ene-2-carboxylate.

A mixture of 2-(trimethylsilyl)ethyl 5R,3-(4-aminophenyl)-6S-(1R-hydroxyethyl)-7-oxo-4-thia-1-azabicyclo[3.2.0]hept-2-ene-2-carboxylate (200mg), dry tetrahydrofuran (5ml) and methyl isocyanate (0.3ml) was stirred at room temperature for 16 hours, and then partitioned between ethyl acetate and water. The organic layer was washed with water and with brine, was dried over anhydrous sodium sulphate and evaporated *in vacuo*. Chromatography of the residue over silica gel, and elution with ethyl acetate-hexane mixtures afforded the title compound (86 mg).

$\nu_{max}$ (film) 1785 $cm^{-1}$

$\delta$ (acetone-$d_6$) 0.01 (6H,s); 0.93 (2H,m); 1.29 (3H,d,J = 6.3Hz); 2.73 (3H,d,J = 4.6Hz); 3.77 (1H,dd,J = 1.6 and 6.9Hz); 4.15 (3H,m); 4.37 (1H,d,J = 5.0Hz); 5.70 (1H,d,J = 1.6Hz); 5.78 (1H, broad); 7.41 and 7.52 (4H,AA'BB',J = 8.8Hz); 8.21 (1H, broad).

Example 90

Potassium 5R,6S-(1R-hydroxyethyl)-3-{4-[(methylamino)-carbonylamino]phenyl}-7-oxo-4-thia-1-azabicyclo[3.2.0]-hept-2-ene-2-carboxylate.

The title compound (55mg) was obtained by a procedure analogous to that used in Example 88, using 2-(trimethylsilyl)ethyl 5R,6S-(1R-hydroxyethyl)-3-{4-[(methylamino)carbonylamino]phenyl}-7-oxo-4-thia-1-azabicyclo[3.2.0]hept-2-ene-2-carboxylate (80mg), a

dried solution of 1M tetra-n-butylammonium fluoride in tetrahydrofuran (0.17ml), tetrahydrofuran (5ml) and potassium bicarbonate (17.3mg).
δ ($D_2O$) 1.3 (3H,d,J=6.3Hz); 2.71 (3H,s); 3.91 (1H,dd,J=1.5 and 5.8Hz); 4.23 (1H,m); 5.70 (1H,d,J=1.5Hz); 7.26 and 7.36 (4H,AA'BB',J=8.7Hz).

Example 91

4-Nitrobenzyl 5R,3-(3-carboxyphenyl)-6S-{1R-[dimethyl-(2-methylprop-2-yl)silyloxy]ethyl}-7-oxo-4-thia-1-aza-bicyclo[3.2.0]hept-2-ene-2-carboxylate.

By a procedure analogous to that used in Example 29, but starting from a solution of isophthaloyl chloride (10.15g) in dioxane (150ml), a solution of 2,4-dimethoxybenzyl alcohol (10.09g) in dioxane (20ml), triethylamine (8.35ml), dichloromethane (100ml), pyridine (4.85ml), and hydrogen sulphide (excess), there was obtained
3-(2,4-dimethoxybenzyloxycarbonyl)thiobenzoic acid (11.6g) :
$\nu_{max}$ 2550, 1721, 1675 and 1615 $cm^{-1}$;
δ (acetone-$d_6$) 3.72 (3H,s); 3.77 (3H,s); 5.26 (2H,s); 6.44 (1H,dd,J=2.4 and 8.3Hz); 6.50 (1H,d,J=2.3Hz); 7.28 (1H,d,J=8.3Hz); 7.60 (1H,m); 8.16 (2H,m); 8.53 (1H,m).

A solution of this thiobenzoic acid (10.9g) in acetone (70ml) was treated in a procedure analogous to that used in Example 30 with 1M-sodium hydroxide (43ml) and a solution of 4-acetoxy-3R-{1R-[dimethyl(2-methyl-prop-2-yl)silyloxy]ethyl}-azetidin-2-one (7.85g) in acetone-water (3:1) (100ml) to afford
4R-[3-(2,4-dimethoxybenzyloxycarbonyl)benzoylthio]-3S-{1R-[dimethyl(2-methylprop-2-yl)silyloxy]ethyl}azetidin-2-one (7.66g):

$\nu_{max}$ ($CDCl_3$) 3418, 1772, 1719 $cm^{-1}$; $\delta$ ($CDCl_3$) 0.07 (6H,s); 0.86 (9H,s); 1.22 (3H,d,J=6.3Hz); 3.27 (1H,dd,J=2.5 and 4Hz); 3.80 (3H,s); 3.81 (3H,s); 4.29 (3H,m); 5.34 (2H,s); 5.46 (1H,d,J=2.5Hz); 6.47 (2H,m); 6.54 (1H,s); 7.30 (1H,d,J=8.9Hz); 7.51 (1H,t,J=7.8Hz); 8.05 (1H,dd,J=1.4 and 7.8Hz); 8.25 (1H,dd,J=1.4 and 7.8Hz); 8.54 (1H,t,J=1.4Hz).

A solution of this azetidinone (2.8g) in dichloromethane (20ml) was treated in a procedure analogous to that used in Example 31 with calcium carbonate (1.25g), diisopropylethylamine (0.97g), and a solution of 4-nitrobenzyl chlorooxalate (1.46g) in dichloromethane (5ml) to afford as a yellow foam 4-nitrobenzyl 4R-[3-(2,4-dimethoxybenzyloxycarbonyl)-benzoylthio]-3S-{1R-[dimethyl(2-methylprop-2-yl)silyl-oxy]ethyl}azetidin-2-on-1-yl]oxoacetate (3.8g). $\nu_{max}$ (film) 1818, 1761 and 1720 $cm^{-1}$; $\delta$ ($CDCl_3$) 0.01 (3H,s); 0.09 (3H,s); 0.83 (9H,s); 1.26 (3H,d,J=6.3Hz); 3.64 (1H,t,J=3Hz); 3.82 (3H,s); 3.83 (3H,s); 4.40 (1H,m); 5.36 (2H,s); 5.40 (2H,s); 6.19 (1H,d,J=3.4Hz); 6.50 (1H,m); 7.32 (1H,d,J=8.9Hz); 7.55 (3H,m); 8.09 (1H,d,J=7.9Hz); 8.21 (2H,d,J=8.8Hz); 8.29 (1H,d,J=7.9Hz); 8.57 (1H,d,J=1.7Hz).

A solution of this foam (3.8g) in xylene (100ml) was treated in a procedure analogous to that used in Example 32 with a solution of triethyl phosphite (1.66g) in xylene (10ml) to afford 4-nitrobenzyl 5R,3-[3-(2,4-dimethoxybenzyloxycarbonyl)-phenyl]-6S-{1R-[dimethyl(2-methylprop-2-yl)silyl-oxy]ethyl}-7-oxo-4-thia-1-azabicyclo[3.2.0]hept-2-ene-2-carboxylate (1.84g): $\nu_{max}$ ($CDCl_3$) 1790 and 1721 $cm^{-1}$;

δ ($CDCl_3$) 0.07 (3H,s); 0.08 (3H,s); 0.85 (9H,s); 3.82 (3H,s+m); 4.30 (1H,m); 5.08, 5.25 (2H,AB,$J_{gem}$=13.7Hz); 5.32 (2H,s); 5.74 (1H,d,J=1.5Hz); 6.48 (2H,m); 7.31 (1H,d,J=8.9Hz); 7.40 (3H,m); 7.59 (1H,m); 8.08 (4H,m);

A solution of this 4-nitrobenzyl carboxylate (1.84g) in dichloromethane (100ml) was treated in a procedure analogous to that used in Example 33 with water (1ml) and 2,3-dichloro-5,6-dicyano-1,4-benzoquinone (DDQ) (1.14g) to afford the title 3-carboxyphenyl compound (0.98g):
δ ($CDCl_3$) 0.07 (3H,s); 0.09 (3H,s); 0.86 (9H,s); 1.28 and 1.29 (3H,2xd,J=6.3Hz); 3.83 (1H,dd,J=1.6 and 4Hz); 4.31 (1H,m); 5.12, 5.28 (2H,AB,$J_{gem}$=13.6Hz); 5.77 (1H,d,J=1.5Hz); 7.46 (3H,m); 7.70 (1H,m); 8.15 (4H,m).

Example 92
4-Nitrobenzyl 5R,3-[3-(2,4-dimethoxybenzyloxycarbonyl)-phenyl]-6S-(1R-hydroxyethyl)-7-oxo-4-thia-1-azabicyclo-[3.2.0]hept-2-ene-2-carboxylate

The title compound (6.6g) was prepared by a procedure analogous to that used in Example 37 by using 4-nitrobenzyl 5R,3-[3-(2,4-dimethoxybenzyloxycarbonyl)-phenyl]-6S-{1R-[dimethyl(2-methylprop-2-yl)silyloxy]-ethyl}-7-oxo-4-thia-1-azabicyclo[3.2.0]hept-2-ene-2-carboxylate (8.0g), glacial acetic acid (6.5g) and a 1M solution of tetra-n-butylammonium fluoride in tetrahydrofuran (32.6ml).

δ ($CDCl_3$) 1.31 (3H,d,J=6Hz); 3.75 (6H,s); 3.80 (1H,dd,J=1.2 and 6.5Hz); 4.03 (1H,m); 5.00, 5.17 (2H,AB,$J_{gem}$=13.6Hz); 5.25 (2H,s); 5.69 (1H,d,J=1.2Hz); 6.42 (2H,m); 7.2-7.5 (5H,m); 7.9-8.2 (4H,m).

Example 93

4-Nitrobenzyl 5R,3-(3-carboxyphenyl)-6S-(1R-hydroxyethyl)-7-oxo-4-thia-1-azabicyclo[3.2.0]hept-2-ene-2-carboxylate

The title compound (1.39g) was obtained by a procedure analogous to that used in Example 40 using 4-nitrobenzyl 5R,3-[3-(2,4-dimethoxybenzyloxycarbonyl)-phenyl]-6S-(1R-hydroxyethyl)-7-oxo-4-thia-1-azabicyclo-[3.2.0]hept-2-ene-2-carboxylate (6.2g), dichloromethane (200ml), water (10ml) and DDQ (4.54g): $\delta$ (acetone-$d_6$) 1.20 (3H,d,J=6.3Hz); 3.84 (1H,dd,J=1.7 and 6.4Hz); 4.10 (1H,m); 5.06, 5.19 (2H,AB,$J_{gem}$=13.8Hz); 5.79 (1H,d,J=1.7Hz); 7.35-7.45 (3H,m); 7.61 (1H,dd,J=7.8 and ~1.4Hz); 7.90-8.06 (4H,m).

Example 94

4-Nitrobenzyl 5R,3-(3-aminocarbonylphenyl)-6S-{1R-[dimethyl-(2-methylprop-2-yl)silyl]oxyethyl}-7-oxo-4-thia-1-azabicyclo[3.2.0]hept-2-ene-2-carboxylate.

By a procedure analogous to that used in Example 34, but using 4-nitrobenzyl 5R,3-(3-carboxyphenyl)-6S-{1R-[dimethyl-(2-methylprop-2-yl)silyloxy]ethyl}-7-oxo-4-thia-1-azabicyclo[3.2.0]hept-2-ene-2-carboxylate (786mg), HOBT (365mg), acetonitrile 840ml), tetrahydrofuran (4ml), 1-(3-dimethylaminopropyl)-3-ethylcarbodiimide hydrochloride (389mg) and a solution of ammonia in ethanol (36 g.$l^{-1}$, 2ml) the title compond was obtained: $\delta$ (acetone-$d_6$) 0.08 (3H,s); 0.10 (3H,s); 0.93 (9H,s); 1.28 (3H,d,J=6.3z); 4.02 (1H,dd,J=1.7 and 3.6Hz); 4.35 (1H,dq,J=3.6 and 6.3Hz); 5.30, 5.15 (2H,AB,$J_{gem}$=13.9Hz); 5.87 (1H,d,J=1.7Hz); 6.92 (1H,bs); 7.47 (1H,t,J=7.8Hz); 7.54, 8.13 (4H,AA'BB',$J_{AB}$=8.6Hz); 7.64 (1H,dt,J=1.4 and

7.8Hz); 8.00 (1H,dt,J=1.4 and 7.8Hz); 8.07 (1H,t,J=1.4Hz).

Example 95

4-Nitrobenzyl 5R,3-(3-aminocarbonylphenyl)-6S-(1R-hydroxyethyl)-7-oxo-4-thia-1-azabicyclo[3.2.0]hept-2-ene-2-carboxylate

By a process analogous to that used in Example 38, but using 4-nitrobenzyl 5R,3-(3-carboxyphenyl)-6S-(1R-hydroxyethyl)-7-oxo-4-thia-1-azabicyclo[3.2.0]hept-2-ene-2-carboxylate (492mg), 1-hydroxybenzotriazole (HOBT) (283mg), 1-(3-dimethylaminopropyl)-3-ethylcarbodiimide hydrochloride 8301mg) and a solution of ammonia in ethanol (29.8 $g.l^{-1}$, 1.78ml), acetonitrile (50ml) and tetrahydrofuran 82ml), the title compound (392mg) was obtained.

$\delta$ (acetone-$d_6$) 1.30 (3H,d,J=6.3Hz);3.93 (1H,dd,J=1.6 and 6.3Hz); 4.18 (1H,m); 5.16, 5.29 (2H,AB,$J_{gem}$=14.0Hz); 5.88 (1H,d,J=1.6Hz); 6.79 (1H,bs); 7.48 (3H,m); 7.62 (1H,dt,J=1.3Hz and 7.8Hz); 7.97 (1H,dt,J=1.3 and 7.8Hz); 8.03 (1H,t,J=1.3Hz); 8.13 (2H,d,J=8.8Hz).

Alternatively, the title compound (314mg) was obtained from the corresponding dimethyl-(2-methylprop-2-yl)silyloxyethyl compound (744mg) prepared in Example 94 by a procedure analogous to that used in Example 35 by using glacial acetic acid (766mg) and a solution of tetra-n-butylammonium fluoride in tetrahydrofuran (1M, 3.84ml).

Example 96

Potassium 5R,3-(3-aminocarbonylphenyl)-6S-(1R-hydroxyethyl)-7-oxo-4-thia-1-azabicyclo[3.2.0]hept-2-ene-2-carboxylate.

By a procedure analogous to that used in Example 36, and using 4-nitrobenzyl 5R,3-(3-aminocarbonylphenyl)-6S-(1R-hydroxyethyl)-7-oxo-4-thia-1-azabicyclo[3.2.0]hept-2-ene-2-carboxylate (250mg), dioxane (1ml), potassium bicarbonate (51mg), water (10ml) and 10% palladium-on-charcoal (250mg), the title compound (125mg) was obtained.

$\delta$ ($D_2O$) 1.30 (3H,d,J=6.3Hz); 3.98 (1H,dd,J=1.5 and 5.9Hz); 4.25 (1H,m); 5.78 (1H,d,J=1.5Hz); 7.48 (1H,t,J=8.0Hz); 7.60 (1H,dt,J=1.4 and 7.9Hz); 7.75-7.79 (2H,m).

Example 97

4-Nitrobenzyl 5R,3-(3-aminocarbonylmethylaminocarbonylphenyl)-6S-(1R-hydroxyethyl)-7-oxo-4-thia-1-azabicyclo[3.2.0]hept-2-ene-2-carboxylate.

By a process analogous to that used in Example 38, but using 4-nitrobenzyl 5R,3-(3-carboxyphenyl)-6S-(1R-hydroxyethyl)-7-oxo-4-thia-1-azabicyclo[3.2.0]hept-2-ene-2-carboxylate (243mg), HOBT (140mg), 1-(3-dimethylaminopropyl)-3-ethylcarbodiimide hydrochloride (148mg), acetonitrile (20ml), tetrahydrofuran (2ml), glycinamide hydrochloride (285mg) and triethylamine (261mg) the title compound was obtained as a yellow solid (132mg).

$\delta$ (acetone-$d_6$) 1.32 (3H,d,J=6.3Hz); 3.96 (1H,dd,J=1.7 and 6.3Hz); 4.03 (2H,d,J=5.6Hz); 4.22 (1H,m); 4.47 (1H,d,J=4.8Hz); 5.18@and 5.31 (2H,AB,$J_{gem}$=13.8Hz); 5.91 (1H,d,J=1.6Hz); 6.44 (1H,bs); 7.05 (1H,bs); 7.49 and 8.16 (4H,AA'BB',J=8.8Hz); 7.52 (1H,m); 7.64 (1H,dt,J=1.3 and 7.8Hz); 7.97 (1H,dt,J=1.4 and 7.8 Hz); 8.04 (1H,m).

Example 98

Potassium 5R,3-(3-aminocarbonylmethylaminocarbonylphenyl)-6S-(1R-hydroxyethyl)-7-oxo-4-thia-1-azabicyclo[3.2.0]hept-2-ene-2-carboxylate.

By a procedure analogous to that used in Example 36, and using 4-nitrobenzyl 5R,3-(3-aminocarbonylmethylaminocarbonylphenyl)-6S-(1R-hydroxyethyl)-7-oxo-4-thia-1-azabicyclo[3.2.0]hept-2-ene-2-carboxylate (130mg), dioxane (10ml), potassium bicarbonate (24.7mg), water (10ml) and 10% palladium-on-charcoal (130mg), the title compound (102mg) was obtained.

($D_2O$) 1.30 (3H,d,J=6.4Hz); 4.00 (1H,dd,J=1.4 and 5.9Hz); 4.08 (2H,s); 4.24 (1H,m); 5.80 (1H,d,J=1.4Hz); 7.48-7.82 (4H,m).

Example 99

5R,3-(3-Cyanomethylaminocarbonylphenyl)-6S-(1R-hydroxyethyl)-7-oxo-4-thia-1-azabicyclo[3.2.0]hept-2-ene-2-carboxylic acid.

By a procedure analogous to Example 34, but using 4-nitrobenzyl 5R,3-(3-carboxyphenyl)-6S-{1R-[dimethyl-(2-methylprop-2-yl)silyloxy]ethyl}-7-oxo-4-thia-1-azabicyclo[3.2.0]hept-2-ene-2-carboxylate (400mg), HOBT (186mg), acetonitrile (10ml), tetrahydrofuran (2ml) and 1-(3-dimethylaminopropyl)-3-ethylcarbodiimide hydrochloride (198mg), triethylamine (209mg), and aminoacetonitrile hydrochloride (192mg), there was obtained 4-nitrobenzyl 5R,3-(3-cyanomethylaminocarbonylphenyl)-6S-{1R-[dimethyl-(2-methylprop-2-yl)silyloxy]ethyl}-7-oxo-4-thia-1-azabicyclo[3.2.0]hept-2-ene-2-carboxylate (165mg).

($CDCl_3$) 0.05 (3H,s); 0.08 (3H,s); 0.84 (9H,s); 1.26 (3H,d,J=6Hz); 3.83 (1H,m); 4.27 (1H,m); 4.33 (2H,d,J=5.7Hz); 5.08 and 5.25 (2H,AB,$J_{gem}$=13.7Hz);

5.75 (1H,d,J=1.5Hz); 7.11 (1H,t,J=5.7Hz); 7.41 and 8.12 (4H,AA'BB',J=8.9Hz); 7.45 (1H,m); 7.61 (1H,dt,J=7.9 and 1.4Hz);7.80 (1H,dt,J=1.4 and 7.9Hz); 7.94 (1H,t,J=1.4Hz)

This product (159mg) was treated by a process analogous to that used in Example 35 but using glacial acetic acid (187mg) and a solution of tetra-n-butylammonium fluoride in tetrahydrofuran (1M, 0.93ml) and tetrahydrofuran (5ml) to afford 4-nitrobenzyl 5R,3-(3-cyanomethylaminocarbonylphenyl)-6S-(1R-hydroxyethyl)-7-oxo-4-thia-1-azabicyclo[3.2.0]hept-2-ene-2-carboxylate as a viscous yellow oil (89mg):

(acetone-$d_6$) 1.22 (3H,d,J=6.3Hz); 3.86 (1H,dd,J=1.6 and 6.3Hz); 4.13 (1H,m); 4.31 (2H,d,J=5.7Hz); 4.50 (1H,broad); 5.08 and 5.21 (2H,AB,$J_{gem}$=13.8Hz); 5.81 (1H,d,J=1.6Hz); 7.39 and 8.05 (4H,AA'BB',J=8.5Hz); 7.44 (1H,m); 7.59 (1H,dt,J=1.5 and 7.6Hz); 7.86 (1H,dt,J=1.5 and 7.6Hz); 7.92 (1H,t,J=1.5Hz).

This 4-nitrobenzyl ester (89mg) was treated in a process analogous to that used in Example 36 with water (10ml), dioxane (10ml), potassium bicarbonate (17.5mg) and 10% palladium-on-charcoal (90mg) to afford the corresponding potassium salt (66mg). This crude product was chromatographed over a silylated silica gel, and elution with acetonitrile-water-formic acid mixtures afforded the title carboxylic acid (20mg).

($D_2O$ containing $KHCO_3$) 1.30 (3H,d,J=6.4Hz); 3.99 (1H,dd,J=1.5 and 4.8Hz); 4.25 (1H,m); 4.36 (2H,s); 5.78 (1H,d,J=1.5Hz); 7.50 (1H,t,J=8Hz); 7.63 (1H,d,J=8Hz); 7.77 (2H,m).

Example 100

Potassium 5R,6S-(1R-hydroxyethyl)-3-[4-(2-hydroxyethyl)-aminocarbonylphenyl]-7-oxo-4-thia-1-azabicyclo[3.2.0]-hept-2-ene-2-carboxylate.

By a procedure analogous to that used in Example 34 and using 4-nitrobenzyl 5R,3-(4-carboxyphenyl)-6S-{1R-[dimethyl(2-methylprop-2-yl)silyloxy]ethyl}-7-oxo-4-thia-1-azabicyclo[3.2.0]hept-2-ene-2-carboxylate (117mg), acetonitrile (10ml), HOBT (54mg), tetrahydrofuran (2ml) and 1-(3-dimethylaminopropyl)-3-ethyl-carbodiimide hydrochloride (58mg) and ethanolamine (13.4mg) there was obtained as a yellow foam 4-nitrobenzyl 5R,6S-{1R-[dimethyl-(2-methylprop-2-yl)-silyloxy]ethyl}-3-[4-(2-hydroxyethyl)aminocarbonylphenyl]-7-oxo-4-thia-1-azabicyclo[3.2.0]hept-2-ene-2-carboxylate (94mg).

($CDCl_3$) 0.06 (3H,s); 0.08 (3H,s); 0.85 (9H,s); 1.27 (3H,d,J=6.3Hz); 3.58 (2H,m); 3.78 (2H,m); 3.84 (1H,dd,J=1.6 and 4Hz); 4.29 (1H,m); 5.08 and 5.26 (2H,AB,$J_{gem}$=13.7Hz); 5.76 (1H,d,J=1.6Hz);7.06 (1H,broad m); 7.41 (2H,d,J=8.7Hz); 7.46 (2H,d,J=8.4Hz); 7.73 (2H,d,J=8.4Hz); 8.13 (2H,d,J=8.7Hz).

This product (94mg) was treated by a process analogous to that used in Example 35 using glacial acetic acid (90mg), a solution of tetra-n-butylammonium fluoride in tetrahydrofuran (1M, 0.45 ml) and tetrahydrofuran (10ml) to afford 4-nitrobenzyl 5R,6S-(1R-hydroxyethyl)-3-[4-(2-hydroxyethyl)aminocarbonylphenyl]-7-oxo-4-thia-1-azabicyclo[3.2.0]hept-2-ene-2-carboxylate (30mg).

(acetone-$d_6$) 1.31 (3H,d,J=6Hz); 3.53 (2H,m); 3.70 (2H,m); 3.95 (1H,dd,J=1.3 and 6.3Hz); 4.22 (1H,m); 5.18 and 5.31 (2H,AB,$J_{gem}$=14Hz); 5.90 (1H,d,J=1.3Hz); 7.46 (2H,d,J=8.7Hz); 7.53 (2H,d,J=8.3Hz); 7.89 (2H,d,J=8.3Hz); 8.15 (2H,d,J=8.7Hz).

This product (30mg) was treated in a process analogous to that used in Example 36 but using dioxane (5ml), water (5ml), potassium bicarbonate (5.9mg) and

10% palladium-on-charcoal (30mg) to afford the title potassium salt (24mg).

δ ($D_2O$) 1.30 (3H,d,J=6.4Hz); 3.53 (2H,m); 3.76 (2H,m); 3.94 '1H,m); 4.28 (1H,m); 5.81 (1H,d,J=1.5Hz); 7.51 and 7.73 (4H,AA'BB',J=8.3Hz).

EXAMPLE 101

2-(Trimethylsilyl)ethyl 5R,3-(3-aminophenyl)-6S-(1R-hydroxyethyl)-7-oxo-4-thia-1-azabicyclo[3.2.0]hept-2-ene-2-carboxylate.

By a process analogous to that used in Example 24 and using 3-nitrobenzoyl chloride (10 g), dichloromethane (200 ml), pyridine (8.7 ml) and hydrogen sulphide (excess) there was obtained 3-(nitro)-thiobenzoic acid as a pale yellow solid (9.3 g).

δ ($CDCl_3$) 4.8 (1H,broad); 7.72 (1H,t,J=8.0Hz); 8.23 (1H,dt,J=1.4 and 7.8Hz); 8.47 (1H,dt,J=1.2 and 8.2Hz); 8.74 (1H,t,J=1.8Hz).

This 3-(nitro)-thiobenzoic acid (9.3 g) was reacted by a procedure analogous to that used in Example 16 with a mixture of 4-acetoxy-3R-{1R-[dimethyl(2-methylprop-2-yl)silyloxy)ethyl}-azetidin-2-one (10g), and sodium hydroxide (2.08g) in acetone (250ml) and water (52ml) to afford 3S-{1R-[dimethyl(2-methylprop-2-yl)silyloxy]ethyl}-4R-(3-nitrobenzoylthio)azetidin-2-one (13g)

$\nu_{max}$ (film) 1770 $cm^{-1}$

δ ($CDCl_3$) 0.10 (6H,s); 0.90 (9H,s); 1.26 (3H,d,J=6.3Hz); 3.34 (1H,~t,J~3Hz); 4.33 (1H,m); 5.53 (1H,d,J=2.4Hz); 6.54 (1H,s); 7.72 (1H,t,J=8Hz); 8.25 (1H,ddd,J=1,2 and 8Hz); 8.49 (1H,ddd,J=1, 2 and 8Hz); 8.76 (1H,t,J=2Hz).

Treatment of this azetidinone (13g) in a procedure analogous to that used in Example 17 by using dichloromethane (300ml), calcium carbonate (13g), diisopropylethylamine (11ml) and 2-(trimethylsilyl)ethyl

chlorooxoacetate (8.9ml) afforded 2-(trimethylsilyl)-ethyl {3S-[1R-(dimethyl(2-methylprop-2-yl)silyloxy)-ethyl]-4R-(3-nitrobenzoylthio)azetidin-2-on-1-yl}-oxoacetate

δ ($CDCl_3$) 0.08 (6H,s); 0.09 (9H,s); 0.91 (11H,s and m); 1.27 (3H,d,J=6.7Hz); 3.65 (1H,t,J=3Hz); 4.42 (3H,m); 6.23 (1H,d,J=3Hz); 7.72 (1H,t,J=8Hz); 8.28 (1H,ddd,J=1, 2 and 8Hz); 8.49 (1H,ddd,J=1, 2 and 8Hz); 8.80 (1H,t,J=2Hz).

This oxoacetate was immediately treated in a procedure analogous to that used in Example 18 with triethyl phosphite (10.8ml), hydroquinone (10mg) and o-xylene (300ml) to afford 2-(trimethylsilyl)ethyl 5R,6S-{1R-[dimethyl(2-methylprop-2-yl)silyloxy]ethyl}-3-(3-nitrophenyl)-7-oxo-4-thia-1-azabicyclo[3,2,0]hept-2-ene-2-carboxylate (10.5g)

$\nu_{max}$($CDCl_3$) 1792, 1710, 1535 $cm^{-1}$;

δ ($CDCl_3$) 0.01 (9H,s); 0.10 (3H,s); 0.12 (3H,s); 0.94 (11H,s and m); 1.28 (3H,d,J=6.3Hz); 3.82 (1H,dd,J = 1.6 and 4.6Hz); 4.15-4.42 (3H,m); 5.75 (1H,d,J=1.6Hz); 7.57 (1H,t, J=8Hz); 7.80 (1H,dt,J=1 and 8Hz); 8.25 (1H,ddd,J=1, 2 and 8Hz); 8.36 (1H,t,J=2Hz).

Treatment of this silylated penem (1g) in a procedure analogous to that used in Example 19 with glacial acetic acid (1ml), tetrahydrofuran (20ml) and a solution of tetra-n-butylammonium fluoride in tetrahydrofuran (1M, 5.6ml) afforded 2-(trimethylsilyl)-ethyl 5R,6S-(1R-hydroxyethyl)-3-(3-nitrophenyl)-7-oxo-4-thia-1-azabicyclo[3,2,0]hept-2-ene-2-carboxylate (300mg)

$\nu_{max}$($CDCl_3$) 1785, 1710, 1532 $cm^{-1}$

δ ($CDCl_3$) 0.01 (9H,s); 0.91 (2H,m); 1.39 (3H,d,J=6.3Hz); 3.86 (1H,dd,J=1.6 and 6.6Hz); 4.1-4.3 (3H,m); 5.78 (1H,d,J=1.6Hz); 7.57 (1H,t,J=8Hz); 7.79 (1H,dt,J=1 and 8Hz); 8.26 (1H,ddd,J=1, 2 and 8Hz); 8.35 (1H,t,J=2Hz).

A solution of this 3-nitrophenyl-penem-carboxylate (4.5g) in dry ethyl acetate (100ml) was hydrogenolysed at 375 kPa (4 atm) at $20^0$ in the presence of platinum dioxide (1.1g), was filtered and evaporated in vacuo. Chromatography of the residue over silica gel, and elution with ethyl acetate - hexane mixtures afforded the title 3-aminophenyl compound $\nu_{max}(CDCl_3)$ 3600(broad),3400(broad), 3178, 1710,1621cm$^{-1}$ $\delta$ $(CDCl_3)$ 0.01 (9H,s); 0.93 (2H,m); 1.36 (3H,d,J=6.3Hz); 2.84 (2H,broad); 3.78 (1H,dd,J=1.5 and 6.6Hz); 4.18 (3H,m); 5.67 (1H,d,J=1.5Hz); 6.70 (1H,dd,J=2.2 and 7.3Hz); 6.77 (1H,m); 6.83 (1H,dd,J=7.7 and 1Hz); 7.14 (1H,t,J=7.7Hz).

Example 102

Potassium 5R,3-(3-formylaminophenyl)-6S-(1R-hydroxyethyl)-7-oxo-4-thia-1-azabicyclo[3,2,0]hept-2-ene-2-carboxylate.

By a procedure analogous to that used in Example 87 and using 2-(trimethylsilyl)ethyl 5R,3-(3-aminophenyl)-6S-(1R-hydroxyethyl)-7-oxo-4-thia-1-azabicyclo[3,2,0]-hept-2-ene-2-carboxylate (200mg) in dry dichloromethane (5ml), formic acid (0.022ml) and 1-(3-dimethylaminopropyl)-3-ethylcarbodiimide hydrochloride (113mg) there was obtained 2-(trimethylsilyl)ethyl 5R,3-(3-formylaminophenyl)-6S-(1R-hydroxyethyl)-7-oxo-4-thia-1-azabicyclo[3,2,0]hept-2-ene-2-carboxylate (171mg): $\nu_{max}(CDCl_3)$ 3500(sh), 3320(broad), 1787, 1695 cm$^{-1}$; $\delta$ $(CDCl_3)$ [observed as a mixture of both formamide conformers] (a) 0.01 (6H,s); 0.91 (2H,m); 1.36 (3H,d, J=6.3Hz); 2.73 (1H,broad); 3.80 (2H,m); 4.17 (3H,m); 5.70 (1H,d,J=1.4Hz); 7.1-7.6 (4H,m); 7.81 (1H,d, J=1.6Hz); 8.33 (1H,d,J=1.6Hz); and (b) 0.01 (6H,s); 0.91 (2H,m); 1.37 (3H,d,J=6.3Hz);

2.68 (1H,broad); 3.80 (2H,m); 4.17 (3H,m); 5.72 (1H,d,J=1.4Hz); 7.1-7.6 (4H,m); 8.19 (1H,d,J=11.3Hz); 8.67 (1H,d,J=11.3Hz).

A solution of this product (168mg) in tetrahydrofuran (5ml) was then treated by a process analogous to that used in Example 88 with a dry solution of tetra-n-butylammonium fluoride in tetrahydrofuran (1M, 0.387ml) and finally with potassium hydrogen carbonate (38.7mg) to afford the crude product (218mg). Purification as in Example 88 then afforded the title compound (37mg). δ ($D_2O$) 1.31 (3H,d,J=6.5Hz); 3.98 (1H,dd,J=1.3 and 5.8Hz); 4.24 (1H,m); 5.77 (1H,d,J=1.3Hz); 7.22-7.27 (2H,m); 7.39 (1H,t,J=7.4Hz); 7.48 (1H,m); 8.24 (1H,s).

Example 103

Potassium 5R,3-(3-acetylaminophenyl)-6S-(1R-hydroxyethyl)-7-oxo-4-thia-1-azabicyclo[3,2,0]hept-2-ene-2-carboxylate.

A mixture of 2-(trimethylsilyl)ethyl 5R,3-(3-aminophenyl)-6S-(1R-hydroxyethyl)-7-oxo-4-thia-1-azabicyclo-[3,2,0]hept-2-ene-2-carboxylate (200mg), tetrahydrofuran (3ml), acetic anhydride (0.051ml) and N-methylmorpholine (0.06ml) was stirred at 20° for 1 hour, was then partitioned between ethyl acetate and water. The organic layer was washed with water, with 1M-citric acid, with saturated sodium bicarbonate solution, and dried. Evaporation in vacuo and chromatography of the residue over silica gel, and elution with ethyl acetate - hexane mixtures afforded 2-(trimethylsilyl)ethyl 5R,3-(3-acetylaminophenyl)-6S-(1R-hydroxyethyl)-7-oxo-4-thia-1-azabicyclo[3,2,0]hept-2-ene-2-carboxylate (210mg); $\nu_{max}$($CDCl_3$) 3350(broad), 1782, 1680 $cm^{-1}$; δ ($CDCl_3$) 0.01 (9H,s); 0.95 (2H,m); 1.39 (3H,d,J=6.3Hz); 2.18 (3H,s); 3.81 (1H,dd,J=1.5 and 6.8Hz); 4.1-4.3 (3H,m); 5.70 (1H,d,J=1.5Hz); 7.1-7.7 (5H,m).

This material (194mg) was treated in a process analogous to that used in Example 88 with dry tetrahydrofuran (5ml), a solution of tetra-n-butylammonium fluoride in tetrahydrofuran (1M,0.433ml) and potassium hydrogen carbonate (43.3mg), to afford crude title compound (260mg). Purification as in Example 88 afforded pure title compound (47mg).
δ ($D_2O$) 1.30 (3H,d,J=6.4Hz); 2.14 (3H,s); 3.98 (1H,dd, J=5.9 and 1.4Hz); 4.25 (1H,m); 5.77 (1H,d,J=1.4Hz); 7.23 (1H,dt,J=7.2 and 1.4Hz); 7.3-7.5 (3H,m).

Example 104

5R,3-(4-[Cyanoacetamido]phenyl)-6S-(1R-hydroxyethyl)-7-oxo-4-thia-1-azabicyclo[3.2.0]hept-2-ene-2-carboxylic acid.

To a stirred solution of 2-(trimethylsilyl)ethyl 5R,3-(4-aminophenyl)-6S-(1R-hydroxyethyl)-7-oxo-4-thia-1-azabicyclo[3.2.0]hept-2-ene-2-carboxylate (400mg) in tetrahydrofuran (20ml) was added cyanoacetic acid (126mg) and dicyclohexylcarbodiimide (304mg). After 30 minutes, the mixture was filtered; the filtrate was partitioned between ethyl acetate and water. The organic layer was washed with 1M-citric acid, with saturated aqueous sodium bicarbonate and brine, was dried and evaporated *in vacuo*. Chromatography of the residue over silica gel and elution with hexane - ethyl acetate mixtures afforded 2-(trimethylsilyl)ethyl 5R,3-(4-[cyanoacetamido]phenyl)-6S-(1R-hydroxyethyl)-7-oxo-4-thia-1-azabicyclo[3.2.0]-hept-2-ene-2-carboxylate (177mg):
$\nu_{max}$($CDCl_3$) 1788 and 1710 $cm^{-1}$;
δ ($CDCl_3$) 0.01 (9H,s); 0.98 (2H,m); 1.38 (3H,d,J=6.3Hz); 3.59 (2H,s); 3.80 (1H,dd,J=1.6 and 7.1 Hz); 4.20 (3H,m); 5.68 (1H,d,J=1.6Hz); 7.53 (4H,m); 8.14 (1H,s).

In a process analogous to that used in Example 88, a solution of this ester (177mg) in dry tetrahydrofuran (5ml) was treated with a 1M solution of dry tetrabutylammonium fluoride (0.374ml), with addition of potassium hydrogen carbonate (37mg) to afford a crude product (145mg) which was purified by reverse-phase HPLC to afford the title acid (9mg):
δ ($D_2O$, containing a trace of $KHCO_3$) 1.30 (3H,d, J=6.4Hz); 3.96 (1H,dd,J=1.4 and 6.0Hz); 4.25 (1H,m); 5.75 (1H,d,J=1.4Hz); 7.44 (4H,s).

Example 105

5R,6S-(1R-Hydroxyethyl)-3-(4-[methylaminothioformylamino]phenyl)-7-oxo-4-thia-1-azabicyclo[3.2.0]hept-2-ene-2-carboxylic acid.

A mixture of 2-(trimethylsilyl)ethyl 5R,3-(4-aminophenyl)-6S-(1R-hydroxyethyl)-7-oxo-4-thia-1-azabicyclo-[3.2.0]hept-2-ene-2-carboxylate (200mg), tetrahydrofuran (5ml) and methyl isothiocyanate (72mg) was stirred for 16 hours, and was then partitioned between ethyl acetate and water. The organic layer was washed with water, with cold 1M-hydrochloric acid, with water, with saturated aqueous sodium bicarbonate, with brine and was evaporated in-vacuo. Chromatography of the residue over silica gel and elution with ethyl acetate - hexane mixtures afforded 2-(trimethylsilyl)ethyl 5R,6S-(1R-hydroxyethyl)-3-(4-[methylaminothioformylamino]-phenyl)-7-oxo-4-thia-1-aza-bicyclo[3.2.0]hept-2-ene-2-carboxylate (80mg):
$\nu_{max}$ ($CDCl_3$) 3600, 3410, 1788, 1708 $cm^{-1}$;
δ ($CDCl_3$) 0.01 (9H,s); 0.99 (2H,m); 1.39 (3H,d,J=6.3Hz); 1.96 (1H,broad); 3.16 (3H,d,J=4.5Hz); 3.81 (1H,dd,J=1.6 and 6.6Hz); 4.23 (3H,m); 5.71 (1H,d,J=1.6Hz); 6.24 (1H,broad); 7.20 and 7.55 (4H,AA'BB',J=8.5Hz); 7.74 (1H,s).

This ester (200mg) was treated in a process analogous to that used in Example 88 with tetrahydrofuran (10ml) and a solution of dry tetrabutylammonium fluoride in tetrahydrofuran (1M, 0.417ml), with addition of potassium bicarbonate (42mg). After ion-exchange and reverse-phase chromatography there was obtained the title acid (16mg):

δ ($D_2O$, containing $KHCO_3$) 1.29 (3H,d,J=6.4Hz); 2.96 (3H,s); 3.96 (1H,dd,J=1.3 and 5.9Hz); 4.24 (1H,m); 5.75 (1H,d,J=1.3Hz); 7.23 and 7.43 (4H,AA'BB',J=8.6Hz).

Example 106

Potassium 5R,6S-(1R-hydroxyethyl)-3-(3-methylsulphinylphenyl)-7-oxo-4-thia-1-aza-bicyclo[3.2.0]hept-2-ene-2-carboxylate.

By procedures analogous to those used in Examples 15,16,17,18 and 19, and starting from 3-(methylthio)-benzoic acid there was obtained 4-nitrobenzyl 5R,6S-(1R-hydroxyethyl)-3-(3-methylthiophenyl)-7-oxo-4-thia-1-aza-bicyclo[3.2.0]hept-2-ene-2-carboxylate as a yellow foam: δ ($CDCl_3$) 1.40 (3H,d,J=6.4Hz); 2.10 (1H,d,J=4.3Hz); 2.44 (3H,s); 3.86 (1H,dd,J=1.5 and 6.4Hz); 4.31 (1H,m); 5.12 and 5.28 (2H,AB,$J_{gem}$=13.8Hz); 5.75 (1H,d,J=1.5Hz); 7.17 (1H,m); 7.28 (3H,m); 8.13 (2H,d,J=8.8Hz).

This sulphide (183mg) was treated by a process analogous to that used in Example 20 with 80% 3-chloroperbenzoic acid (92mg) to afford 4-nitrobenzyl 5R,6S-(1R-hydroxyethyl)-3-(3-methylsulphinylphenyl)-7-oxo-4-thia-1-azabicyclo[3.2.0]hept-2-ene-2-carboxylate (107mg) δ (acetone-$d_6$) 1.33 (3H,d,J=6.3Hz); 2.67 (3H,s); 3.98 (1H,dd,J=1.7 and 6.2Hz); 4.23 (1H,m); 4.48 (1H,d,J=4.9Hz); 5.21 and 5.35 (2H,AB,$J_{gem}$=14.2Hz); 5.93 (1H,d,J=1.7Hz); 7.51 and 8.16 (4H,AA'BB',J=8.5Hz); 7.6-7.8 (4H,m).

In a process analogous to that used in Example 7 this sulphoxide (107mg) was hydrogenolysed in the presence of 10% palladium-on-charcoal (100mg), potassium hydrogen carbonate, dioxane (6ml) and water (6ml) to afford the title compound (65mg): δ ($D_2O$) 1.29 (3H,d,J=6.5Hz); 2.85 (3H,s); 3.99 (1H,dd,J=1.5 and 6Hz); 4.25 (1H,m); 5.79 (1H,d,J=1.5Hz); 7.58-7.80 (4H,m).

Example 107

Potassium 5R,6S-(1R-hydroxyethyl)-3-(3-methylsulphonylphenyl)-7-oxo-4-thia-1-azabicyclo[3.2.0]hept-2-ene-2-carboxylate.

By a procedure analogous to that used in Example 22, and using 4-nitrobenzyl 5R,6S-(1R-hydroxyethyl)-3-(3-methylsulphinylphenyl)-7-oxo-4-thia-1-azabicyclo-[3.2.0]hept-2-ene-2-carboxylate (414mg), 80% 3-chloroperbenzoic acid (165mg) and ethyl acetate (40ml), there was obtained 4-nitrobenzyl 5R,6S-(1R-hydroxyethyl)-3-(3-methylsulphonylphenyl)-7-oxo-4-thia-1-azabicyclo[3.2.0]-hept-2-ene-2-carboxylate (76mg): δ (acetone-$d_6$) 1.33 ((3H,d,J=6.3Hz); 2.84 (3H,s); 4.00 (1H,dd,J=1.7 and 6.1Hz); 4.23 (1H,m); 4.47 (1H,d,J=4.9Hz); 5.20 and 5.35 (2H,AB,$J_{gem}$=14Hz); 5.95 (1H,d,J=1.7Hz); 7.52 and 8.18 (4H,AA'BB',J=8.9Hz); 7.70 (1H,t,J=7.6Hz); 7.85 (1H,~d,J=7.4Hz); 8.00 (1H,dd,J=1.2 and 7.3Hz); 8.06 (1H,m).

In a procedure analogous to that used in Example 7, this sulphone (76mg) was hydrogenolysed in the presence of 10% palladium-on-charcoal (75mg), potassium hydrogen carbonate (15.3mg), dioxane (4ml) and water (4ml) to afford the title potassium salt (39mg): δ ($D_2O$) 1.31 (3H,d,J=6.4Hz); 3.25 (3H,s); 4.00 (1H,dd,J=1.4 and 5.9Hz) 4.26 (1H,m); 5.80 (1H,d,J=1.4Hz); 7.63 (1H,t,J=7.8Hz); 7.77 (1H,m); 7.91 (1H,dm, J~7.8Hz); 7.98 (1H,t,J~1.6Hz).

Example 108

2-(Trimethylsilyl)ethyl 5R,6S-(1R-acetoxyethyl)-3-(4-formylaminophenyl)-7-oxo-4-thia-1-aza-bicyclo[3.2.0]-hept-2-ene-2-carboxylate

A mixture of 2-(trimethylsilyl)ethyl 5R,3-(4-formylamino)phenyl-6S-(1R-hydroxyethyl)-7-oxo-4-thia-1-azabicyclo[3.2.0]hept-2-ene-2-carboxylate (500mg), tetrahydrofuran (10ml), 4-dimethylaminopyridine (15mg) and acetic anhydride (1.09ml) was stirred at room temperature for 30 min., and then partitioned between ethyl acetate and water. The organic layer was washed with saturated aqueous sodium bicarbonate, with brine, was dried over anhydrous sodium sulphate, and evaporated in vacuo. Chromatography on silica gel and elution with ethyl acetate - hexane mixtures afforded the title compound (211mg):

$\delta$ ($CDCl_3$) 0.00 (9H,s); 0.97 (2H,m); 1.42 (3H,d,J=6.4Hz); 2.08 (3H,s); 3.90 (1H,dd,J=1.5 and 7.6Hz); 4.20 (2H,m); 5.29 (1H,m); 5.64 and 5.62 (1H,2d,J=1.5Hz); 7.06 (~0.8H, d,J=8.6Hz); 7.4-7.6 (~3.8H,m); 7.88 (~0.4H,d,J=11.4Hz); 8.37 (~0.6H,d,J=1.6Hz) and 8.75 (~0.4H,d,J=11.4Hz).

$\delta$ (DMSO-$d_6$) 0.00 (9H,s); 0.84 (2H,t,J=7.5Hz); 1.30 (3H,d,J=6.3Hz); 2.03 (3H,s); 4.12 (2H,m); 4.21 (1H,dd,J=1.5 and 5.5Hz); 5.17 (1H,m); 5.78 (1H,d,J=1.5Hz); 7.43 and 7.62 (4H,AA'BB',$J_{AB}$=8.8Hz); 8.31 (~0.8H,d,J=1.5Hz); 8.89 (~0.2H,d,J=11.5Hz); 9.36 (~0.2H,d,J=11.5Hz) and 10.44 (~0.8H,d,J =1.5Hz).

Example 109

5R,6S-(1R-Acetoxyethyl)-3-(4-formylaminophenyl)-7-oxo-4-thia-1-azabicyclo[3.2.0]hept-2-ene-2-carboxylic acid

The crude product obtained by a procedure analogous to that used in Example 88 but using 2-(trimethylsilyl)-ethyl 5R,6S-(1R-acetoxyethyl)-3-(4-formylaminophenyl)-7-oxo-4-thia-1-azabicyclo[3.2.0]hept-2-ene-2-carboxylate (200 mg), tetrahydrofuran (10ml) and a solution of dry tetrabutylammonium fluoride in tetrahydrofuran (1M, 0.41ml), with addition of potassium bicarbonate (41mg), was purified by ion-exchange and reverse-phase chromatography to afford the pure title compound (49mg): δ ($D_2O$) 1.41 (3H,d,J=6.3Hz); 2.15 (3H,s); 4.21 (1H,dd, J=1.5 and 5.5Hz); 5.30 (1H,m); 5.82 (1H,d,J=1.5Hz); 7.45 and 7.50 (4H,AA'BB',$J_{AB}$=8.8Hz); 8.25 (1H,bs).

Example 110

4-nitrobenzyl 5S,3-(3-aminocarbonylphenyl)-6S-(1R-hydroxyethyl-7-oxo-4-thia-1-azabicyclo[3.2.0]hept-2-ene-2-carboxylate

A mixture of 4-nitrobenzyl 5R,3-(3-aminocarbonylphenyl)-6S-(1R-hydroxyethyl)-7-oxo-4-thia-1-azabicyclo-[3.2.0]hept-2-ene-2-carboxylate (120mg) and acetonitrile (7.5ml) was heated in a sealed flask at 85±3° for 20 hours, and was then evaporated in vacuo. Chromatography of the residue afforded some starting trans-penem, and the title compound (20mg):
δ (DMSO-$d_6$) 1.48 (3H,d,J=6.3Hz); 4.02 (1H,dd,J=3.8 and 5.5Hz); 4.30 (1H,m); 5.15 and 5.30 (2H,AB,$J_{gem}$=13.8Hz); 5.95 (1H,d,J=3.8Hz); 7.2-8.1 (10H,m).

Example 111

Potassium 5S,3-(3-aminocarbonylphenyl)-6S-(1R-hydroxyethyl)-7-oxo-4-thia-1-azabicyclo[3.2.0]hept-2-ene-2-carboxylate

By a procedure analogous to that used in Example 36, and using 4-nitrobenzyl 5S,3-(3-aminocarbonyl-phenyl)-6S-(1R-hydroxyethyl)-7-oxo-4-thia-1-azabicyclo-[3.2.0]hept-2-ene-2-carboxylate (50mg), dioxane (5ml), water (5ml), potassium bicarbonate (10mg) and 10%

palladium-on-charcoal (50mg), the title compound 27mg) was obtained (27mg):
δ ($D_2O$) 1.42 (3H,d,J=6.3Hz); 4.05 (1H,dd,J=4 and 6 Hz); 4.26 (1H,m); 5.90 (1H,d,J=4Hz); 7.4-7.8 (4H,m).

Example 112

4-Nitrobenzyl 5R,3-[3-(allyloxycarbonyl)phenyl]-6S-(1R-hydroxyethyl)-7-oxo-4-thia-1-azabicyclo[3.2.0]hept-2-ene-2-carboxylate

By a procedure analogous to that used in Example 39, but starting from a solution of isophthaloyl chloride (60.9g) in dioxane (400ml), allyl alcohol (20.9g), triethylamine (36.4g), dichloromethane (600ml), pyridine (28.5g) and hydrogen sulphide (excess) there was obtained 3-(allyloxycarbonyl)thiobenzoic acid:
$\nu_{max}$ 2550, 1725 $cm^{-1}$;
δ ($CDCl_3$) 5.0 (2H,m); 5.5 (3H,broad m); 6.0 (1H,m); 7.5-9.1 (4H,m).

A solution of this thiobenzoic acid (5g) in acetone (35ml) was treated in a procedure analogous to that used in Example 30 with a mixture of 1M-sodium hydroxide solution (22ml) and a solution of 4-acetoxy-3R-{1R-[dimethyl(2-methylprop-2-yl)silyloxy]ethyl}-azetidin-2-one (4.3g) in acetone:water (3:1) (50ml) to afford 4R-[3-(allyloxycarbonyl]benzoylthio]-3S-{1R-[dimethyl(2-methylprop-2-yl)silyloxy]ethyl}azetidin-2-one (4.3g):
δ ($CDCl_3$) 0.00 (6H,s); 0.80 (9H,s); 1.15 (3H,d,J=6.3Hz); 3.22 (1H,dd,J=2.5 and 3.6Hz); 4.22 (1H,m); 4.76 (1H,dd,J=1.1 and 6.8Hz); 5.2-5.4 (2H,m); 5.41 (1H,d, J=2.5Hz); 5.92 (1H,m); 6.64 (1H,s); 7.5-8.6 (4H,m).

A solution of this azetidinone (22.1g) in dichloromethane (250ml) was treated in a procedure

analogous to that used in Example 31 with calcium carbonate (12.3g), and diisopropylethylamine (9.5g) and a solution of 4-nitrobenzyl chlorooxalate (14.4g) in dichloromethane (25ml) to afford as a yellow oil 4-nitrobenzyl {4R,3-[3-(allyloxycarbonyl)benzoylthio]-3S-{1R-[dimethyl(2-methylprop-2-yl)silyloxy]ethyl}-azetidin-2-on-1-yl}oxoacetate

δ ($CDCl_3$) 0.00 (3H,s); 0.09 (3H,s); 0.83 (9H,s); 1.26 (3H,d,J=6.3Hz); 3.65 (1H,~t,J=3.5Hz); 4.41 (1H,m); 4.85 (2H,~d,J=5.8Hz); 5.30-5.45 (4H,m); 6.02 (1H,m); 6.20 (1H,d,J=3.5Hz); 7.5-7.6 (3H,m); 8.09-8.3 (4H,m); 8.60 (1H,~t,J=1.6Hz).

A solution of this oil in xylene (500ml) was treated in a procedure analogous to that used in Example 32 with a solution of triethyl phosphite (16.3g) in xylene (100ml) to afford 4-nitrobenzyl 5R,3-[3-(allyloxycarbonyl)-phenyl]-6S-{1R-[dimethyl(2-methylprop-2-yl)silyloxy]-ethyl}-7-oxo-4-thia-1-azabicyclo[3.2.0]hept-2-ene-2-carboxylate (25.3g)

δ ($CDCl_3$) 0.06 (3H,s); 0.08 (3H,s); 0.85 (9H,s); 1.27 (3H,d,J=6.3Hz); 3.83 (1H,dd,J=1.3 and 3.9Hz); 4.28 (1H,m); 4.81 (2H,d,J=5.5Hz); 5.09 (1H,d,J=13.7Hz); 5.2-5.4 (3H,m); 5.75 (1H,d,J=1.3Hz); 6.00 (1H,m); 7.3-7.6 (4H,m); 8.1-8.3 (4H,m).

A solution of this 4-nitrobenzyl carboxylate (34.2g) in tetrahydrofuran (70ml) was treated in a procedure analogous to that used in Example 37 with glacial acetic acid (32.8g) and a 1M solution of tetrabutylammonium fluoride in tetrahydrofuran (163ml) to afford the title hydroxyethyl compound (20.4g):

δ ($CDCl_3$) 1.40 (3H,d,J=6.3Hz); 3.88 (1H,dd,J=1.6 and 6.5Hz); 4.31 (1H,m); 4.80 (2H,m); 5.10 (1H,d,J=13.7Hz);

5.2-5.5 (3H,m); 5.79 (1H,d,J=1.6Hz); 6.00 (1H,m); 7.33 (2H,d,J=8.8Hz); 7.44 (1H,t,J=7.8z); 7.61 (1H,m); 8.05-8.23 (4H,m).

Example 113

4-Nitrobenzyl 5R,6S-(1R-acetoxyethyl)-3-[3-(allyloxycarbonyl)phenyl]-7-oxo-4-thia-1-azabicyclo[3.2.0]hept-2-ene-2-carboxylate

A mixture of 4-nitrobenzyl 5R,3[3-(allyloxycarbonyl)phenyl]-6S-(1R-hydroxyethyl)-7-oxo-4-thia-1-azabicyclo[3.2.0]hept-2-ene-2-carboxylate (1.0g) dry tetrahydrofuran (15ml), acetic anhydride (1.0g) and 4-dimethylaminopyridine (23mg) was stirred under a nitrogen atmosphere for 30 min., and then partitioned between ethyl acetate and water. The organic layer was washed with water, with saturated aqueous sodium bicarbonate solution and was then dried and evaporated *in vacuo*. Chromatography of the residue over silica gel and elution with ethyl acetate - hexane mixtures afforded the title compound (0.9g): $\delta$ ($CDCl_3$) 1.45 (3H,d,J=6.3Hz); 2.09 (3H,s); 3.99 (1H,dd,J=1.8 and 5.8Hz); 4.81 (2H,~d,J=5.8Hz)); 5.13 (1H,d,J=13.7Hz); 5.2-5.4 (4H,m); 5.73 (1H,d,J=1.8Hz); 6.01 (1H,m); 7.35 (2H,d,J=8.7Hz); 7.45 (1H,~t,J=7.9Hz); 7.62 (1H,m); 8.07-8.16 (4H,m).

Example 114

4-Nitrobenzyl 5R,6S-(1R-acetoxyethyl)-3-(3-carboxyphenyl)-4-thia-1-azabicyclo[3.2.0]hept-2-ene-2-carboxylate

A mixture of 4-nitrobenzyl 5R,6S-(1R-acetoxyethyl)-3-[3-(allyloxycarbonyl)phenyl]-7-oxo-4-thia-1-azabicyclo[3.2.0]hept-2-ene-2-carboxylate (0.9g), dichloromethane (10ml), potassium 2-ethylhexanoate

(0.46g), triphenylphosphine (17mg) and tetrakis-(triphenylphosphine)palladium (38mg) was stirred at room temperature for one hour, and the pH was then adjusted to 4.4 by addition of potassium dihydrogen phosphate solution. The resulting mixture was extracted with ethyl acetate, which was then dried and evaporated in vacuo. Chromatography of the residue and elution with hexane - ethyl acetate - formic acid mixtures afforded the title compound:

δ ($CDCl_3$) 1.45 (3H,d,J=6.4Hz); 2.09 (3H,s); 3.99 (1H,dd,J=1.5 and 7.5Hz); 5.13 and 5.26 (2H,AB, $J_{gem}$=13.1Hz); 5.31 (1H,m); 5.73 (1H,d,J=1.5Hz); 7.36 (2H,d,J=8.8Hz); 7.45-7.72 (2H,m); 7.9-8.2 (4H,m).

Example 115

4-Nitrobenzyl 5R,6S-(1R-acetoxyethyl)-3-(3-aminocarbonylphenyl)-7-oxo-4-thia-1-azabicyclo[3.2.0]hept-2-ene-2-carboxylate

To a stirred suspension of 4-nitrobenzyl 5R,6S-(1R-acetoxyethyl)-3-(3-carboxyphenyl)-7-oxo-4-thia-1-azabicyclo[3.2.0]hept-2-ene-2-carboxylate (200mg) in dry dichloromethane (10ml) at 0 was added triethylamine (0.11ml) and ethyl chloroformate (47mg), followed after one hour by a solution of ammonia (20mg) in ethanol (1ml). The mixture was stirred a further hour, and was then evaporated in vacuo. The residue was chromatographed on silica gel. Elution with ethyl acetate - hexane mixtures afforded the title compound (98mg):

δ (DMSO-$d_6$) 1.42 (3H,d,J=6.3Hz); 2.13 (3H,s); 4.22 (1H,dd,J=1.5 and 6 Hz); 5.15 and 5.30 (2H,AB, $J_{gem}$=13.8Hz); 5.14 (1H,d,J=6Hz); 5.92 (1H,d,J=1.5Hz); 6.85 (1H,bs); 7.47-7.8 (4H,m); 7.99 (1H,dt,J=1 and 8Hz); 8.03 (1H,t,J=1Hz); 8.13 (2H,d,J=8.8Hz).

Alternatively, the title compound (83mg) was obtained by a procedure analogous to that used in Example 113 by using 4-nitrobenzyl 5R,3-(3-aminocarbonylphenyl)-6S-(1R-hydroxyethyl)-7-oxo-4-thia-1-azabicyclo[3.2.0]hept-2-ene-2-carboxylate (200mg), dry tetrahydrofuran (3ml), acetic anhydride (0.2ml) and 4-dimethylaminopyridine (10mg).

Example 116

Potassium 5R,6S-(1R-acetoxyethyl)-3-(3-aminocarbonylphenyl)-7-oxo-4-thia-1-azabicyclo[3.2.0]hept-2-ene-2-carboxylate

By a procedure analogous to that used in Example 36, and using 4-nitrobenzyl 5R,6S-(1R-acetoxyethyl)-3-(3-aminocarbonylphenyl)-7-oxo-4-thia-1-azabicyclo[3.2.0]hept-2-ene-2-carboxylate (100mg), dioxane (1ml), potassium bicarbonate (19mg), water (1ml) and 10% palladium-on-charcoal (100mg), the title compound (57mg) was obtained:

$\delta$ ($D_2O$) 1.38 (3H,d,J=6.3Hz); 2.13 (3H,s); 4.20 (1H,dd,J=1.4 and 5.9Hz); 5.31 (1H,m); 5.88 (1H,d,J=1.4Hz); 7.48 (1H,t,J=8Hz); 7.60 (1H,dt,J=1.4 and 8Hz); 7.7-7.8 (2H,m).

Example 117

2-(Trimethylsilyl)ethyl-2-{3S-[1R-(dimethyl(2-methyl-prop-2-yl)silyloxy)ethyl]-4R-[4-nitrobenzoylthio]azetidin-2-on-1-yl}-3,3,3-triethoxy-3-phospha-propenoate

To a solution of 2-(trimethylsilyl)ethyl {3S-[1R-(dimethyl-(2-methylprop-2-yl)silyloxy)ethyl]-4R-[4-nitrobenzoylthio]-azetidin-2-on-1-yl oxoacetate (330 mg) in toluene (5 ml) at 100° was added dropwise over 30 minutes a solution of triethyl phosphite (0.24 ml) in toluene (1 ml). After the mixture had been heated at 100° for a further hour, it was evaporated in vacuo to afford a residue which was chromatographed over silica gel. Elution with hexane - ethyl acetate mixtures afforded the title compound (150 mg) as a yellow gum.

ν ($CDCl_3$) 1752, 1670 and 1620 $cm^{-1}$,

δ: ($CDCl_3$) 0.10 (6H,s); 0.84 (11H,m); 1.22 (3H,d, J=6.3Hz); 1.33 (9H,t,J=7Hz); 3.27 (1H,m); 4.21 (9H,m); 5.81 (1H,broad); 8.10 and 8.33 (4H,AA'BB',$J_{AB}$= 8.8Hz).

A mixture of this phosphorane (150 mg) and o-xylene (3 ml) was heated at 140° for one hour, and then evaporated in vacuo. Chromatography of the residue over silica gel and elution with hexane - ethyl acetate mixtures afforded 2-(trimethylsilyl) 5R,6S-{1R-[dimethyl(2-methylprop-2-yl)-silyloxy]ethyl}-3-(4-nitrophenyl)-7-oxo-4-thia-1-azabicyclo [3.2.0]hept-2-ene-2-carboxylate (97 mg) with properties identical to those described in Example 83.

Example 118

4-Nitrobenzyl 2-{4R-[3-aminocarbonylbenzoylthio]-3S-[1R-(dimethyl(2-methylprop-2-yl)silyloxy)ethyl]-azetidin-2-on-1-yl}-3-methylpropenoate.

A solution of silver 3S-{1R-[dimethyl(2-methylprop-2-yl)silyloxy]ethyl-1-[2-methyl-1-(4-nitrobenzyloxycarbonyl)propenyl]-azetidin-2-one-4R-thiolate (400 mg) in acetonitrile (10 ml) was added dropwise to a stirred solution of isophthaloyl chloride (612 mg) in acetonitrile (29 ml) at 0°. After 30 minutes a solution of ammonia (90 mg) in chloroform (5 ml) was added; the mixture was filtered and the filtrate evaporated *in vacuo*. Chromatography of the residue on silica gel, and elution with hexane - ethyl acetate mixtures afforded the title compound (206 mg).

δ: ($CDCl_3$) 0.03 (3H,s); 0.06 (3H,s); 0.82 (9H,s); 1.26 (3H,d,J=6.3Hz); 2.03 (3H,s); 2.21 (3H,s); 3.36 (1H,dd,J=2.6 and 5.6 Hz); 4.25 (1H,m); 5.34 (2H,s); 5.90 (1H,d,J=2.6 Hz); 6.2 (2H, broad); 7.50-7.61 (3H,m); 8.17-8.36 (5H,m).

Example 119

4-Nitrobenzyl 2-{4R-[3-aminocarbonylbenzoylthio]-3S-[1R-(dimethyl(2-methylprop-2-yl)silyloxy)ethyl]-azetidin-2-on-1-yl}oxoacetate.

A solution of 4-nitrobenzyl 2-{4R-[3-aminocarbonylbenzoyl-thio]-3S-[1R-(dimethyl(2-methylprop-2-yl)silyloxy)ethyl]-azetidin-2-on-1-yl}-3-methylpropenoate (182 mg) in dry dichloromethane (30 ml) was treated at -78° with ozone (excess); oxygen was then bubbled through the mixture for 5 minutes, and dimethyl sulphide (excess) was added. The mixture was warmed to room temperature, was evaporated *in vacuo*. The resulting oil was partitioned between ethyl acetate and water; the organic layer was separated, was washed with water, with brine, was dried and evaporated *in vacuo* to afford the title compound (140 mg).

δ ($CDCl_3$) 0.03 (3H,s); 0.06 (3H,s); 0.82 (9H,s); 1.27 (3H,d,J=6Hz); 3.64 (1H,~t,J=3Hz); 4.40 (1H,m); 5.36 and 5.43 (2H,AB,J=12.9Hz); 6.20 (1H,d,J=3.5Hz); 6.2 (2H,broad); 7.5-7.7 (3H,m); 8.1-8.4 (5H,m).

Example 120

4-Nitrobenzyl 5R,3-(3-aminocarbonylphenyl)-6S-{1R-[dimethyl(2-methylprop-2-yl)silyloxy]ethyl}-7-oxo-4-thia-1-azabicyclo[3.2.0]hept-2-ene-2-carboxylate.

To a mixture of 4-nitrobenzyl 2-{4R-[3-aminocarbonyl-benzoylthio]-3S-[1R-(dimethyl(2-methylprop-2-yl)-silyloxy)ethyl]-azetidin-2-on-1-yl}oxoacetate (140 mg) in xylene (15 ml) at 130° was added a mixture of triethyl phosphite (0.073 ml) in xylene (5 ml). The mixture was heated a further hour at 130°, and then cooled and evaporated *in vacuo*. Chromatography of the residue on silica gel, and elution with hexane-ethyl acetate mixtures afforded the title compound (100 mg) with properties identical to those described in Example 94.

Example 121

1-(Acetoxyethyl) 5R,3-(3-aminocarbonylphenyl)-6S-(1R-hydroxyethyl)-7-oxo-4-thia-1-azabicyclo[3.2.0]hept-2-ene-2-carboxylate.

Under a nitrogen atmosphere a stirred mixture of potassium 5R,3-(3-aminocarbonylphenyl)-6S-(1R-hydroxyethyl)-7-oxo-4-thia-1-azabicyclo[3.2.0]hept-2-ene-2-carboxylate (100 mg) sodium idoide (80 mg) and dry dimethylformamide (2.5 ml) was treated at room temperature with a solution of 1-chloroethyl acetate (164 mg) in chloroform (0.26 ml). After 18 hours the mixture was partitioned between ethyl acetate and water; the organic layer was evaporated to dryness and chromatographed on silica gel. Elution with ethyl acetate - ethanol mixtures afforded the title compound (16 mg) as a yellow gum as a mixture of diastereoisomers.

$\delta$($CDCl_3$) 1.37 (6H, d, J=6.5 Hz); 2.01 (3H, s);
3.82 (1H, dd, J=1.5 and 6.7 Hz); 4.27 (1H, m);
5.73 (1H, d, J=1.5 Hz); 5.95 (1H, broad);
6.57 (1H, broad); 6.79 (1H, m); 7.48 (1H, ~t, J=7.8 Hz);
7.60 (1H, m); 7.91 (1H, m).

Example 122

Acetoxymethyl 5R,3-(3-aminocarbonylphenyl)-6S-(1R-hydroxyethyl)-7-oxo-4-thia-1-azabicyclo[3.2.0]-hept-2-ene-2-carboxylate.

By a procedure analogous to that used in Example 121, but using potassium 5R,3-(3-aminocarbonylphenyl)-6S-(1R-hydroxyethyl)-7-oxo-4-thia-1-azabicyclo[3.2.0]-hept-2-ene-2-carboxylate (100 mg), sodium iodide (80 mg), dry dimethylformamide (2 ml) and chloromethyl acetate (150 mg), the title compound (25 mg) was obtained as a yellow gum.

δ($CD_3CN$) 1.22 (3H, d, J=6.3 Hz); 2.01 (3H, s); 3.85(1H, dd, J=1.5 and 6 Hz); 4.2 (1H, m); 5.75 (1H, d, J=1.5 Hz); 5.72 and 5.80 (2H, AB, J=6 Hz); 6.0 and 6.9 (2H, broad s); 7.50 (1H, ~t, J=8 Hz); 7.60 (1H, m) and 7.92 (1H, m).

Claims :

1. A compound of formula I

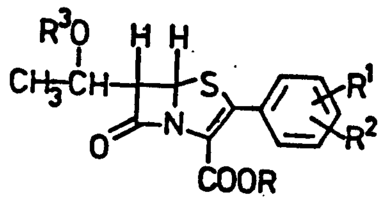

(I)

in which

R represents a hydrogen atom or a carboxylic acid esterifying group;

$R^1$ represents

(i) one of the following groups

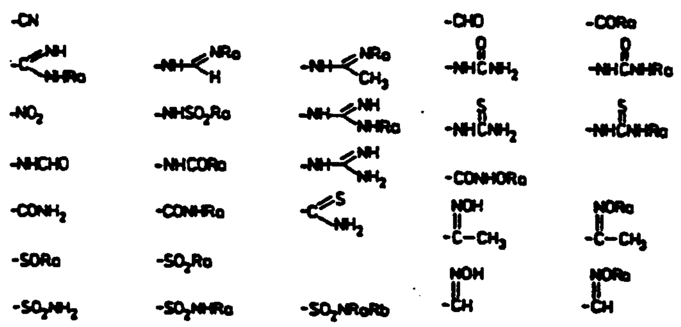

in which Ra and Rb, which may be the same or different, each represents an alkyl group having from 1 to 4 carbon atoms, or

(ii) a $-CONH(CH_2)_mQ$ or $-NHCO(CH_2)_mQ$ group in which m represents an integer of from 1 to 3, and Q represents one of the following groups

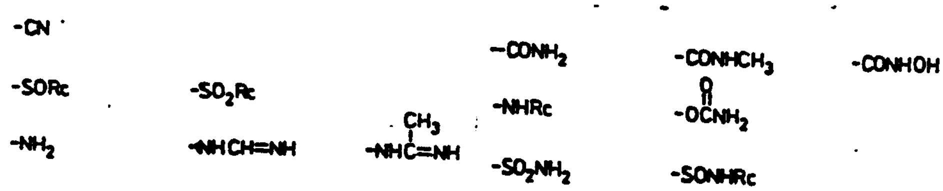

in which Rc represents a methyl or ethyl group, or

(iii) a $-CO_2Rd$ group, in which Rd represents a methyl or ethyl group which is unsubstituted or is substituted

by one or more substituents, which may be the same or different, selected from

(a) halogen atoms and vinyl groups,

(b) phenyl groups which are unsubstituted or are substituted by one or more groups selected from alkoxy groups having from 1 to 4 carbon atoms, nitro groups and halogen atoms,

(c) silyl groups SiReRfRg, the groups Re, Rf and Rg being the same or different, each representing a phenyl group or an alkyl group having from 1 to 4 carbon atoms, and

(d) groups Q as defined above; or

(iv) a $-CO_2SiReRfRg$ group, in which Re, Rf and Rg are defined as in (c) above, or

(v) a $CO_2$-phenyl group, in which the phenyl moiety is unsubstituted or substituted as defined in (b) above;

$R^2$ represents (i) a hydrogen atom,

(ii) a group as defined above for $R^1$ ($R^1$ and $R^2$ being the same or different), or

(iii) a chlorine, bromine or iodine atom, an alkyl group having from 1 to 4 carbon atoms, an $-NH_2$, -NHRa or -NRaRb group, an -OH or -ORa group, or an $-OCOCH_3$ group, Ra and Rb being defined as above, and

$R^3$ represents a hydrogen atom or a hydroxy protecting group; and in which $R^1$ and $R^2$, independently of each other, may be present at any position on the phenyl ring;

and salts thereof, especially physiologically tolerable salts; and isomers thereof.

2. A compound of formula I as claimed in claim 1, wherein in a group containing an alkyl group Ra or Rb, Ra or Rb denotes a methyl or ethyl group; in a group containing the symbol "m", "m" denotes the

integer 1 or 2.

3. A compound of formula I as claimed in claim 1, wherein $R^1$ represents one of the following groups; $-NHCO(CH_2)_mQ$

$-CONH(CH_2)_mQ$   $-NH\overset{O}{\overset{\|}{C}}NH_2$   $-CONH_2$   $-CONHRa$

$-NHCHO$   $-NHCORa$

$-SORa$   $-SO_2NH_2$   $-SO_2NHRa$   $-\overset{S}{\overset{\|}{C}}NH_2$

wherein Q is as defined in claim 1 and Ra and m are as defined in claim 2.

4. A compound of formula I as claimed in any one of claims 1 to 3, wherein a protected carboxy group -COOR is an esterified carboxy group that can be converted by hydrolysis, by photolysis, by oxidation, by reduction or by esterase action to give the free acid of formula I or a carboxylate, for example the group R is a phenyl group or a methyl group substituted by one or more unsubstituted or substituted phenyl groups; and wherein a phenyl group, either as R or as a substituent of a methyl group, is optionally substituted by one or more substituents selected from methoxy and nitro groups and halogen atoms, for example , R represents a benzyl, nitrobenzyl, methoxybenzyl, dimethoxybenzyl, phthalidyl, benzhydryl or trityl group; or R represents a phenoxyethyl or trichloroethyl group; or R represents a methyl or ethyl group optionally substituted by an acyl or acyloxy group, by an alkoxycarbonyloxy group, by an aminoalkanoyloxy group or by an optionally substituted amino group, for example, R represents an acyloxymethyl or acyloxyethyl group having from 2 to 12 for example 2 to 6 carbon atoms in the acyl moiety, an aminoalkanoyloxymethyl group having from 2 to 12 for example 2 to 6 carbon

atoms in the alkanoyl moiety, a 1'-(alkoxycarbonyloxy)ethyl group, or an optionally substituted 2-aminoethyl group, especially a glycyloxymethyl, L-valinyloxymethyl or L-leucyloxymethyl group, a 1'-(methoxycarbonyloxy)ethyl group, a pivaloyloxymethyl, ethoxycarbonyloxymethyl, acetylmethyl, acetoxymethyl, 1'-(acetoxy)ethyl, 1'-(acetyl)ethyl or 1'-(ethoxycarbonyloxy)ethyl group or a 2-diethylaminoethyl or 2-(1-morpholino)-ethyl group; or R represents a 5-methyldioxalen-2-on-4-yl-methyl group; or R represents a trialkylsilyl or trialkylsilylalkyl group, in which groups alkyl moieties have from 1 to 4 carbon atoms.

5. A compound of formula I as claimed in any one of claims 1 to 4, wherein a hydroxy protecting group $R^3$ is a group that can be converted by hydrolysis, by photolysis, by reduction or by esterase enzyme action to give a compound of formula I having a free 8-hydroxy group, for example $R^3$ is a carboxylic acid acyl group of the formula $R^{20}CO-$ in which $R^{20}$ represents a hydrogen atom or a straight or branched chain alkyl group having from 1 to 6 carbon atoms, or represents a phenyl group or a phenoxyalkyl group in which the alkyl moiety is straight-chained or branched and has from 1 to 4 carbon atoms, for example, $R^{20}$ represents a methyl, ethyl or t-butyl group, or a phenoxymethyl group.

6. A compound of formula I as claimed in any one of claims 1 to 5, wherein R represents a hydrogen atom or a physiologically tolerable salt forming group or a group that can be cleaved _in vivo_ to give a free carboxy group or a carboxylate group, and $R^3$ represents a hydrogen atom or a group that can be cleaved _in vivo_ to give a free hydroxy group.

7. A compound as claimed in any one of claims 1 to 6, having, independently or in any combination, 5R-stereochemistry, 6S-stereochemistry and 8R-stereochemistry, especially having 5R, 6S, 8R-stereochemistry.

8. A compound as claimed in claim 1 which is 5R,3-(4-Aminocarbonylphenyl)-6S-(1R-hydroxyethyl)-7-oxo-4-thia-1-azabicyclo[3.2.0]hept-2-ene-2-carboxylic acid, or an ester thereof at the 8- and/or 2-position, or a salt thereof, especially a physiologically tolerable salt.

9. A compound as claimed in claim 1 which is 5R,3-(3-Aminocarbonylphenyl)-6S-(1R-hydroxyethyl)-7-oxo-4-thia-1-azabicyclo[3.2.0]hept-2-ene-2-carboxylic acid, or an ester thereof at the 8- and/or 2-position, or a salt thereof, especially a physiologically tolerable salt.

10. A compound as claimed in claim 1 which is 5R,3-(4-Formylaminophenyl)-6S-(1R-hydroxyethyl)-7-oxo-4-thia-1-azabicyclo[3.2.0]hept-2-ene-2-carboxylic acid, or an ester thereof at the 8- and/or 2-position, or a salt thereof, especially a physiologically tolerable salt.

11. A compound as claimed in claim 1 which is 5R,3-(3-Formylaminophenyl)-6S-(1R-hydroxyethyl)-7-oxo-4-thia-1-azabicyclo[3.2.0]hept-2-ene-2-carboxylic acid, or an ester thereof at the 8- and/or 2-position, or a salt thereof, especially a physiologically tolerable salt.

12. A process for the production of a compound of formula I as claimed in claim 1, which comprises
(A) reacting a compound of formula II

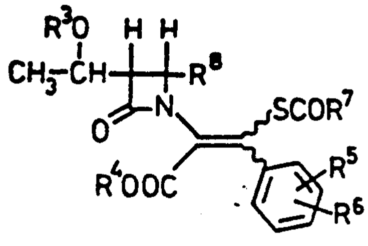

(II)

in which $R^3$ is as defined in claim 1,
$R^4$ represents a carboxy protecting group,
$R^5$ represents a group $R^1$ as defined in claim 1 or a group that can be converted into a group $R^1$,
$R^6$ represents a group $R^2$ as defined in claim 1 or a group that can be converted into a group $R^2$,
$R^7$ represents a phenyl group or an alkyl group having from 1 to 4 carbon atoms, and
$R^8$ represents a bromine or chlorine atom, with a base for example, ammonia, an alkali metal carbonate, bicarbonate or hydroxide; a primary amine, an alkali metal alkoxide or a heterocyclic base having a $pK_a$ within the range of from 5 to 9; or
(B) effecting cyclisation of a compound of formula III

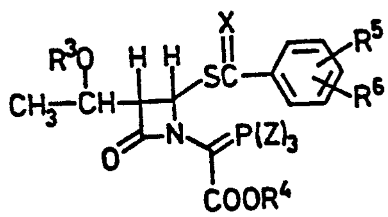 (III)

in which $R^3$, $R^4$, $R^5$ and $R^6$ are defined as above, X represents an oxygen or sulphur atom, and the group $P(Z)_3$ represents a group derived from a trivalent organophosphorus reagent, by allowing compound III to stand at room temperature or by heating compound III, or
(C) reacting a compound of formula IV or V

(IV) 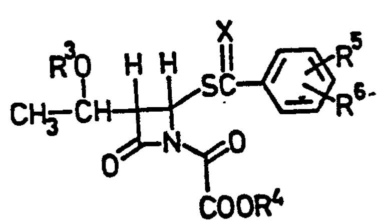 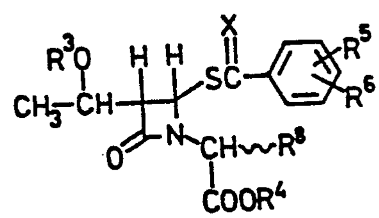 V)

in which X, $R^3$, $R^4$, $R^5$, $R^6$, and $R^8$ are defined as above, with a trivalent organophosphorus compound and effecting cyclisation of the reaction product by heating it or allowing it to stand, or

(D) reacting a compound of formula VI

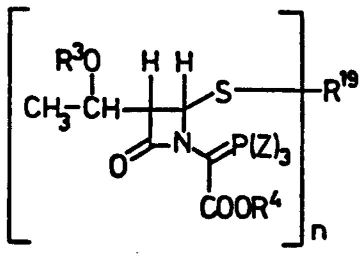

(VI)

in which $R^3$, $R^4$ and the group $P(Z)_3$ are defined as above, and $R^{19}$ represents Cu(II), Pb(II) or Hg(II), in which case n represents 2, or $R^{19}$ represents Ag(I), in which case n represents 1, with a compound of formula VII

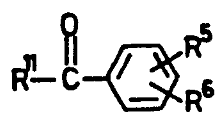

(VII )

in which $R^{11}$ represents an activating ester group or a halogen atom, and X, $R^5$ and $R^6$ are defined as above, and effecting cyclisation of the reaction product by heating it or allowing it to stand, and

(E) in an appropriate compound in which $R^5$ and/or $R^6$ represents a group that can be converted into a group $R^1$ and/or $R^2$, respectively, converting such a group or groups $R^5$ and/or $R^6$ into such a group or groups $R^1$ and/or $R^2$ and,

(F) if desired or required, in an appropriate compound converting a group $R^1$ and/or a group $R^2$ into another group $R^1$ and/or $R^2$, respectively; and

(G) if desired or required, carrying out any one or

more of the following steps in any desired order:
a) hydrolysing a 2-carboxylic ester group in an appropriate compound to give the corresponding free acid,
b) treating an appropriate free acid or a salt thereof with an agent capable of forming a 2-carboxylic acid ester to give a 2-carboxylic acid ester thereof,
c) carrying out an acid- or base-catalysed ester interchange on an appropriate 2-carboxylic acid ester to give a different ester of that compound,
d) treating an appropriate free acid compound with a base to give a salt at the carboxy group at position 2,
e) treating an appropriate free acid or 2-carboxylic acid ester having a basic group present with an acid to give an acid addition salt thereof,
f) treating a salt of an appropriate compound with an acid to give a free acid of that compound,
g) removing a hydroxy protecting group from an appropriate compound having a protected 8-hydroxy group to give the corresponding compound having a free 8-hydroxy group,
h) treating an appropriate compound having a free hydroxy group at the 8-position with an organic acid derivative to form an ester at the 8-position, and
i) treating an appropriate compound to effect a change in the stereochemical configuration.

13. A process as claimed in claim 12(A), wherein a compound of formula II is produced by halogenating a compound of formula XI

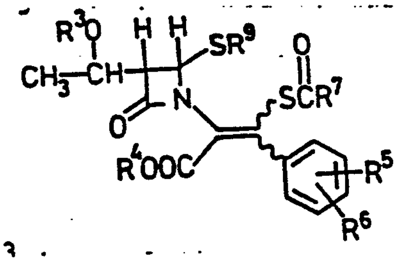

(XI)

in which $R^3$ is as defined in claim 1 and is especially a hydrogen atom, and $R^4$, $R^5$, $R^6$ and $R^7$ are as defined in claim 12, and $R^9$ represents an alkyl group having from 1 to 8 carbon atoms, an alkenyl group having from 2 to 4 carbon atoms, or a phenyl group, using an agent capable of splitting a carbon sulphur bond and of introducing a halogen atom, for example, molecular chlorine, sulphuryl chloride, t-butyl hypochlorite, cyanogen chloride or molecular bromine.

14. A process as claimed in claim 12 or claim 13, wherein a compound of formula XI is produced by reacting a compound of formula X

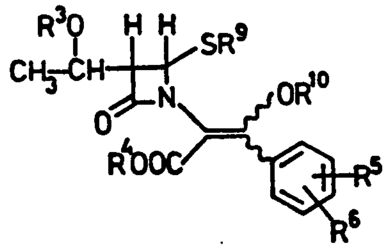

(X)

in which $R^3$ is as defined in claim 1, $R^4$, $R^5$ and $R^6$ are defined as in claim 12, $R^9$ is defined as in claim 13, and $R^{10}$ represents a group $-SO_2-Rh$ or $-CORh$ in which Rh represents an alkyl group having from 1 to 4 carbon atoms, an optionally substituted phenyl group, or a polyfluoroalkyl group, with a compound of formula XIII

$R^7COSH$ (XIII)

in which $R^7$ is as defined in claim 12; and wherein a compound of formula X is preferably produced by reacting a compound of formula IX or a tautomer thereof

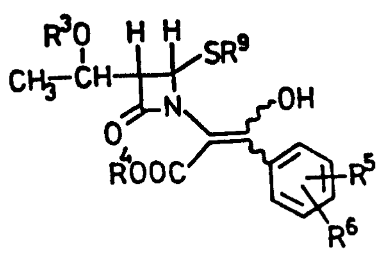

(IX)

in which $R^3$ is as defined in claim 1, $R^4$, $R^5$ and $R^6$ defined as in claim 12 and $R^9$ is defined as in claim 13, in the presence of a base with a compound of formula XII

$$R^{10} - R^{11} \qquad (XII)$$

in which $R^{10}$ is as defined above, and $R^{11}$ represents an activating group; and wherein a compound of formula IX is preferably produced by reacting a compound of formula VIII

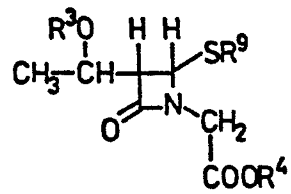

(VIII)

in which $R^3$ is as defined in claim 1, $R^4$ is defined as in claim 12 and $R^9$ is defined as in claim 13, in the presence of a base with an activated carboxylic acid derivative of formula VIIa

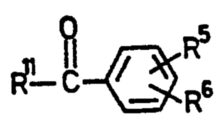

(VIIa)

in which $R^5$ and $R^6$ are defined as in claim 12, and $R^{11}$ is defined as above.

15. A process as claimed in claim 12(C),
(i) wherein a compound of formula IV is produced by reacting a compound of formula XV

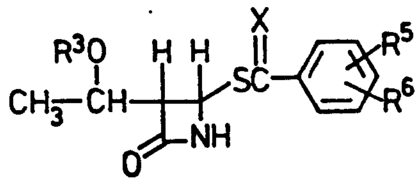 (XV)

in which $R^3$ is defined as in claim 1, and $R^5$ and $R^6$ and X are defined as in claim 12, with a compound of formula XIX

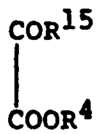 (XIX)

in which $R^4$ is as defined in claim 12 and $R^{15}$ represents a group that can be displaced by the azetidinone nitrogen in the compound of formula XV, for example, a halogen atom, an imidazolide group or a mixed anhydride group, to give a compound of formula IV, or

(ii) wherein a compound of formula V is produced by halogenating a compound of formula XVI

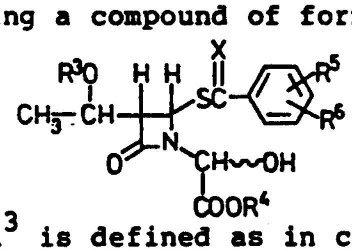 (XVI)

in which $R^3$ is defined as in claim 1, and $R^4$, $R^5$, $R^6$ and X are defined as in claim 12 using a halogen ating agent for example, thionyl chloride or bromide, phosphorus oxychloride or oxybromide, or a phosphorus halide, or a mixture of two or more thereof, and wherein a compound of formula XVI is preferably produced by reacting a compound of formula XV as defined in (i) above with a glyoxylic ester of formula XXVI

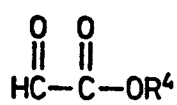 (XXVI)

in which $R^4$ is as defined in claim 9, or with a reactive derivative thereof, and

(iii) wherein a compound of formula XV is preferably produced by reacting a compound of formula XIV

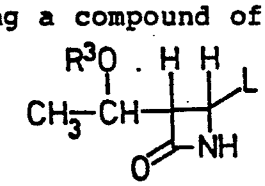

(XIV)

in which $R^3$ is as defined in claim 1, and L represents a leaving group, for example, a halogen atom, an alkylcarbonyloxy group in which the alkyl moiety has from 1 to 4 carbon atoms, has a straight or branched chain, and may be substituted by an electron-withdrawing group, for example, a halogen atom, especially a fluorine atom, a phenylcarbonyloxy group, or an $-SO_2Rj$ group in which Rj represents an alkyl group having from 1 to 4 carbon atoms or a phenyl group, and is preferably an acetoxy group, with a compound of formula XVII

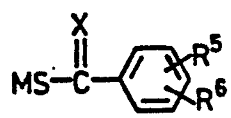

(XVII)

in which $R^5$, $R^6$ and X are defined as in claim 12, and M represents a hydrogen atom or an alkali metal or alkaline earth metal atom, or an ammonium group that is unsubstituted or substituted (when M represents hydrogen the reaction is carried out in the presence of a base), and

wherein a compound of formula XVII is preferably produced

(a) by reacting a compound of formula VII

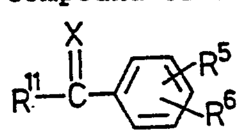

( VII )

in which X, $R^5$, $R^6$ are defined as in claim 12 and $R^{11}$-

is defined as in claim 12 with a compound of formula MSH or $M_2S$, in which M is as defined above, (when M represents hydrogen the reaction is carried out in the presence of a base); or

(b) by reacting a compound of formula XVIII

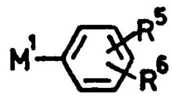

(XVIII)

in which $M^1$ represents an alkali metal or alkaline earth metal radical, together with a counter-ion, if required in the case where $M^1$ represents a divalent metal ion, with carbon disulphide or carbon oxysulphide, giving a compound of formula XVII in which M represents an alkali metal or alkaline earth metal atom.

16. A process as claimed in any one of claims 12 to 15 wherein a protected group $-OR^3$ is that from which the protecting group $R^3$ can be removed under acidic conditions, for example, a tetrahydropyranyloxy or tetrahydrofuranyloxy group; an acetal or ketal group; or a silyl ether, wherein an acetal or ketal group preferably has the formula

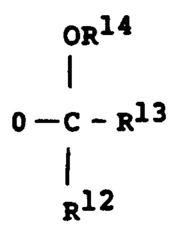

in which $R^{12}$ and $R^{13}$, which may be the same or different, each represents a hydrogen atom or an alkyl group having from 1 to 4 carbon atoms, or $R^{12}$ and $R^{13}$ together with the carbon atoms to which they are attached, represent a cycloalkyl ring having from 4 to 7 carbon atoms, and $R^{14}$ represents an alkyl group

having from 1 to 4 carbon atoms, or $R^{12}$ and $R^{14}$, together with the carbon atom and the oxygen atom to which they are attached, respectively, represent a tetrahydropyranyl ring; and a silyl ether has three substituents on the silicon atom and up to 24 carbon atoms in total, the three substituents being the same or different, and selected from alkyl, alkenyl and cycloalkyl groups, and phenyl and phenalkyl groups which may be unsubstituted or substituted, and wherein a silyl ether is preferably a -OSiReRfRg group in which Re, Rf and Rg are as defined in claim 1, and wherein a hydroxy protecting group $R^3$ is especially a tetrahydropyranyl, 2-methoxyprop-2-yl, trimethylsilyl, triethylsilyl or t -butyldimethylsilyl group.

17. A process as claimed in any one of claims 12 to claim 16, wherein in a compound of formula I or any appropriate precursor thereof, any one or more of the following conversions are carried out:

(a) a group $R^5$ to a group $R^1$ or to another group $R^5$, (including conversion of a radical present as part of a group $R^5$);

(b) a group $R^6$ to a group $R^2$ or to another group $R^6$, (including conversion of a radical present as part of a group $R^6$);

(c) a group $R^1$ to another group $R^1$, (including conversion of a radical present as part of a group $R^1$);

(d) A group $R^2$ to a group $R^2$, (including conversion of a radical present as part of a group $R^2$);

the conversion being carried out as follows:

(i) $-COORd$, $-COOSiReRfRg$ or $-COO$-phenyl to $-COOH$

(ii) $-COOH$ to $-CONH_2$, $-CONHRa$ or $-CONH(CH_2)_mQ$ or

(iii) $-COOH$ to $-COOR^4$ or $-CO_2CH_2Q$ or $-CO_2CH_2CH_2Q$ or $-CO_2CH(CH_3)Q$

(iv) $-COOH$ to $-CORa$

(v) $-NHRm$ or $-NRmRn$ in which Rm and Rn are protecting groups, to $-NH_2$

(vi) $-NH_2$ to

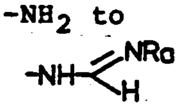 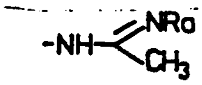 $-NHSO_2Ra$ 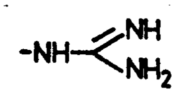

$-NHCHO$ $-NHCORa$ 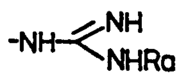

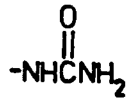 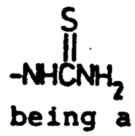 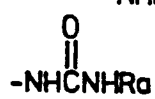 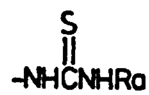

(vii) $-CONRaRm$, Rm being a protecting group, to $-CONHRa$

(viii) $-N_3$ to $-NH_2$, which is then optionally converted to a group $R^1$ as described in (vi) above,

(ix) halogen to $-CN$ or $-COOH$

(x) $-SRa$ to $-SORa$ or $-SO_2Ra$

(xi) $-CN$ to $-CH_2NH_2$, which is then optionally converted to a group as defined in (vi) above,

(xii) $-SORa$ to $-SO_2Ra$

(xiii) $-NO_2$ to $NH_2$, which is then optionally converted further as described in (vi) above.

18. A pharmaceutical preparation which comprises a compound of formula I as claimed in any one of claims 1 to 11 or a physiologically tolerable salt thereof, or a mixture of two or more such substances as active ingredient, in admixture or conjunction with a pharmaceutically suitable carrier.

19. A compound of formula I as claimed in any one of claims 1 to 11 or a physiologically tolerable salt

thereof for use in the manufacture of a medicament for the treatment of bacterial infections.

20. A compound of formula II

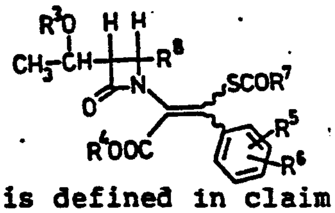

(II)

in which $R^3$ is defined in claim 1 and $R^4$, $R^5$, $R^6$, $R^7$ and $R^8$ are as defined in claim 8.

21. A compound of formula XXVII

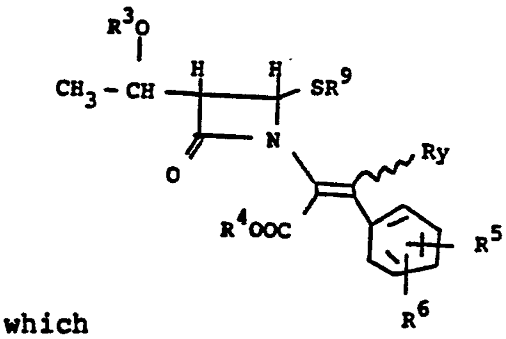

(XXVII)

in which

$R^3$ is as defined in claim 1,

$R^4$, $R^5$ and $R^6$ are as defined in claim 12,

$R^9$ is an alkyl group having 1 to 8 carbon atoms, an alkenyl group having 2 to 4 carbon atoms, or a phenyl group, and

Ry is $SCOR^7$, OH or $OR^{10}$ in which

$R^7$ is as defined in claim 12 and

$R^{10}$ is a group $-SO_2Rh$ or -CORh in which Rh is an alkyl group having from 1 to 4 carbon atoms, an optionally substituted phenyl group, or a polyfluoroalkyl group.

22. A compound of formula XXVIII

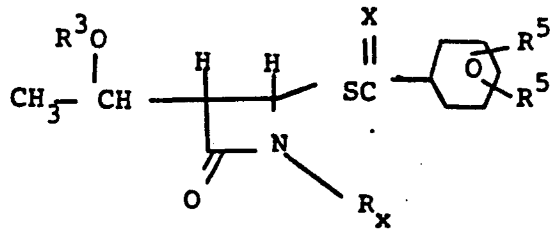

(XXVIII)

in which $R_x$ is H or a group

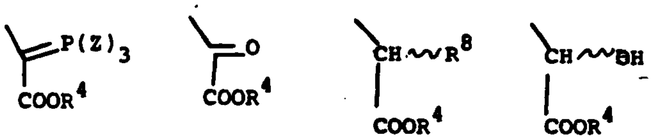

in which
$R^3$ is as defined in claim 1,
X, $R^4$, $R^5$, $R^6$, $R^7$, $R^8$, and $P(Z)_3$ are as defined in claim 12.

Patent Claims for Austria:

1. A process for the preparation of a compound of formula I

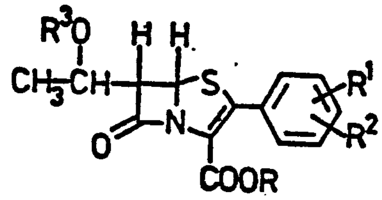

(I)

in which

R represents a hydrogen atom or a carboxylic acid esterifying group;

$R^1$ represents

(i) one of the following groups

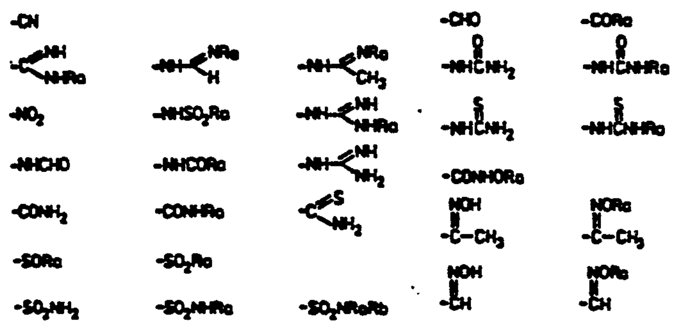

in which Ra and Rb, which may be the same or different, each represents an alkyl group having from 1 to 4 carbon atoms, or

(ii) a $-CONH(CH_2)_mQ$ or $-NHCO(CH_2)_mQ$ group in which m represents an integer of from 1 to 3, and Q represents one of the following groups

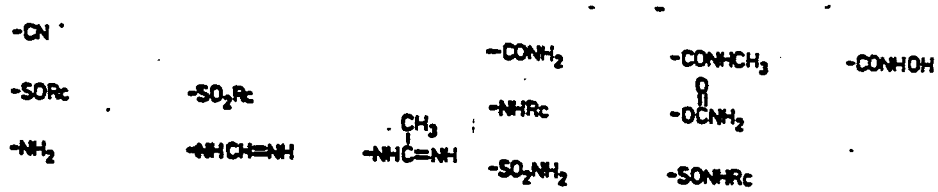

in which Rc represents a methyl or ethyl group, or

(iii) a $-CO_2Rd$ group, in which Rd represents a methyl or ethyl group which is unsubstituted or is substituted

by one or more substituents, which may be the same or different, selected from

(a) halogen atoms and vinyl groups,

(b) phenyl groups which are unsubstituted or are substituted by one or more groups selected from alkoxy groups having from 1 to 4 carbon atoms, nitro groups and halogen atoms,

(c) silyl groups SiReRfRg, the groups Re, Rf and Rg being the same or different, each representing a phenyl group or an alkyl group having from 1 to 4 carbon atoms, and

(d) groups Q as defined above; or

(iv) a $-CO_2SiReRfRg$ group, in which Re, Rf and Rg are defined as in (c) above, or

(v) a $CO_2$-phenyl group, in which the phenyl moiety is unsubstituted or substituted as defined in (b) above;

$R^2$ represents (i) a hydrogen atom,

(ii) a group as defined above for $R^1$ ($R^1$ and $R^2$ being the same or different), or

(iii) a chlorine, bromine or iodine atom, an alkyl group having from 1 to 4 carbon atoms, an $-NH_2$, -NHRa or -NRaRb group, an -OH or -ORa group, or an $-OCOCH_3$ group, Ra and Rb being defined as above, and

$R^3$ represents a hydrogen atom or a hydroxy protecting group; and in which $R^1$ and $R^2$, independently of each other, may be present at any position on the phenyl ring;

and salts thereof, especially physiologically tolerable salts; and isomers thereof,

which comprises

(A) reacting a compound of formula II

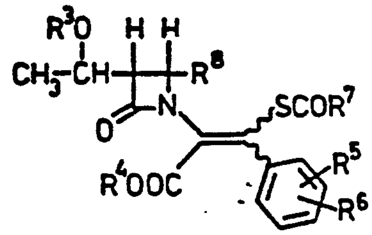

(II)

 

in which $R^3$ is as defined above,
$R^4$ represents a carboxy protecting group,
$R^5$ represents a group $R^1$ as defined above or a group that can be converted into a group $R^1$,
$R^6$ represents a group $R^2$ as defined above or a group that can be converted into a group $R^2$,
$R^7$ represents a phenyl group or an alkyl group having from 1 to 4 carbon atoms, and
$R^8$ represents a bromine or chlorine atom, with a base for example, ammonia, an alkali metal carbonate, bicarbonate or hydroxide; a primary amine, an alkali metal alkoxide or a heterocyclic base having a $pK_a$ within the range of from 5 to 9; or

(B) effecting cyclisation of a compound of formula III

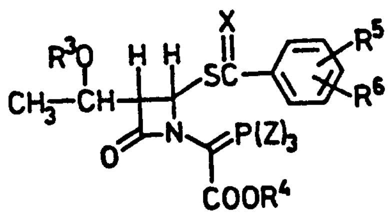

(III)

in which $R^3$, $R^4$, $R^5$ and $R^6$ are defined as above, X represents an oxygen or sulphur atom, and the group $P(Z)_3$ represents a group derived from a trivalent organophosphorus reagent, by allowing compound III to stand at room temperature or by heating compound III, or

(C) reacting a compound of formula IV or V

(IV) 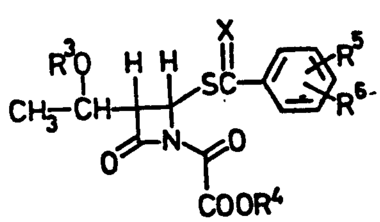 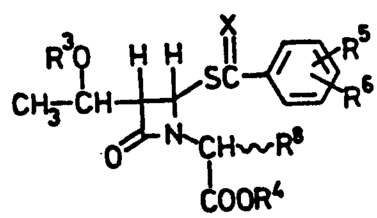 V)

in which X, $R^3$, $R^4$, $R^5$, $R^6$, and $R^8$ are defined as above, with a trivalent organophosphorus compound and effecting cyclisation of the reaction product by heating it or allowing it to stand, or

(D) reacting a compound of formula VI

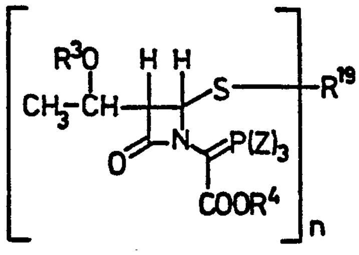

(VI)

in which $R^3$, $R^4$ and the group $P(Z)_3$ are defined as above, and $R^{19}$ represents Cu(II), Pb(II) or Hg(II), in which case n represents 2, or $R^{19}$ represents Ag(I), in which case n represents 1, with a compound of formula VII

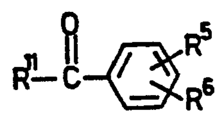

(VII )

in which $R^{11}$ represents an activating ester group or a halogen atom, and X, $R^5$ and $R^6$ are defined as above, and effecting cyclisation of the reaction product by heating it or allowing it to stand, and

(E) in an appropriate compound in which $R^5$ and/or $R^6$ represents a group that can be converted into a group $R^1$ and/or $R^2$, respectively, converting such a group or groups $R^5$ and/or $R^6$ into such a group or groups $R^1$ and/or $R^2$ and,

(F) if desired or required, in an appropriate compound converting a group $R^1$ and/or a group $R^2$ into another group $R^1$ and/or $R^2$, respectively; and

(G) if desired or required, carrying out any one or

more of the following steps in any desired order:
a) hydrolysing a 2-carboxylic ester group in an appropriate compound to give the corresponding free acid,
b) treating an appropriate free acid or a salt thereof with an agent capable of forming a 2-carboxylic acid ester to give a 2-carboxylic acid ester thereof,
c) carrying out an acid- or base-catalysed ester interchange on an appropriate 2-carboxylic acid ester to give a different ester of that compound,
d) treating an appropriate free acid compound with a base to give a salt at the carboxy group at position 2,
e) treating an appropriate free acid or 2-carboxylic acid ester having a basic group present with an acid to give an acid addition salt thereof,
f) treating a salt of an appropriate compound with an acid to give a free acid of that compound,
g) removing a hydroxy protecting group from an appropriate compound having a protected 8-hydroxy group to give the corresponding compound having a free 8-hydroxy group,
h) treating an appropriate compound having a free hydroxy group at the 8-position with an organic acid derivative to form an ester at the 8-position, and
i) treating an appropriate compound to effect a change in the stereochemical configuration.

2. A process as claimed in claim 1 wherein a protected group $-OR^3$ is that from which the protecting group $R^3$ can be removed under acidic conditions, for example, a tetrahydropyranyloxy or tetrahydrofuranyloxy group; an acetal or ketal group; or a silyl ether, wherein an acetal or ketal group preferably has the formula

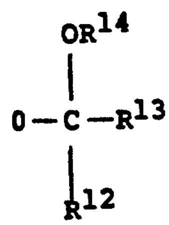

in which $R^{12}$ and $R^{13}$, which may be the same or different, each represents a hydrogen atom or an alkyl group having from 1 to 4 carbon atoms, or $R^{12}$ and $R^{13}$ together with the carbon atoms to which they are attached, represent a cycloalkyl ring having from 4 to 7 carbon atoms, and $R^{14}$ represents an alkyl group

having from 1 to 4 carbon atoms, or $R^{12}$ and $R^{14}$, together with the carbon atom and the oxygen atom to which they are attached, respectively, represent a tetrahydropyranyl ring; and a silyl ether has three substituents on the silicon atom and up to 24 carbon atoms in total, the three substituents being the same or different, and selected from alkyl, alkenyl and cycloalkyl groups, and phenyl and phenalkyl groups which may be unsubstituted or substituted, and wherein a silyl ether is preferably a -OSiReRfRg group in which Re, Rf and Rg are as defined in claim 1, and wherein a hydroxy protecting group $R^3$ is especially a tetrahydropyranyl, 2-methoxyprop-2-yl, trimethylsilyl, triethylsilyl or t -butyldimethylsilyl group.

3. A process as claimed in claim 1 or claim 2 , wherein in a compound of formula I or any appropriate precursor thereof, any one or more of the following conversions are carried out:

(a) a group $R^5$ to a group $R^1$ or to another group $R^5$, (including conversion of a radical present as part of a group $R^5$);

(b) a group $R^6$ to a group $R^2$ or to another group $R^6$, (including conversion of a radical present as part of a group $R^6$);

(c) a group $R^1$ to another group $R^1$, (including conversion of a radical present as part of a group $R^1$);

(d) A group $R^2$ to a group $R^2$, (including conversion of a radical present as part of a group $R^2$);

the conversion being carried out as follows:

(i) -COORd, -COOSiReRfRg or -COO-phenyl to -COOH

(ii) -COOH to $-CONH_2$, -CONHRa or $-CONH(CH_2)_mQ$ or

(iii) -COOH to $-COOR^4$ or $-CO_2CH_2Q$ or $-CO_2CH_2CH_2Q$ or $-CO_2CH(CH_3)Q$

(iv) -COOH to -CORa

(v) -NHRm or -NRmRn in which Rm and Rn are protecting groups, to $-NH_2$

(vi) $-NH_2$ to

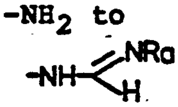 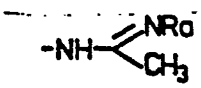 $-NHSO_2Ra$ 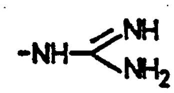

-NHCHO -NHCORa 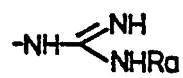

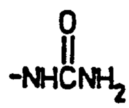 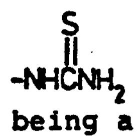 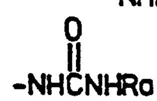 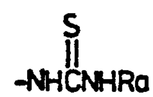

(vii) -CONRaRm, Rm being a protecting group, to -CONHRa

(viii) $-N_3$ to $-NH_2$, which is then optionally converted to a group $R^1$ as described in (vi) above,

(ix) halogen to -CN or -COOH

(x) -SRa to -SORa or $-SO_2Ra$

(xi) -CN to $-CH_2NH_2$, which is then optionally converted to a group as defined in (vi) above,

(xii) -SORa to $-SO_2Ra$

(xiii) $-NO_2$ to $NH_2$, which is then optionally converted further as described in (vi) above.

4. A process as claimed in any one of claims 1 to 3 wherein in a group containing an alkyl group Ra or Rb, Ra or Rb denotes a methyl or ethyl group; in a group containing the symbol "m", "m" denotes the integer 1 or 2.

5. A process as claimed in any one of claims 1 to 4 wherein $R^1$ represents one of the following groups; $-NHCO(CH_2)_mQ$

$-CONH(CH_2)_mQ$ $\quad -NH\overset{O}{\overset{\|}{C}}NH_2$ $\quad -CONH_2$ $\quad -CONHRa$

$-NHCHO$ $\quad -NHCORa$

$-SORa$ $\quad -SO_2NH_2$ $\quad -SO_2NHRa$ $\quad -\overset{S}{\overset{\|}{C}}NH_2$

wherein Q is as defined in claim 1 and Ra and m are as defined in claim 1.

6. A process as claimed in any one of claims 1 to 5, wherein a protected carboxy group -COOR, is an esterified carboxy group that can be converted by hydrolysis, by photolysis, by oxidation, by reduction or by esterase action to give the free acid of formula I or a carboxylate, for example the group R is a phenyl group or a methyl group substituted by one or more unsubstituted or substituted phenyl groups; and wherein a phenyl group, either as R or as a substituent of a methyl group, is optionally substituted by one or more substituents selected from methoxy and nitro groups and halogen atoms, for example , R represents a benzyl, nitrobenzyl, methoxybenzyl, dimethoxybenzyl, phthalidyl, benzhydryl or trityl group; or R represents a phenoxyethyl or trichloroethyl group; or R represents a methyl or ethyl group optionally substituted by an acyl or acyloxy group, by an alkoxycarbonyloxy group, by an aminoalkanoyloxy group or by an optionally substituted amino group, for example, R represents an acyloxymethyl or acyloxyethyl group having from 2 to 12 for example 2 to 6 carbon atoms in the acyl moiety, an aminoalkanoyloxymethyl group having from 2 to 12 for example 2 to 6 carbon

atoms in the alkanoyl moiety, a 1'-(alkoxycarbonyloxy)ethyl group, or an optionally substituted 2-aminoethyl group, especially a glycyloxymethyl, L-valinyloxymethyl or L-leucyloxymethyl group, a 1'-(methoxycarbonyloxy)ethyl group, a pivaloyloxymethyl, ethoxycarbonyloxymethyl, acetylmethyl, acetoxymethyl, 1'-(acetoxy)ethyl, 1'-(acetyl)ethyl or 1'-(ethoxycarbonyloxy)ethyl group or a 2-diethylaminoethyl or 2-(1-morpholino)-ethyl group; or R represents a 5-methyldioxalen-2-on-4-yl-methyl group; or R represents a trialkylsilyl or trialkylsilylalkyl group, in which groups alkyl moieties have from 1 to 4 carbon atoms.

7. A process as claimed in any one of claims 1 to 6, wherein a hydroxy protecting group $R^3$ is a group that can be converted by hydrolysis, by photolysis, by reduction or by esterase enzyme action to give a compound of formula I having a free 8-hydroxy group, for example $R^3$ is a carboxylic acid acyl group of the formula $R^{20}CO$- in which $R^{20}$ represents a hydrogen atom or a straight or branched chain alkyl group having from 1 to 6 carbon atoms, or represents a phenyl group or a phenoxyalkyl group in which the alkyl moiety is straight-chained or branched and has from 1 to 4 carbon atoms, for example, $R^{20}$ represents a methyl, ethyl or t-butyl group, or a phenoxymethyl group.

8. A process as claimed in any one of claims 1 to 6, wherein a hydroxy protecting group $R^3$ a group that can be cleaved <u>in vivo</u> to give a free carboxy group or a carboxylate group, and $R^3$ represents a hydrogen atom or a group that can be cleaved <u>in vivo</u> to give a free hydroxy group.

9. A process as claimed in any one of claims 1 to 8 leading to, independently or in any combination, 5R-stereochemistry, 6S-stereochemistry and 8R-stereochemistry, especially having 5R, 6S, 8R-stereochemistry.

10. A process as claimed in claim 1 according to which 5R,3-(4-Aminocarbonylphenyl)-6S-(1R-hydroxyethyl)-7-oxo-4-thia-1-azabicyclo/3.2.0/hept-2-ene-2-carboxylic acid, or an ester thereof at the 8- and/or 2-position, or a salt thereof, especially a physiologically tolerable salt is produced.

11. A process as claimed in claim 1 according to which 5R,3-(3-Aminocarbonyl)-6S-(1R-hydroxyethyl)-7-oxo-4-thia-1-azabicyclo/3.2.0/hept-2-ene-2-carboxylic acid, or an ester thereof at the 8- and/or 2-position, or a salt thereof, expecially a physiologically tolerable salt is produced.

12. A process as claimed in claim 1 according to which 5R,3-(4-Formylaminophenyl)-6S-(1R-hydroxyethyl)-7-oxo-4-thia-1-azabicyclo[3.2.0]hept-2-ene-2-carboxylic acid, or an ester thereof at the 8- and/or 2-position, or a salt thereof, especially a physiologically tolerable salt is produced.

13. A process as claimed in claim 1 according to which 5R,3-(3-Formylaminophenyl)-6S-(1R-hydroxyethyl)-7-oxo-4-thia-1-azabicyclo[3.2.0]hept-2-ene-2-carboxylic acid, or an ester thereof at the 8- and/or 2-position, or a salt thereof, especially a physiologically tolerable salt is produced.

14. A process for the production of a pharmaceutical preparation which comprises mixing a compound of formula I as produced according to claims 1 to 13 or a physiologically tolerable salt thereof, or a mixture of two or more such substances as active ingredient, with a pharmaceutically suitable carrier.

15. A process for the production of a

compound of formula II

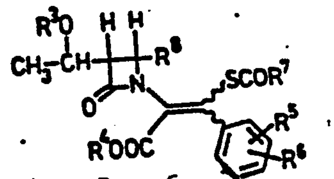

(II)

in which $R^3$, $R^4$, $R^5$, $R^6$, $R^7$ and $R^8$ are as defined in claim 1, wherein a compound of formula II is produced by halogenating a compound of formula XI

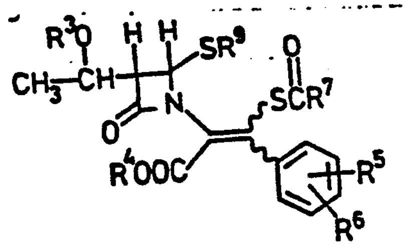

(XI)

in which $R^9$ represents an alkyl group having from 1 to 8 carbon atoms, an alkenyl group having from 2 to 4 carbon atoms, or a phenyl group, using an agent capable of splitting a carbon sulphur bond and of introducing a halogen atom, for example, molecular chlorine, sulphuryl chloride, t-butyl hypochlorite, cyanogen chloride or molecular bromine.

16. A process for the production of a compound of formula IV as defined in claim 1 wherein either

(a) a compound of formula XV

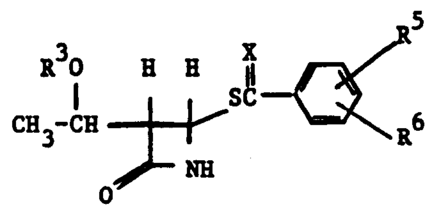

XV

in which $R^3$, $R^5$ and $R^6$ are as defined in Claim 1, is reacted with a compound of formula XIX

$R^4OOC-COR^{15}$ XIX

in which $R^4$ is as defined in claim 1 and $R^{15}$ represents a group that can be displaced by the azetidinone nitrogen in the compound of formula XV, for example, a halogen atom, an imidazolide group or a mixed anhydride group, such reaction preferably being carried out in the presence of a base, or

(b) a compound of formula XXVII

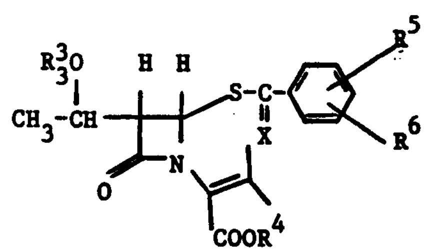

XXVII

in which $R^3$, $R^4$, $R^5$, $R^6$ and X are as defined in claim 1 is treated with an oxidising agent.

0212404

European Patent Office

# EUROPEAN SEARCH REPORT

Application number

EP 86 11 0710

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | EP-A-0 002 210 (MERCK)<br>* Pages 63,64, compound 18; pages 125,126, compounds 18,19,22; claims * | 1-7,12-22 | C 07 D 499/00<br>C 07 D 205/08<br>C 07 F 7/18<br>A 61 K 31/43 |
| D,Y | EP-A-0 070 204 (SUMITOMO)<br>* Claims * | 1-7,12-22 | |
| D,Y | GB-A-2 042 515 (BRISTOL-MYERS)<br>* Claims * | 1-7,12-22 | |
| P,Y | EP-A-0 174 561 (HOECHST)<br>* Claims * | 1-7,12-22 | |

TECHNICAL FIELDS SEARCHED (Int. Cl.4)

C 07 D 499/00
C 07 D 205/00
A 61 K 31/00

The present search report has been drawn up for all claims

| Place of search | Date of completion of the search | Examiner |
|---|---|---|
| THE HAGUE | 23-09-1986 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82